(19) Europäisches Patentamt / European Patent Office / Office européen des brevets

(11) **EP 4 679 433 A1**

(12) **EUROPEAN PATENT APPLICATION**
published in accordance with Art. 153(4) EPC

(43) Date of publication:
**14.01.2026 Bulletin 2026/03**

(21) Application number: 24766980.7

(22) Date of filing: **28.02.2024**

(51) International Patent Classification (IPC):
**G16C 20/30** (2019.01)    **C08J 5/04** (2006.01)
**G16C 20/70** (2019.01)

(52) Cooperative Patent Classification (CPC):
**B29B 17/00; C08J 5/04; C08K 3/013; C08K 3/34; C08K 7/14; C08L 21/00; C08L 23/10; C08L 101/00; G16C 20/30; G16C 20/70; G16C 60/00; Y02W 30/62**

(86) International application number:
**PCT/JP2024/007237**

(87) International publication number:
**WO 2024/185609 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.03.2023 JP 2023035983**

(71) Applicant: **Sumitomo Chemical Company, Limited Tokyo 103-6020 (JP)**

(72) Inventors:
• **ISHIZUKA, Kenta**
  **Ichihara-shi, Chiba 299-0195 (JP)**
• **SATO, Hiroki**
  **Ichihara-shi, Chiba 299-0195 (JP)**

(74) Representative: **Global IP Europe Patentanwaltskanzlei Pfarrstraße 14 80538 München (DE)**

(54) **COMPOSITION PROPOSAL SYSTEM, METHOD FOR PRODUCING RECYCLED POLYMER COMPOSITE MATERIAL, AND RECYCLED POLYMER COMPOSITE MATERIAL**

(57)    A composition proposal system according to the present invention includes a prediction unit 103 that outputs a parameter including a descriptor of a recycled polymer material predicted by inputting a physical property value of the recycled polymer material acquired by a first acquisition unit 101 to a first learned model M5-1, a setting unit 107 that sets a target value of a physical property of a virtual recycled polymer composite material, and an optimization unit 112 that changes a parameter including a descriptor of the virtual recycled polymer composite material so that the physical property value of the virtual recycled polymer composite material approaches a target value, in which the optimization unit 112 optimizes a mass ratio of the recycled polymer material and other components contained in the virtual recycled polymer composite material by changing the parameter including the descriptor of the virtual recycled polymer composite material each time the physical property value of the virtual recycled polymer composite material is predicted.

FIG. 3

Physical property value of recycled polymer material

Target value of physical property of virtual recycled polymer composite material

<Composition proposal system> 10

M5-1 First learned model

101 First acquisition unit

102 Preprocessing unit

103 Prediction unit

Parameter (including descriptor of recycled polymer material)

104 Second acquisition unit

105 Learning data set generation unit

Learning data set

Parameter (including descriptor of recycled polymer composite material) and physical property value of recycled polymer composite material

106 <Learning unit>

M5-2 Second learned model

Parameter (including descriptor of virtual recycled polymer composite material)

107 Setting unit

Parameter (including descriptor of virtual recycled polymer composite material)

Physical property value of virtual recycled polymer composite material

Third acquisition unit 108

Physical property prediction unit 109

Comparison unit 110

112 Optimization unit

Virtual descriptor

Determination unit 111

113 Display unit

2

**Description**

TECHNICAL FIELD

The present invention relates to a composition proposal system, a method for producing a recycled polymer composite material, and a recycled polymer composite material.

BACKGROUND ART

A polymer composite material formed of a composition containing a plurality of polymer materials is widely used as a material for an automobile part, a home electric appliance, a food and medical container, a building and civil engineering industrial material, and the like. As the polymer composite material, for example, a propylene-based resin composition containing a propylene-based polymer is used.

As a propylene-based resin composition, for example, a propylene resin composition containing a propylene homopolymer, a random copolymer of propylene and a monomer other than propylene, or a propylene-based polymer containing a heterophasic propylene polymerization material, and an ethylene-$\alpha$-olefin copolymer is disclosed (see, for example, Patent Document 1).

PRIOR ART DOCUMENT

PATENT DOCUMENT

Patent Document 1: JP-A-2018-178107

SUMMARY OF THE INVENTION

PROBLEMS TO BE SOLVED BY THE INVENTION

Here, when producing a polymer composite material, it is required to determine the type of the polymer used for producing the polymer composite material, the composition of the polymer composite material, and the like so as to satisfy arbitrary characteristics of the polymer composite material and the like according to the application of the polymer composite material and the like. In order to determine the type of the polymer, the composition of the polymer composite, and the like, actually, in experiments, it is required to produce a polymer composite material using a composition containing various polymers and measure physical properties thereof, or to search for a composition of the polymer composite material having optimal physical properties; thus, a lot of effort was required, and it was required to prepare raw materials and devices.

Therefore, from the viewpoint of improving energy efficiency generated in production of a polymer composite material or the like, a method capable of efficiently proposing physical properties of the polymer composite material from parameters such as a composition and a structure of the polymer composite material is required.

In particular, when a virgin polymer and a polymer that is recycled (recycled polymer) are used for producing a polymer composite material, a composition of the recycled polymer is often not constant, and therefore, it takes time and effort to accurately analyze characteristics such as a composition and a rubber amount. Therefore, in order to obtain a polymer composite material having desired physical properties, it was difficult to determine the degree to which the virgin polymer and the recycled polymer should be blended.

An object of one aspect of the present invention is to provide a polymer composite material having desired physical properties.

MEANS FOR SOLVING THE PROBLEMS

One aspect of the present invention is
a composition proposal system that proposes a composition of a constituent component contained in a recycled polymer composite material containing a recycled polymer material so that the recycled polymer composite material satisfies one or more desired physical property values, the composition proposal system including:

an acquisition unit that acquires a physical property value of the recycled polymer material;
a prediction unit that outputs a parameter including a descriptor of the recycled polymer material predicted by a first learned model by inputting the physical property value of the recycled polymer material acquired by the acquisition unit to the first learned model learned using a first learning data set in which a physical property value of a virgin polymer composite material containing a virgin polymer material and a parameter including a descriptor of the virgin polymer composite material are associated with each other;

a setting unit that sets a target value of a physical property of a virtual recycled polymer composite material; and an optimization unit that changes a parameter including a descriptor of the virtual recycled polymer composite material so that a physical property value of the virtual recycled polymer composite material predicted by inputting the parameter including the descriptor of the virtual recycled polymer composite material to a second learned model learned using a second learning data set in which the parameter including the descriptor of the recycled polymer composite material and the physical property value of the recycled polymer composite material are associated with each other approaches the target value,

in which the optimization unit optimizes a mass ratio of the recycled polymer material and components other than the recycled polymer material contained in the virtual recycled polymer composite material as the constituent components by changing the parameter including the descriptor of the virtual recycled polymer composite material each time the physical property value of the virtual recycled polymer composite material is predicted.

Another aspect of the present invention is

a recycled polymer composite material containing a recycled polymer material and components other than the recycled polymer material as constituent components,

in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.08 $g/cm^3$ to 1.16 $g/cm^3$,

(II) an elastic modulus of the recycled polymer composite material is $128 \times Wt + 213 \times Wg - 102 \times Wr + 1,081 > 5,000$ MPa,

(III) a flexural strength of the recycled polymer composite material is $-0.375 \times Wt + 1.52 \times Wg - 3.30 \times Wr + 106 > 80$ MPa, and

(IV) an impact of the recycled polymer composite material is $-0.102 \times Wt + 0.149 \times Wg + 0.180 \times Wr + 4.49 > 6$.

Another aspect of the present invention is

a recycled polymer composite material containing a recycled polymer material and components other than the recycled polymer material as constituent components,

in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.20 $g/cm^3$ to 1.25 $g/cm^3$,

(II) an elastic modulus of the recycled polymer composite material is $128 \times Wt + 213 \times Wg - 102 \times Wr + 1,081 > 7,500$ MPa,

(III) a flexural strength of the recycled polymer composite material is $-0.375 \times Wt + 1.52 \times Wg - 3.30 \times Wr + 106 > 115$ MPa, and

(IV) an impact of the recycled polymer composite material is $-0.102 \times Wt + 0.149 \times Wg + 0.180 \times Wr + 4.49 > 9.0$.

Another aspect of the present invention is

a recycled polymer composite material containing a recycled polymer material and components other than the recycled polymer material as constituent components,

in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.30 $g/cm^3$ to 1.35 $g/cm^3$,

(II) an elastic modulus of the recycled polymer composite material is $128 \times Wt + 213 \times Wg - 102 \times Wr + 1,081 > 10,000$ MPa,

(III) a flexural strength of the recycled polymer composite material is $-0.375 \times Wt + 1.52 \times Wg - 3.30 \times Wr + 106 > 140$ MPa, and

(IV) an impact of the recycled polymer composite material is $-0.102 \times Wt + 0.149 \times Wg + 0.180 \times Wr + 4.49 > 11.0$.

EFFECT OF THE INVENTION

One aspect of the present invention can provide a polymer composite material having desired physical properties.

BRIEF DESCRIPTION OF THE DRAWINGS

Fig. 1 is a block diagram illustrating a schematic configuration of a first learning device and a first prediction device.
Fig. 2 is a block diagram illustrating a schematic configuration of a second learning device and a second prediction device.
Fig. 3 is a block diagram illustrating a configuration of a composition proposal system according to an embodiment of the present invention.
Fig. 4 is a block diagram illustrating a hardware configuration of a first learning device, a second learning device, a first prediction device, a second prediction device, and a composition proposal system.
Fig. 5 is a flowchart illustrating a first learning method.
Fig. 6 is a flowchart illustrating a first prediction method.
Fig. 7 is a flowchart illustrating a second learning method.
Fig. 8 is a flowchart illustrating a second prediction method.
Fig. 9 is a flowchart illustrating a composition proposal method according to an embodiment of the present invention.
Fig. 10 is a flowchart for illustrating a method for producing a recycled polymer composite material according to an embodiment of the present invention.
Fig. 11 is a diagram showing a relationship between an actual measurement value and a predicted value of a talc amount.
Fig. 12 is a diagram showing a relationship between an actual measurement value and a predicted value of a rubber amount.
Fig. 13 is a diagram showing a relationship between an actual measurement value and a predicted value of a flexural modulus.
Fig. 14 is a diagram showing a relationship between an actual measurement value and a predicted value of flexural strength.
Fig. 15 is a diagram showing a relationship between an actual measurement value and a predicted value of room-temperature Charpy impact strength.
Fig. 16 is a diagram showing a relationship between a predicted value of room-temperature Charpy impact strength and a predicted value of a flexural modulus FM.
Fig. 17 is a diagram showing a relationship between a density and an actual flexural modulus.
Fig. 18 is a diagram showing a relationship between a density and an actual flexural strength.
Fig. 19 is a diagram showing a relationship between a density, an actual room-temperature Charpy impact strength, and a density.

MODE FOR CARRYING OUT THE INVENTION

Hereinafter, embodiments of the present invention will be described in detail. Note that, in order to facilitate understanding of the description, the same components are denoted by the same reference numerals in each of the drawings, and redundant description will be omitted. In addition, "to" indicating a numerical range in the present embodiment means that numerical values described before and after the numerical range are included as a lower limit value and an upper limit value unless otherwise specified.
A composition proposal system according to the present embodiment will be described. The composition proposal system according to the present embodiment proposes a composition or the like of a constituent component of a recycled polymer material contained in a recycled polymer composite material containing the polymer material that is recycled (recycled polymer material) so that the recycled polymer composite material satisfies one or more desired physical property values.
The polymer material contained in the polymer composite material may be formed using a recycled polymer material in addition to a virgin polymer material. Unlike the virgin polymer material, the recycled polymer material may have a different composition depending on a raw material to be recycled, production conditions, a manufacturer, and the like. When a recycled polymer material is used, in order to produce a recycled polymer composite material having desired physical properties, it is required to determine the composition of the recycled polymer composite material in consideration of the composition and the like for each recycled polymer material to be obtained.

The composition proposal system according to the present embodiment first predicts a parameter including a descriptor of the recycled polymer material from a physical property value of the recycled polymer material. Next, the composition proposal system according to the present embodiment predicts a physical property value of the recycled polymer composite material containing a recycled polymer material having a characteristic of a predicted parameter, and optimizes the parameter of the recycled polymer composite material based on a prediction result of the physical property value. Accordingly, the composition proposal system according to the present embodiment can propose an optimum descriptor such as a composition of a constituent component (for example, a recycled polymer material or the like) contained in a recycled polymer composite material having a desired physical property value.

Note that, in the present embodiment, the polymer composite material is a composite material (composition) containing a polymer material and a filler as constituent components. The polymer composite material may be a virgin polymer composite material, a recycled polymer composite material, or both a virgin polymer composite material and a recycled polymer composite material.

In the present specification, the virgin polymer material, the recycled polymer material, the virgin polymer composite material, and the recycled polymer composite material are defined as follows.

The "virgin polymer material" is a material containing a polymer produced using only a new raw material (virgin polymer), and may be composed of only a virgin polymer.

The "recycled polymer material" is a material containing a polymer produced by containing a recycled raw material (recycled polymer), and may contain a recycled polymer and another component, or may be composed only of a recycled polymer. The recycled polymer material may be a polymer material produced only from a recycled raw material, or may be a polymer material produced by containing a new raw material and a recycled raw material. The recycled polymer material may be a mixture containing, in addition to the same polymer as the virgin polymer, components other than the polymer (for example, a polyolefin elastomer and a filler) depending on the type of the polymer to be recycled. For example, when the recycled polymer material is a propylene-based polymer, the recycled polymer material may constitute a mixture containing a propylene-based polymer and a component (for example, a polyolefin elastomer and a filler) other than the propylene-based polymer.

The "virgin polymer composite material" refers to a virgin composite material containing a new virgin polymer material as a polymer material and not containing a recycled polymer material. The virgin polymer composite material is a mixture (composition) containing a virgin polymer material and a filler as constituent components, and may be a virgin fiber reinforced composite material containing a virgin polymer material and a fibrous filler as constituent components.

The "recycled polymer composite material" contains a recycled polymer material as a polymer material, and is a mixture containing a recycled polymer material and components other than the recycled polymer material as constituent components. The "recycled polymer composite material" contains a recycled polymer material as a polymer material, and may be a mixture containing a recycled polymer material and a filler not derived from recycling as constituent components, or may be a recycled fiber reinforced composite material containing a recycled polymer material and a fibrous filler not derived from recycling as constituent components. The recycled polymer composite material includes a case where a polymer material is produced only from a recycled polymer material and a case where a polymer material is produced by containing a virgin polymer material and a recycled polymer material. The "recycled polymer composite material" may be a mixture containing a recycled polymer material and components other than the recycled polymer material as constituent components, and the "recycled polymer composite material" may be a mixture containing a recycled polymer material, components other than the recycled polymer material, and a filler derived from recycling as constituent components.

Here, in describing the composition proposal system according to the present embodiment, the polymer composite material will be described.

<Polymer composite material>

The polymer composite material is a mixture (composition) containing a polymer material and a filler as constituent components, and may be a fiber reinforced composite material containing a polymer material and a fibrous filler as constituent components. The polymer composite material may be a molded product molded into a predetermined shape according to an application thereof and the like. In the present embodiment, a case where the polymer material is a propylene-based resin composition containing a propylene-based polymer will be described. Note that the polymer material is not limited to a polypropylene-based resin composition, and may be a composition containing a polymer other than a propylene-based polymer.

[Polypropylene-based resin composition]

The polypropylene-based resin composition which is a polymer material contains at least one propylene-based polymer (A).

(Propylene-based polymer (A))

The propylene-based polymer (A) is a polymer having a monomer unit derived from propylene. Examples of the propylene-based polymer (A) include a propylene homopolymer, a random copolymer of propylene and a monomer other than propylene, and a heterophasic propylene polymerization material. The propylene-based resin composition may contain only one kind or two or more kinds of the propylene-based polymer (A). From the viewpoint of rigidity and impact resistance of a molded body of the propylene-based resin composition, the propylene-based polymer (A) preferably contains at least one selected from the group consisting of a propylene homopolymer and a heterophasic propylene polymerization material.

((Propylene homopolymer))

When the propylene-based polymer (A) contains a propylene homopolymer, a limiting viscosity number ($\eta$) of the propylene homopolymer is preferably 0.10 dL/g to 2.00 dL/g from the viewpoint of fluidity during melting of the propylene-based resin composition and toughness of the molded body of the propylene-based composition.

Note that, in the present embodiment, the limiting viscosity number (unit: dL/g) is a value measured at a temperature of 135°C using tetralin as a solvent by the following method.

A reduced viscosity is measured at three points of concentrations of 0.1 g/dL, 0.2 g/dL, and 0.5 g/dL using an Ubbelohde viscometer. The limiting viscosity number is determined by an extrapolation method in which a reduced viscosity is plotted with respect to a concentration and the concentration is extrapolated to zero. A method for calculating the limiting viscosity number by the extrapolation method is described, for example, in "Polymer solution, Polymer Experiment 11" (published by KYORITSU SHUPPAN CO., LTD., 1982), page 491.

A molecular weight distribution (Mw/Mn) of the propylene homopolymer is preferably 3.0 or more. A molecular weight distribution of the propylene-based polymer (A) is preferably 3.0 to 30.0.

In the present embodiment, the molecular weight distribution refers to a ratio of a weight average molecular weight (Mw) to a number average molecular weight (Mn) (Mw/Mn), which is calculated using the weight average molecular weight (Mw) and the number average molecular weight (Mn) measured by gel permeation chromatography (GPC) under the following conditions.

((Conditions))

    Apparatus: manufactured by Tosoh Corporation HLC-8121 GPC/HT
    Separation column: manufactured by Tosoh Corporation
    GMHHR-H(S)HT 3 columns
    Measurement temperature: 140°C
    Carrier: orthodichlorobenzene
    Flow rate: 1.0 mL/min
    Sample concentration: about 1 mg/mL
    Sample injection amount: 400 $\mu$L
    Detector: differential refractometer
    Calibration curve creation method: standard polystyrene is used

The propylene homopolymer can be produced, for example, by polymerizing propylene using a polymerization catalyst.

Examples of the polymerization catalyst include a Ziegler catalyst; a Ziegler-Natta catalyst; a catalyst containing a compound of a transition metal of Group 4 in the periodic table having a cyclopentadienyl ring and alkylaluminoxane; a catalyst containing a compound of a transition metal of Group 4 in the periodic table having a cyclopentadienyl ring, a compound that reacts with the transition metal compound to form an ionic complex, and an organoaluminum compound; and a modified catalyst formed by supporting catalytic components (a compound of a transition metal of Group 4 in the periodic table having a cyclopentadienyl ring, a compound that forms an ionic complex, an organoaluminum compound, and the like) on inorganic particles (silica, clay minerals, and the like).

Examples of a polymerization method include bulk polymerization, solution polymerization, and gas phase polymerization.

Examples of a polymerization manner include a batch manner, a continuous manner, and a combination thereof. The polymerization manner may be a multistage manner in which a plurality of polymerization reaction tanks are connected in series.

Various conditions (a polymerization temperature, a polymerization pressure, a monomer concentration, a catalyst loading amount, a polymerization time, and the like) in a polymerization step may be appropriately determined according to the molecular structure of the target polymer.

After the polymerization step, the polymer may be dried at a temperature equal to or lower than a temperature at which the polymer melts as necessary in order to remove a residual solvent contained in the polymer, an ultra-low molecular weight oligomer by-produced during production, and the like.

((Random copolymer of propylene and monomer other than propylene))

A random copolymer of propylene and a monomer other than propylene contains a monomer unit derived from propylene and a monomer unit derived from a monomer other than propylene. The random copolymer preferably contains 0.01 mass% to 20 mass% of a monomer unit derived from a monomer other than propylene based on the mass of the random copolymer.

The monomer other than propylene is a monomer derived from ethylene or the like. Examples of the monomer other than propylene include ethylene and an α-olefin having 4 to 12 carbon atoms. Among them, at least one selected from ethylene and an α-olefin having 4 to 10 carbon atoms is preferable, and at least one selected from the group consisting of ethylene, 1-butene, 1-hexene, and 1-octene is more preferable.

Note that the "α-olefin" means an aliphatic unsaturated hydrocarbon having a carbon-carbon unsaturated double bond at the α-position.

Examples of the random copolymer include a propylene-ethylene random copolymer, a propylene-1-butene random copolymer, a propylene-1-hexene random copolymer, a propylene-1-octene random copolymer, a propylene-ethylene-1-butene random copolymer, a propylene-ethylene-1-hexene random copolymer, and a propylene-ethylene-1-octene random copolymer.

When the propylene-based polymer (A) contains a random copolymer of propylene and a monomer other than propylene, a limiting viscosity number (η) of the random copolymer is preferably, for example, 1.00 dL/g to 10.00 dL/g, from the viewpoint of fluidity during melting of the propylene-based resin composition.

A molecular weight distribution (Mw/Mn) of the random copolymer is preferably 3.0 to 30.0.

The random copolymer can be produced, for example, by polymerizing propylene and a monomer other than propylene according to a polymerization catalyst, a polymerization method, and a polymerization manner that can be used in production of a propylene homopolymer.

((Heterophasic propylene polymerization material))

A heterophasic propylene polymerization material contains a polymer (I) containing 80 mass% or more of a monomer unit derived from propylene (provided that a total mass of the polymer (I) is 100 mass%), and a polymer (II) containing a monomer unit derived from at least one α-olefin selected from the group consisting of ethylene and α-olefins having 4 to 12 carbon atoms and a monomer unit derived from propylene. The heterophasic propylene polymerization material means a mixture having a structure in which the polymer (II) is dispersed in a matrix of the polymer (I).

The heterophasic propylene polymerization material can be produced, for example, by performing a first polymerization step of forming a polymer (I) and a second polymerization step of forming a polymer (II). Examples of the polymerization catalyst, the polymerization method, and the polymerization manner adopted in these polymerization steps are the same as those described above.

The polymer (I) may be, for example, a propylene homopolymer or may contain a monomer unit derived from a monomer other than propylene. When the polymer (I) contains a monomer unit derived from a monomer other than propylene, a content thereof may be, for example, 0.01 mass% or more and less than 20 mass% based on the total mass of the polymer (I).

Examples of the monomer other than propylene include ethylene and an α-olefin having 4 or more carbon atoms. Among them, at least one selected from the group consisting of ethylene and an α-olefin having 4 to 10 carbon atoms is preferable, and at least one selected from the group consisting of ethylene, 1-butene, 1-hexene, and 1-octene is more preferable.

Examples of the polymer containing a monomer unit derived from a monomer other than propylene include a propylene-ethylene copolymer, a propylene-1-butene copolymer, a propylene-1-hexene copolymer, a propylene-1-octene copolymer, a propylene-ethylene-1-butene copolymer, a propylene-ethylene-1-hexene copolymer, and a propylene-ethylene-1-octene copolymer.

From the viewpoint of dimensional stability of a molded body of the propylene-based resin composition, the polymer (I) is preferably a propylene homopolymer, a propylene-ethylene copolymer, a propylene-1-butene copolymer, or a propylene-ethylene-1-butene copolymer, and more preferably a propylene homopolymer.

A content of the polymer (I) is preferably 50 mass% to 99 mass% with respect to a total mass of the heterophasic propylene polymerization material.

The polymer (II) preferably contains 40 mass% or more of a monomer unit derived from at least one α-olefin selected from the group consisting of ethylene and an α-olefin having 4 to 12 carbon atoms, and contains a monomer unit derived from propylene.

In the polymer (II), a content of the monomer unit derived from at least one α-olefin selected from the group consisting of ethylene and an α-olefin having 4 to 12 carbon atoms may be 40 to 70 mass%, or may be 45 mass% to 60 mass%.

In the polymer (II), the at least one α-olefin selected from the group consisting of ethylene and an α-olefin having 4 to 12 carbon atoms is preferably at least one selected from the group consisting of ethylene and an α-olefin having 4 to 10 carbon atoms, more preferably at least one selected from the group consisting of ethylene, 1-butene, 1-hexene, 1-octene, and 1-decene, and still more preferably at least one selected from the group consisting of ethylene and 1-butene.

Examples of the polymer (II) include a propylene-ethylene copolymer, a propylene-ethylene-1-butene copolymer, a propylene-ethylene-1-hexene copolymer, a propylene-ethylene-1-octene copolymer, a propylene-ethylene-1-decene copolymer, a propylene-1-butene copolymer, a propylene-1-hexene copolymer, a propylene-1-octene copolymer, and a propylene-1-decene copolymer. Among them, a propylene-ethylene copolymer, a propylene-1-butene copolymer, and a propylene-ethylene-1-butene copolymer are preferable, and a propylene-ethylene copolymer is more preferable.

A content of the polymer (II) is preferably 1 mass% to 50 mass% based on the total mass of the heterophasic propylene polymerization material.

The heterophasic propylene polymerization material may contain a xylene-insoluble (CXIS) component and a xylene-soluble (CXS) component.

A content of the CXIS component in the heterophasic propylene polymerization material is preferably 50 mass% to 99 mass% with respect to the total mass of the heterophasic propylene polymerization material.

A content of the CXS component in the heterophasic propylene polymerization material is preferably 1 mass% to 50 mass% with respect to the total mass of the heterophasic propylene polymerization material.

In the present embodiment, the CXIS component in the heterophasic propylene polymerization material is mainly composed of the polymer (I), and the CXS component in the heterophasic propylene polymerization material is mainly composed of the polymer (II).

Examples of the heterophasic propylene polymerization material include a (propylene)-(propylene-ethylene) polymerization material, a (propylene)-(propylene-ethylene-1-butene) polymerization material, a (propylene)-(propylene-ethylene-1-hexene) polymerization material, a (propylene)-(propylene-ethylene-1-octene) polymerization material, a (propylene)-(propylene-1-butene) polymerization material, a (propylene)-(propylene-1-hexene) polymerization material, a (propylene)-(propylene-1-octene) polymerization material, a (propylene)-(propylene-1-decene) polymerization material, a (propylene-ethylene)-(propylene-ethylene) polymerization material, a (propylene-ethylene)-(propylene-ethylene-1-butene) polymerization material, a (propylene-ethylene)-(propylene-ethylene-1-hexene) polymerization material, a (propylene-ethylene)-(propylene-ethylene-1-octene) polymerization material, a (propylene-ethylene)-(propylene-ethylene-1-decene) polymerization material, a (propylene-ethylene)-(propylene-1-butene) polymerization material, a (propylene-ethylene)-(propylene-1-hexene) polymerization material, a (propylene-ethylene)-(propylene-1-octene) polymerization material, a (propylene-ethylene)-(propylene-1-decene) polymerization material, a (propylene-1-butene)-(propylene-ethylene) polymerization material, a (propylene-1-butene)-(propylene-ethylene-1-butene) polymerization material, a (propylene-1-butene)-(propylene-ethylene-1-hexene) polymerization material, a (propylene-1-butene)-(propylene-ethylene-1-octene) polymerization material, a (propylene-1-butene)-(propylene-ethylene-1-decene) polymerization material, a (propylene-1-butene)-(propylene-1-butene) polymerization material, a (propylene-1-butene)-(propylene-1-hexene) polymerization material, a (propylene-1-butene)-(propylene-1-octene) polymerization material, a (propylene-1-butene)-(propylene-1-decene) polymerization material, a (propylene-1-hexene)-(propylene-1-hexene) polymerization material, a (propylene-1-hexene)-(propylene-1-octene) polymerization material, a (propylene-1-hexene)-(propylene-1-decene) polymerization material, a (propylene-1-octene)-(propylene-1-octene) polymerization material, and a (propylene-1-octene)-(propylene-1-decene) polymerization material.

Note that the "(propylene)-(propylene-ethylene) polymerization material" means a "heterophasic propylene polymerization material in which a polymer (I) is a propylene homopolymer and a polymer (II) is a propylene-ethylene copolymer". The same applies to other similar expressions.

A limiting viscosity number of the polymer (I) (ηI) is preferably, for example, 0.10 dL/g to 2.00 dL/g.

A limiting viscosity number of the polymer (II) (ηII) is preferably 1.00 dL/g to 10.00 dL/g.

A ratio of the limiting viscosity number of the polymer (II) (ηII) to the limiting viscosity number of the polymer (I) (ηI) ([η]II/[η]I) is preferably 1 to 20.

Examples of a method for measuring the limiting viscosity number of the polymer (I) (ηI) include a method for measuring the limiting viscosity number of the polymer after forming the polymer (I).

The limiting viscosity number of the polymer (II) (ηII) can be calculated by the following Equation (I) using, for example, the limiting viscosity number of the heterophasic propylene polymerization material (ηTotal), the limiting viscosity number of the polymer (I) ($\eta$I), and the contents of the polymer (II) and the polymer (I).

$$\eta II = (\eta Total - \eta I \times XI)/XII \cdots (I)$$

Note that, in Equation (I), $\eta$Total, $\eta$I, XI, and XII have the following meanings.

$\eta$Total: limiting viscosity number (dL/g) of heterophasic propylene polymerization material
$\eta$I: limiting viscosity number (dL/g) of polymer (I)
XI: ratio of mass of polymer (I) to total mass of heterophasic propylene polymerization material (mass of polymer (I)/mass of heterophasic propylene polymerization material)
XII: ratio of mass of polymer (II) to total mass of heterophasic propylene polymerization material (mass of polymer (II)/mass of heterophasic propylene polymerization material)

Here, XI and XII can be determined from a mass balance during polymerization.
Note that XII may be calculated by measuring the heat of fusion of the polymer (I) and the heat of fusion of the heterophasic propylene polymerization material and using the following Equation (II).

$$XII = 1 - (\Delta Hf)T/(\Delta Hf)P \cdots (II)$$

In Equation (II), $(\Delta Hf)T$ and $(\Delta Hf)P$ have the following meanings.

$(\Delta Hf)T$: heat of fusion (J/g) of heterophasic propylene polymerization material
$(\Delta Hf)P$: heat of fusion (J/g) of polymer (I)

A limiting viscosity number of the CXIS component ($\eta$CXIS) is preferably 0.10 dL/g to 2.00 dL/g.
A limiting viscosity number of the CXS component ($\eta$CXS) is preferably 1.00 dL/g to 10.00 dL/g.
A ratio of the limiting viscosity number of the CXS component ($\eta$CXS) to the limiting viscosity number of the CXIS component ($\eta$CXIS) ($\eta$CXS/$\eta$CXIS) is preferably 1 to 20.
A molecular weight distribution of the polymer (I) (Mw(I)/Mn(I)) is preferably 3.0 or more.
A molecular weight distribution of the CXIS component (Mw(CXIS)/Mn(CXIS)) is preferably 3.0 or more.
A melt flow rate (MFR) of the propylene-based polymer (A) at a temperature of 230°C and a load of 2.16 kgf is preferably 5 g/10 min to 300 g/10 min from the viewpoint of molding processability of the propylene-based resin composition.
Note that, in the present embodiment, the MFR refers to a value measured in accordance with JIS K7210.

(Polyolefin elastomer (B))

The propylene-based resin composition may contain a polyolefin elastomer (B). In the polyolefin elastomer (B), a total of a content of a monomer unit derived from ethylene and a content of a monomer unit derived from an $\alpha$-olefin having 4 or more carbon atoms contained in the polyolefin elastomer (B) may be 100 mass% with respect to 100 mass% of a total mass of the polyolefin elastomer (B).
Examples of the $\alpha$-olefin having 4 or more carbon atoms include an $\alpha$-olefin having 4 to 12 carbon atoms. Examples of the $\alpha$-olefin having 4 to 12 carbon atoms include 1-butene, 1-pentene, 1-hexene, 4-methyl-1-pentene, 1-octene, and 1-decene. Among them, 1-butene, 1-hexene, and 1-octene are preferable. The $\alpha$-olefin may be an $\alpha$-olefin having a cyclic structure such as vinylcyclopropane or vinylcyclobutane.
Examples of the polyolefin elastomer (B) include an ethylene-1-butene copolymer, an ethylene-1-hexene copolymer, an ethylene-1-octene copolymer, an ethylene-1-decene copolymer, an ethylene-(3-methyl-1-butene) copolymer, and a copolymer of ethylene and an $\alpha$-olefin having a cyclic structure (ethylene-$\alpha$-olefin copolymer).
Note that the ethylene-$\alpha$-olefin copolymer is a copolymer containing a monomer unit derived from ethylene and a monomer unit derived from an $\alpha$-olefin having 4 or more carbon atoms, and means a copolymer not containing a monomer unit derived from propylene.
In the polyolefin elastomer (B), a content of the monomer unit derived from an $\alpha$-olefin having 4 or more carbon atoms is preferably 1 mass% to 49 mass% based on the total mass of the polyolefin elastomer (B).
An MFR of the polyolefin elastomer (B) at a temperature of 190°C and a load of 2.16 kgf is preferably 0.1 g/10 min to 80 g/10 min.
A density of the polyolefin elastomer (B) is preferably 0.850 g/cm$^3$ to 0.890 g/cm$^3$ from the viewpoint of impact resistance of the molded body of the propylene-based resin composition.

The polyolefin elastomer (B) can be produced by polymerizing ethylene and an $\alpha$-olefin having 4 or more carbon atoms using a polymerization catalyst.

Examples of the polymerization catalyst include a homogeneous catalyst represented by a metallocene catalyst and a Ziegler-Natta catalyst.

Examples of the homogeneous catalyst include a catalyst containing a compound of a transition metal of Group 4 in the periodic table having a cyclopentadienyl ring and alkylaluminoxane; a catalyst containing a compound of a transition metal of Group 4 in the periodic table having a cyclopentadienyl ring, a compound that reacts with the transition metal compound to form an ionic complex, and an organoaluminum compound; and a modified catalyst formed by supporting catalytic components (a compound of a transition metal of Group 4 in the periodic table having a cyclopentadienyl ring, a compound that forms an ionic complex, an organoaluminum compound, and the like) on inorganic particles (silica, clay minerals, and the like).

Examples of the Ziegler-Natta type catalyst include a catalyst in which a titanium-containing solid transition metal component and an organometallic component are combined.

As the polyolefin elastomer (B), a commercially available product may be used. Examples of the commercially available product include ENGAGE (registered trademark) manufactured by Dow Chemical Japan Ltd., TAFMER (registered trademark) manufactured by Mitsui Chemicals, Inc., NEO-ZEX (registered trademark) and ULT-ZEX (registered trademark) manufactured by Prime Polymer Co., Ltd., and EXCELLEN FX (registered trademark), SUMICASEN (registered trademark), and ESPRENE SPO (registered trademark) manufactured by Sumitomo Chemical Co., Ltd.

(Filler (C))

The propylene-based resin composition may contain a filler (C). The filler (C) contained in the propylene-based resin composition has, for example, a function of enhancing dimensional stability of the molded body of the propylene-based resin composition. Examples of the filler (C) include an inorganic filler and an organic filler. The propylene-based resin composition may contain only one kind or two or more kinds of the fillers (C).

Examples of the inorganic filler include glass, hydrated magnesium silicate (talc ($3MgO\cdot4SiO_2\cdot H_2O$)), silicate mineral, alumina, silica, silicon dioxide, titanium oxide, iron oxide, aluminum oxide, magnesium oxide, antimony oxide, barium ferrite, strontium ferrite, beryllium oxide, magnesium hydroxide, aluminum hydroxide, basic magnesium carbonate, calcium carbonate, magnesium carbonate, carbonate mineral, calcium sulfate, magnesium sulfate, basic magnesium sulfate, calcium sulfite, carbon black, and cadmium sulfide.

Examples of the organic filler include polyester, aromatic polyamide, cellulose, and vinylon.

The filler (C) is preferably an inorganic filler, and more preferably a filler (talc) which is a plate-shaped silicate mineral, from the viewpoint of rigidity, impact resistance, and dimensional stability of the molded body.

The shape of the filler may be a plate shape, a fiber shape, or a needle shape.

An average particle diameter $D_{50}$ of the filler (C) may be, for example, 0.5 $\mu$m to 20.0 $\mu$m from the viewpoint of rigidity, impact resistance, and dimensional stability of the molded body.

Note that the average particle diameter $D_{50}$ is determined based a volume-based particle diameter distribution measurement data measured by a laser diffraction method or a centrifugal precipitation method according to a method specified in JIS R1629, and in the particle diameter distribution measurement data, the average particle diameter $D_{50}$ refers to a particle diameter (particle diameter equivalent to 50%) when a cumulative number of particles from a smaller particle diameter side reaches 50%. The particle diameter defined as described above is generally referred to as a "50% equivalent particle diameter" and is denoted by "D50".

A content of the filler (C) may be 10 parts by mass to 60 parts by mass with respect to 100 parts by mass of a total of the propylene-based polymer (A) and the polyolefin elastomer (B) from the viewpoint of the dimensional stability of the molded body of the propylene-based resin composition.

The propylene-based resin composition may contain components other than the above components. Examples of such components include neutralizing agents, antioxidants, ultraviolet absorbers, nucleating agents, lubricants, antistatic agents, antiblocking agents, processing aids, organic peroxides, colorants (inorganic pigments, organic pigments, pigment dispersants, and the like), foaming agents, nucleating agents, plasticizers, flame retardants, crosslinking agents, crosslinking aids, brightness enhancers, antibacterial agents, and light diffusing agents. The propylene-based resin composition may contain only one kind or two or more kinds of these components.

[Fibrous filler]

The fibrous filler is a filler in which an inorganic filler or an organic filler is formed into a fibrous shape. As the inorganic filler or the organic filler, the inorganic filler or the organic filler used in the filler (C) may be used. The fibrous filler is preferably a fibrous inorganic filler in which an inorganic filler is formed into a fibrous shape. As the fibrous inorganic filler, for example, a glass fiber, a carbon fiber, or the like is used.

When the polymer composite material is a recycled polymer composite material, the recycled polymer composite material contains a recycled polymer and components other than the recycled polymer as constituent components, and when a filler (talc) amount in the recycled polymer composite material is Wt, a glass fiber amount in the recycled polymer composite material is Wg, and a rubber amount in the recycled polymer composite material is Wr, the following parameters (I) to (IV) are satisfied:

(I): a density of the recycled polymer composite material is 1.08 g/cm$^3$ to 1.16 g/cm$^3$,
(II) an elastic modulus of the recycled polymer composite material is $128 \times Wt + 213 \times Wg - 102 \times Wr + 1,081 > 5,000$ MPa,
(III) a flexural strength of the recycled polymer composite material is $-0.375 \times Wt + 1.52 \times Wg - 3.30 \times Wr + 106 > 80$ MPa, and
(IV) an impact of the recycled polymer composite material is $-0.102 \times Wt + 0.149 \times Wg + 0.180 \times Wr + 4.49 > 6$.

In describing the composition proposal system according to the present embodiment, a learning device that generates a learned model used in the composition proposal system and a prediction device that performs prediction using the generated learned model will be described. The learning device and the prediction device include a first learning device and a first prediction device, and a second learning device and a second prediction device as two learning devices and prediction devices having different explanatory variables and objective variables.

<First learning device>

The first learning device will be described. Note that, here, the propylene-based polymer (A) which is a polymer material contained in the polymer composite material is a heterophasic propylene polymerization material, and may contain an elastomer, and the filler is talc. In addition, the filler used in a first prediction device 2 to be described below is also similar to that in a first learning device 1.
In addition, the polymer material or the polymer composite material used in the first learning device 1 is a virgin polymer material or a virgin polymer composite material, and a physical property value of the polymer material or the polymer composite material and a parameter including a descriptor of the polymer material or the polymer composite material are a physical property value of the virgin polymer material or the virgin polymer composite material and a parameter including a descriptor of the virgin polymer material or the virgin polymer composite material.

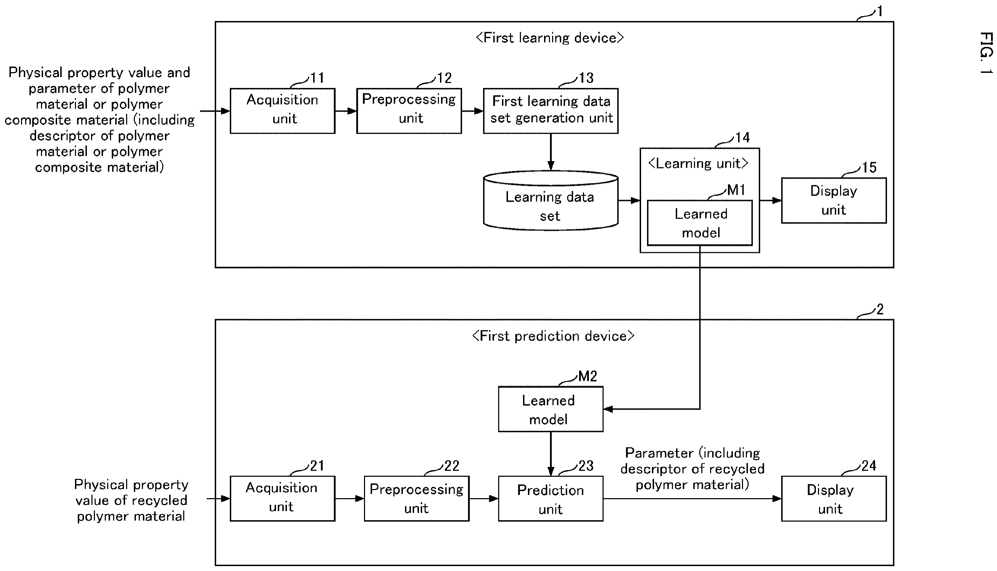

Fig. 1 is a block diagram illustrating a schematic configuration of a learning device. As illustrated in Fig. 1, a first learning device 1 includes an acquisition unit 11, a preprocessing unit 12, a learning data set generation unit 13, a learning unit 14, and a display unit 15. The first learning device 1 generates a learned model M1 for predicting a parameter including a descriptor of a polymer material or a polymer composite material from a physical property value of a polymer material or a polymer composite material.
In the first learning device 1, when the input physical property value is a physical property value of the polymer material, a learned model M1 for predicting a parameter including a descriptor of the polymer material is generated. When the input physical property value is a physical property value of the polymer composite material, a learned model M1 for predicting a parameter including a descriptor of the polymer composite material is generated.
Here, a case where the input physical property value is a physical property value of the polymer composite material and the predicted parameter is a parameter including a descriptor of the polymer composite material will be described; however, when the polymer composite material to be used does not contain a filler, the "polymer composite material" used for description of the first learning device 1 is regarded as a "polymer material".
The acquisition unit 11 acquires a physical property value of the polymer composite material as an explanatory variable, and acquires a parameter including a descriptor of the polymer composite material as an objective variable.
Examples of the physical property value of the polymer composite material used as an explanatory variable in the first learning device 1 include moldability (fluidity), density, yield stress, tensile strength, tensile modulus, flexural strength, deflection temperature under load, Rockwell hardness, rigidity, impact resistance, and glossiness. Note that the moldability can be evaluated by the MFR or the like of the polymer composite material. The impact resistance can be evaluated by IZOD impact strength at room temperature or a low temperature and a Charpy impact test.
Examples of the descriptor of the polymer composite material used as the objective variable in the first learning device 1 include a mass ratio of constituent components contained in the polymer composite material, and a characteristic value and a structural value for each type of constituent components.
The descriptor of the polymer composite material includes at least one of a mass ratio of constituent components contained in the polymer composite material, and a characteristic value and a structural value for each type of constituent components.
The constituent components include one kind of polymer as an essential component, and may include components other than the kind of polymer as optional components. A plurality of the same polymers and a plurality of components other than the same polymer may be contained. Since the constituent component includes one type of polymer, the characteristic value and the structural value for each type of constituent components may be the characteristic value and the structural value of one type of polymer.

The mass ratio of the constituent components is a composition of the constituent components and a ratio thereof. For example, the mass ratio is a ratio of each of the heterophasic propylene polymerization material, the polyolefin elastomer (B), and the filler (C) contained in the polymer composite material. The mass ratio of the constituent components includes a talc amount, a rubber amount, a glass fiber amount, and the like contained in the polymer composite material. The mass ratio of the constituent components may include one or more of the talc amount, the rubber amount, and the glass fiber amount.

Examples of the characteristic value for each type of the constituent components include a limiting viscosity and an MFR of the constituent component.

The limiting viscosity of the constituent components includes a limiting viscosity of at least one polymer material included in the polymer composite material. The limiting viscosity of the constituent components is, for example, a limiting viscosity of the heterophasic propylene polymerization material and the polyolefin elastomer (B) contained in the polymer composite material.

The limiting viscosity of the heterophasic propylene polymerization material is a limiting viscosity of at least one of the polymer (I) and the polymer (II) constituting the heterophasic propylene polymerization material, and may be a limiting viscosity of both the polymer (I) and the polymer (II).

The structural value for each type of constituent components is a molecular weight of the constituent component, a content of the constituent component, a monomer unit ratio of the copolymer contained in the constituent component, a composition ratio of the copolymer, and the like.

The structural value preferably includes at least one of a content of the constituent components and a monomer unit ratio of the copolymer contained in the constituent component. The monomer unit ratio of the copolymer may be a monomer unit ratio of the copolymer in the copolymer (for example, the heterophasic propylene polymerization material, the polyolefin elastomer (B), and the like) containing a monomer unit derived from ethylene.

A molecular weight of the constituent components includes a molecular weight of the polymer material and other polymers. The molecular weight of the constituent components is, for example, a molecular weight of the heterophasic propylene polymerization material, the polyolefin elastomer (B), and the like contained in the polymer composite material.

The molecular weight of the heterophasic propylene polymerization material is a molecular weight of at least one of the polymer (I) and the polymer (II) contained in the heterophasic propylene polymerization material, and may be a molecular weight of both the polymer (I) and the polymer (II), or may be a molecular weight of the polymer (I) or the polymer (II).

A content of the constituent components includes a content of the polymer material and other polymers. The content of the constituent components is, for example, a content of the heterophasic propylene polymerization material, the polyolefin elastomer (B), and the filler (C) contained in the polymer composite material.

The content of the heterophasic propylene polymerization material is a content of at least one of the polymer (I) and the polymer (II) constituting the heterophasic propylene polymerization material, and may be a content of only the polymer (II).

The monomer unit ratio of the copolymer contained in the constituent components is, for example, an ethylene amount contained in the heterophasic propylene polymerization material contained in the polymer composite material and an ethylene amount contained in the polyolefin elastomer (B). Note that the ethylene amount is a monomer unit ratio of the copolymer in the copolymer containing a monomer unit derived from ethylene such as the heterophasic propylene polymerization material and the polyolefin elastomer (B).

The ethylene amount contained in the heterophasic propylene polymerization material is a content of a structural unit derived from ethylene contained in at least one of the polymer (I) and the polymer (II) contained in the heterophasic propylene polymerization material, and may be only a content of a structural unit derived from ethylene contained in the polymer (II).

The composition ratio of the copolymer contained in the constituent components is a ratio of the composition of the copolymer contained in the heterophasic propylene polymerization material, the polyolefin elastomer (B), and the like contained in the polymer composite material. The composition ratio of the copolymer contained in the heterophasic propylene polymerization material may be a ratio of the polymer (I) and the polymer (II).

When the constituent component is a polymer material, the characteristic value and the structural value for each type of the constituent components preferably include a parameter indicating the characteristic or structure of the polymer material. Therefore, the characteristic value and the structural value may include, for example, a parameter correlated with the molecular weight and the monomer unit ratio of the copolymer as a parameter indicating the characteristic or structure of the terophasic propylene polymerization material, the polyolefin elastomer (B), and the like, which are polymer materials.

Examples of the parameter correlated with the molecular weight include a limiting viscosity and a melt flow rate (MFR).

Examples of the parameter correlated with the monomer unit ratio of the copolymer include a density and a glass transition temperature.

At least one of the characteristic value and the structural value of the constituent components preferably includes an average value for each type of the constituent components, the characteristic value for each type of the constituent components preferably includes an average characteristic value, and the structural value for each type of the constituent components preferably includes an average structural value.

That is, the molecular weight of the constituent components, the content of the constituent components, the limiting viscosity of the constituent components, the monomer unit ratio of the copolymers contained in the constituent components, and the ratio of the compositions of the copolymers may be an average molecular weight of the constituent components, an average content of the constituent components, an average limiting viscosity of the constituent components, an average monomer unit ratio of the copolymers contained in the constituent components, and an average ratio of the compositions of the copolymers, respectively.

When the average values of the characteristic values and the structural values are used, the number of parameters including descriptors of the polymer composite material used for learning can be equalized between a case where one polymer material is contained as a constituent component of the polymer composite material and a case where two or more polymer materials are contained, such that the prediction accuracy of the learned model M1 may be improved. For example, a polymer composite material containing two propylene materials is present in the learning data set, and a characteristic value and a structural value of a first component and a characteristic value and a structural value of a second component are used as parameters including descriptors of the polymer composite material. In this case, when a polymer composite material containing one propylene material is present in another learning data set, since a second component of the material is not present, a characteristic value and a structural value of the second component are missing data. When there is missing data in the parameter including the descriptor of the polymer composite material used for learning, the prediction accuracy of the obtained learned model decreases. In such a case, average values of characteristic values and structural values for each component are used as parameters including descriptors of the polymer composite material. Accordingly, the number of parameters including corresponding descriptors can be equalized between a polymer composite material containing two propylene materials and a polymer composite material containing one propylene material, and a defect does not occur in the learning data, such that the prediction accuracy of the learned model is improved.

The descriptor of the polymer composite material may include, as an additional descriptor, a cross term between the calculated characteristic value or structural value (average characteristic value or average structural value) and a mass ratio of each of the components. Note that the characteristic value or the structural value of each component may be an average characteristic value or an average structural value thereof. That is, the descriptor of the polymer composite material may include, as an additional descriptor, a value obtained by adding a product of the calculated characteristic value or structural value and mass ratio of each of the components.

Note that the cross term is effective for expressing an interaction (so-called synergistic effect) between physical property values of the polymer composite material, which are explanatory variables. In a case where a nonlinear model is used, it is not always required to include the nonlinear model; however, in a case where the number of pieces of data in the learning data set is small, there is a case where the nonlinear model contributes to improvement of accuracy; thus, it is preferable to use the cross term. As the descriptor of the polymer composite material creating the cross term, a combination of descriptors that seems to work for the physical properties of the polymer composite material as an objective variable may be appropriately selected, or a combination with high prediction accuracy may be selected by trying all patterns.

The preprocessing unit 12 preprocesses the acquired physical properties (explanatory variables) of the polymer composite material and parameters (objective variables) including the descriptors of the polymer composite material. Note that the first learning device 1 does not need to include the preprocessing unit 12 in a case where the acquisition unit 11 acquires a physical property of the polymer composite material and a parameter including a descriptor of the polymer composite material that are preprocessed in advance.

When the polymer composite materials acquired by the acquisition unit 11 overlap, the preprocessing unit 12 may calculate an average value (median value) of overlapping values of the physical properties of the polymer composite materials and the parameters including the descriptors of the polymer composite materials.

The preprocessing unit 12 may calculate, as the physical property value, an average value of moldability, density, yield stress, tensile strength, tensile modulus, flexural strength, deflection temperature under load, Rockwell hardness, rigidity, impact resistance, glossiness, and the like. The preprocessing unit 12 may calculate average values of characteristic values and structural values for each type of the polymer composite materials as parameters including descriptors of the polymer composite material.

The learning data set generation unit 13 extracts each of the preprocessed physical property of the polymer composite material as an explanatory variable and the preprocessed parameter including the descriptor of the polymer composite material as an objective variable, and adds the extracted variables to the learning data set. The learning data set generation unit 13 creates a learning data set by associating the input physical property of the polymer composite material with the input parameter including the descriptor of the polymer composite material.

Note that, in a case where the preprocessing unit 12 does not perform preprocessing of a physical property of the polymer composite material and a parameter including a descriptor of the polymer composite material, the learning data set generation unit 13 may extract each of the physical property (explanatory variable) of the polymer composite material and the parameter (objective variable) including the descriptor of the polymer composite material acquired by the acquisition unit 11, and may add the extracted variables to the learning data set.

The learning unit 14 generates a learned model M1 by performing learning using a learning data set in which a physical property (explanatory variable) of the polymer composite material and a parameter (objective variable) including a descriptor of the polymer composite material are associated with each other.

The learned model M1 is a learned model in which machine learning is performed in advance using a learning data set (learning data table) stored in a storage unit (not illustrated). The learned model M1 is applied with a learning result of a correspondence relationship between a physical property (explanatory variable) of the polymer composite material and a parameter (objective variable) including a descriptor of the polymer composite material, which is obtained by performing machine learning using a learning data set stored in a storage unit (not illustrated) of the first learning device 1. The learned model M1 is a program for modeling an input/output relationship between a physical property of a polymer composite material and a parameter including a descriptor of the polymer composite material, with the physical property of the polymer composite material as an explanatory variable and the parameter including the descriptor of the polymer composite material as an objective variable. Note that the learned model M1 may be expressed by a mathematical expression such as a function.

As the learned model M1, it is preferable to apply an algorithm of supervised learning among machine learning. Examples of the supervised learning include linear regression, regularized regression, partial least squares regression, polynomial regression, kernel regression, logistic regression, random forest, gradient boosting regression tree, support vector machine (SVM), and neural network. The neural network can use deep learning in which the neural network is multilayered rather than three layers. As the type of the neural network, for example, a convolutional neural network (CNN), a recurrent neural network (RNN), a general regression neural network, or the like can be used.

The display unit 15 displays information on a learning data set used in learning of the learned model M1, information on the learned model M1, and the like.

The first learning device 1 may output a parameter including a descriptor of the polymer composite material in one learned model M1. Therefore, the first learning device 1 preferably has the learned model M1 corresponding to each parameter including the descriptor of the polymer composite material for each parameter including the descriptor of the polymer composite material to be output.

As described above, since the first learning device 1 includes the learning unit 14, the learned model M1 for predicting the parameter including the descriptor of the polymer composite material from the physical property value of the polymer composite material is generated. The learned model M1 generated by the first learning device 1 performs learning to predict a parameter including a descriptor of the polymer composite material from the input physical property value of the polymer composite material.

In addition, since the first learning device 1 generates the learned model M1, by using the first learning device 1, it is possible to reduce the burden and time required for predicting the parameter including the descriptor of the polymer composite material from the physical property value of the polymer composite material. That is, when a polymer composite material is produced, in order to produce a polymer composite material satisfying arbitrary characteristics such as moldability according to the use of the polymer composite material or the like, actually, in experiments, a polymer composite material containing various polymer materials is produced, and physical properties thereof are measured. By performing such a step, the type of the polymer effective for the production of the polymer composite material, the composition of the component, and the like are determined; thus, much effort is required, and the preparation of various materials requires a large burden of cost. The first learning device 1 is used to predict a parameter including a descriptor of the polymer composite material from the physical property of the polymer composite material, thereby efficiently predicting the parameter including the descriptor of the polymer composite material while reducing the burden. Therefore, the first learning device 1 can reduce energy consumption when predicting a parameter including a descriptor of the polymer composite material.

<First prediction device>

The first prediction device will be described. Fig. 1 is a block configuration diagram illustrating a configuration of the prediction device. As illustrated in Fig. 1, a first prediction device 2 includes an acquisition unit 21, a preprocessing unit 22, a learned model M2, a prediction unit 23, and a display unit 24. The first prediction device 2 predicts a parameter including a descriptor of the recycled polymer material from a physical property value of the recycled polymer material.

As described above, the recycled polymer material may contain, in addition to the same polymer as the virgin polymer, components other than the polymer. For example, when the recycled polymer material is a propylene-based polymer, the recycled polymer material may be a mixture containing a propylene-based polymer and, for example, a polyolefin elastomer and a filler as components other than the propylene-based polymer, and the physical properties of the recycled polymer material vary depending on a content of components other than the propylene-based polymer. The first prediction device 2 predicts a descriptor of the recycled polymer material, such as compositions of the recycled polymer and other components contained in the recycled polymer material, from a physical property value of the recycled polymer material.

The acquisition unit 21 acquires a physical property value of the recycled polymer material to be predicted as an explanatory variable. Since the physical property value of the recycled polymer material is the same as the physical property value of the polymer composite material acquired by the first learning device 1 described above, the details thereof will be omitted.

The preprocessing unit 22 performs preprocessing of the acquired physical property value (explanatory variable) of the recycled polymer material to be predicted. Since the details of the preprocessing are similar to those of the preprocessing unit 12 of the first learning device 1, the details thereof will be omitted.

The learned model M2 is learned using a learning data set in which the physical property value (explanatory variable) of the polymer composite material and the parameter (objective variable) including the descriptor of the polymer composite material that are prepared in advance are associated with each other. The learned model M1 generated by the first learning device 1 may be used as the learned model M2.

The prediction unit 23 inputs the physical property value of the recycled polymer material to be predicted acquired by the acquisition unit 21 to the learned model M2, thereby outputting the parameter including the descriptor of the recycled polymer material to be predicted, which is predicted by the learned model M2. That is, the prediction unit 23 predicts, from one learned model M2, a parameter including a descriptor of the recycled polymer material to be predicted, corresponding to one type of physical property value of the recycled polymer material to be predicted.

Since the parameter including the descriptor of the recycled polymer material is similar to the parameter including the descriptor of the polymer composite material acquired by the first learning device 1 described above, the details thereof will be omitted.

In addition, the prediction unit 23 may predict a parameter including a descriptor of the recycled polymer material to be predicted corresponding to a group including physical property values of the recycled polymer material to be predicted by using a plurality of learned models M2. For example, the prediction unit 23 may predict predicted values such as a mass ratio, a characteristic value, and a structural value of the recycled polymer material to be predicted corresponding to a plurality of physical property values of the recycled polymer material to be predicted. Here, the same physical properties of the recycled polymer material may be input, and a predicted value of a parameter including a descriptor of each recycled polymer material may be obtained from each learned model, or the same recycled polymer material to be predicted may pass through an individual preprocessing unit corresponding to each learned model.

In addition, the prediction unit 23 may predict a parameter including a descriptor of the recycled polymer material to be predicted corresponding to a group including a plurality of physical property values of the recycled polymer material to be predicted.

The display unit 24 displays the parameter including the descriptor of the recycled polymer material predicted by the learned model M2 as an objective variable.

As described above, the first prediction device 2 includes the prediction unit 23, and the prediction unit 23 predicts a parameter including a descriptor of the recycled polymer material from the physical property value of the recycled polymer material by the learned model M2. Therefore, the first prediction device 2 predicts a parameter including a descriptor of the recycled polymer material corresponding to the input physical property value of the recycled polymer material.

In addition, the first prediction device 2 includes the prediction unit 23, such that the first prediction device 2 predicts a parameter including a descriptor of the recycled polymer material while reducing the burden and time required for prediction of the parameter including the descriptor of the recycled polymer material from the physical property value of the recycled polymer material. Therefore, since the first prediction device 2 efficiently predicts a parameter including a descriptor of the recycled polymer material, it is possible to reduce energy consumption when predicting the parameter including the descriptor of the recycled polymer material.

<Second learning device>

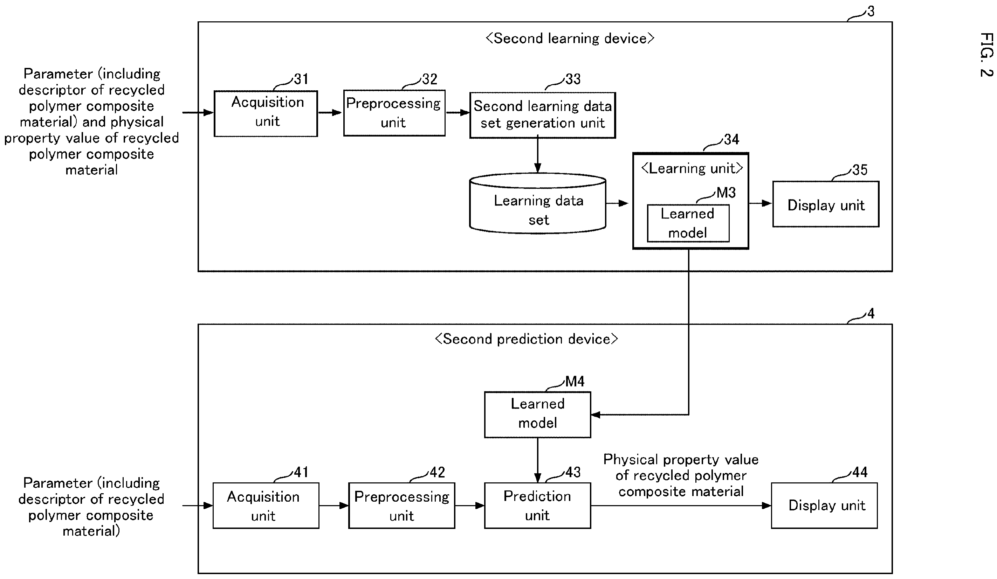

The second learning device will be described. Fig. 2 is a block diagram illustrating a schematic configuration of the second learning device. As illustrated in Fig. 2, a second learning device 3 includes an acquisition unit 31, a preprocessing unit 32, a learning data set generation unit 33, a learning unit 34, and a display unit 35. The second learning device 3 generates a learned model M3 that predicts a physical property value of the recycled polymer composite material from a parameter including a descriptor of the recycled polymer composite material. That is, the second learning device 3 changes the polymer composite material of the explanatory variable and the objective variable of the first learning device 1 illustrated in Fig. 1 to a recycled polymer composite material, and generates a learned model by reversing the explanatory variable and the objective variable of the first learning device 1.

The acquisition unit 31 acquires a parameter including a descriptor of the recycled polymer composite material as an explanatory variable, and acquires a physical property value of the recycled polymer composite material as an objective variable.

The parameter including the descriptor of the recycled polymer composite material acquired as the explanatory variable in the second learning device 3 and the physical property value of the recycled polymer composite material acquired as the objective variable in the second learning device 3 are similar to the parameter including the descriptor of the polymer composite material and the physical property value of the polymer composite material acquired in the first learning device 1 described above, and thus, the details thereof will be omitted.

The preprocessing unit 32 performs preprocessing of the acquired parameter (explanatory variable) including the descriptor of the recycled polymer composite material and the acquired physical property value (objective variable) of the recycled polymer composite material. Since the preprocessing unit 32 is similar to the preprocessing unit 12 of the first learning device 1 except that the explanatory variable and the objective variable are reversed from those of the preprocessing unit 12, the details thereof will be omitted. Note that when the acquisition unit 31 acquires the parameter including the descriptor of the recycled polymer composite material and the physical property value of the recycled polymer material that are preprocessed in advance, the second learning device 3 may not include the preprocessing unit 32.

The learning data set generation unit 33 extracts each of the preprocessed parameter including the descriptor of the recycled polymer composite material as an explanatory variable and the preprocessed physical property value of the recycled polymer composite material as an objective variable, and adds the extracted variables to the learning data set. The learning data set generation unit 33 creates a learning data set by associating the input parameter including the descriptor of the recycled polymer composite material with the physical property value of the recycled polymer composite material.

Note that, in a case where the preprocessing unit 32 does not perform preprocessing of the parameter including the descriptor of the recycled polymer composite material and the physical property value of the recycled polymer composite material, similarly to the preprocessing unit 12 of the first learning device 1, the learning data set generation unit 13 may extract each of the parameter (explanatory variable) including the descriptor of the recycled polymer composite material and the physical property value (objective variable) of the recycled polymer composite material acquired by the acquisition unit 31, and may add the extracted variables to the learning data set.

The learning unit 34 generates the learned model M3 by performing learning using a learning data set in which a parameter (explanatory variable) including a descriptor of the recycled polymer composite material and a physical property value (objective variable) of the recycled polymer composite material are associated with each other.

Since the learned model M3 is similar to the learned model M1 except that the explanatory variable and the objective variable are reversed from those of the learned model M1 of the first learning device 1, the details thereof will be omitted.

The display unit 35 displays information on a learning data set used in learning of the learned model M3, information on the learned model M3, and the like.

The second learning device 3 outputs one physical property value of the recycled polymer composite material with one learned model M3. Therefore, the second learning device 3 preferably has a learned model M3 corresponding to each physical property value of the recycled polymer composite material for each physical property value of the recycled polymer composite material to be output.

As described above, since the second learning device 3 includes the learning unit 34, the second learning device 3 generates the learned model M3 for predicting the physical property value of the recycled polymer composite material. The learned model M3 generated by the second learning device 3 performs learning to predict the physical property value of the recycled polymer composite material from the input parameter including the descriptor of the recycled polymer composite material.

In addition, since the second learning device 3 generates the learned model M3, by using the second learning device 3, it is possible to reduce the burden and time required for predicting the physical property value of the recycled polymer composite material from the parameter including the descriptor of the recycled polymer composite material. The second learning device 3 is used to predict the physical property value of the recycled polymer composite material from the parameter including the descriptor of the recycled polymer composite material, thereby efficiently predicting the physical property value of the recycled polymer composite material while reducing the burden. Therefore, the second learning device 3 can reduce energy consumption when predicting the physical property value of the recycled polymer composite material.

<Second prediction device>

The second prediction device will be described. Fig. 2 is a system configuration diagram illustrating a configuration of the second prediction device. As illustrated in Fig. 2, a second prediction device 4 includes an acquisition unit 41, a preprocessing unit 42, a learned model M4, a prediction unit 43, and a display unit 44. The second prediction device 4 predicts a physical property value of the recycled polymer composite material from a parameter including a descriptor of the recycled polymer composite material. That is, the second prediction device 4 is obtained by reversing the explanatory variable and the objective variable of the first prediction device 2 illustrated in Fig. 1.

The acquisition unit 41 acquires a parameter including a descriptor of the recycled polymer composite material to be predicted as an explanatory variable. Since the parameter including the descriptor of the recycled polymer composite material is similar to the parameter including the descriptor of the recycled polymer composite material acquired by the second learning device 3 described above, the details thereof will be omitted.

The preprocessing unit 42 preprocesses the acquired parameter (explanatory variable) including the descriptor of the recycled polymer composite material to be predicted. Since the details of the preprocessing are similar to those of the preprocessing unit 32 of the second learning device 3, the details thereof will be omitted.

The learned model M4 is learned using a learning data set in which the parameter (explanatory variable) including the descriptor of the recycled polymer composite material and the physical property value (objective variable) of the recycled polymer composite material that are prepared in advance are associated with each other. The learned model M3 generated by the second learning device 3 may be used as the learned model M4.

The prediction unit 43 inputs the parameter including the descriptor of the recycled polymer composite material to be predicted acquired by the acquisition unit 41 to the learned model M4, thereby outputting the physical property value of the recycled polymer composite material to be predicted by the learned model M4. That is, the prediction unit 43 predicts, from one learned model M4, one kind of physical property value of the recycled polymer composite material to be predicted corresponding to the parameter including the descriptor of the recycled polymer composite material to be predicted. Since the prediction unit 43 is similar to the prediction unit 23 except that the explanatory variable input to the prediction unit 23 included in the first prediction device 2 and the objective variable output from the prediction unit 23 are reversed, the details thereof will be omitted.

The display unit 44 displays the physical property value and the like of the recycled polymer composite material predicted by the learned model M4 as an objective variable.

As described above, the second prediction device 4 includes the prediction unit 43, and the prediction unit 43 predicts a physical property value of the recycled polymer composite material from the parameter including the descriptor of the recycled polymer composite material by the learned model M4. Therefore, the second prediction device 4 predicts a physical property value of the recycled polymer composite material from the input parameter including the descriptor of the recycled polymer composite material.

In addition, the second prediction device 4 includes the prediction unit 43 to predict the physical property value of the recycled polymer composite material while reducing the burden and time required for predicting the physical property value of the recycled polymer composite material from the parameter including the descriptor of the recycled polymer composite material. Therefore, since the second prediction device 4 efficiently predicts a physical property value of the recycled polymer composite material, it is possible to reduce energy consumption when predicting the physical property value of the recycled polymer composite material.

<Composition proposal system>

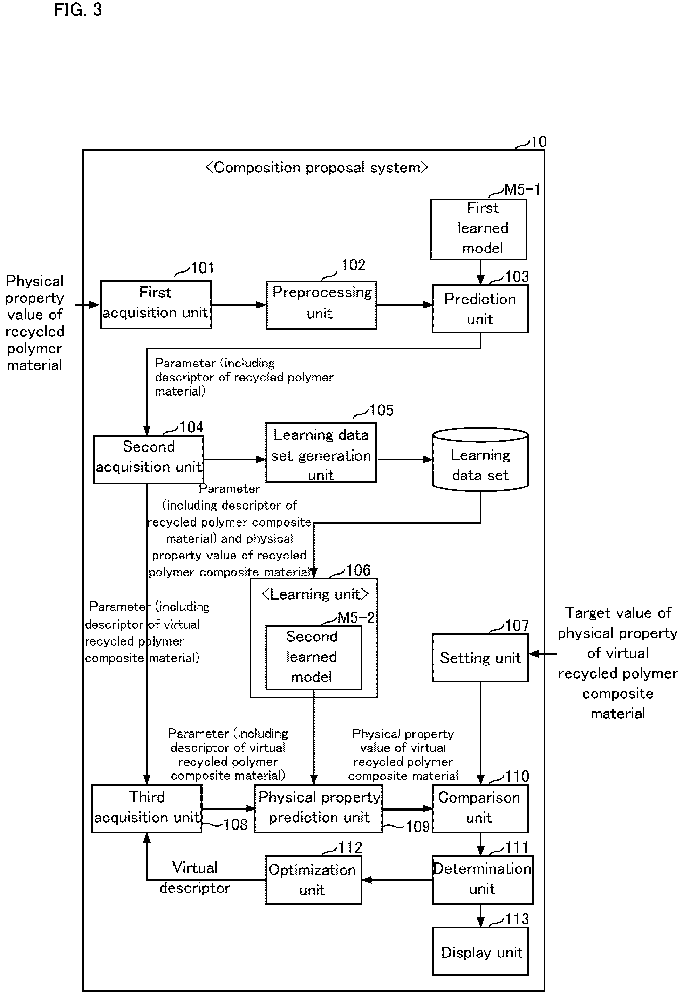

The composition proposal system according to the present embodiment will be described. Fig. 3 is a block diagram illustrating a configuration of the composition proposal system according to an embodiment. As illustrated in Fig. 3, a composition proposal system 10 includes a first acquisition unit 101, a preprocessing unit 102, a prediction unit 103, a second acquisition unit 104, a learning data set generation unit 105, a learning unit 106, a setting unit 107, a third acquisition unit 108, a physical property prediction unit 109, a comparison unit 110, a determination unit 111, an optimization unit 112, a display unit 113, a first learned model M5-1, and a second learned model M5-2. The composition proposal system 10 proposes a composition of a constituent component contained in the recycled polymer composite material so that the recycled polymer composite material containing at least the recycled polymer material as a constituent component satisfies one or more desired physical property values.

The first acquisition unit 101 acquires a physical property value of the recycled polymer material as an explanatory variable.

The physical property value of the recycled polymer material initially input to the first acquisition unit 101 may be a randomly acquired physical property value, and for example, randomly acquired moldability (fluidity), density, yield stress, tensile strength, tensile modulus, flexural strength, deflection temperature under load, Rockwell hardness, rigidity, impact resistance, glossiness, and the like of the recycled polymer material may be used. In addition, the recycled polymer material initially input may be a plurality of groups corresponding to the plurality of recycled polymer materials.

Since the physical property value of the recycled polymer material is the same as the physical property value of the polymer material acquired by the first prediction device 2 or the physical property value of the recycled polymer composite material predicted by the second prediction device 4, the details thereof will be omitted.

The preprocessing unit 102 performs preprocessing of the physical property value of the recycled polymer material acquired by the first acquisition unit 101. Note that, when the physical property value of the recycled polymer material preprocessed in advance is acquired by first acquisition unit 101, the composition proposal system 10 may not include the preprocessing unit 102.

When the physical property values of the recycled polymer materials acquired by the first acquisition unit 101 overlap, the preprocessing unit 102 may calculate an average value (median value) of the overlapping physical property values of the recycled polymer material.

The preprocessing unit 102 may calculate an average value of physical property values for each type of the recycled polymer materials as the physical property value of the recycled polymer material.

The prediction unit 103 inputs the physical property value of the recycled polymer material acquired by the preprocessing unit 102 to the first learned model M5-1, and predicts a parameter including a descriptor of the recycled polymer material. The prediction unit 103 inputs the physical property value of the recycled polymer material acquired by the preprocessing unit 102 to the first learned model M5-1, thereby outputting a parameter including the descriptor of the recycled polymer material predicted by the first learned model M5-1.

The prediction unit 103 may predict a group including parameters including descriptors of the recycled polymer material corresponding to the physical property value of the recycled polymer material. The group including the parameters including the descriptors of the recycled polymer material is a group of predicted values of the parameters the descriptors of the recycled polymer material predicted by the plurality of first learned models M5-1. For example, the prediction unit 103 may predict a group of a mass ratio of the constituent component (for example, a rubber amount, a talc amount, and a glass fiber amount), and a characteristic value and a predicted value of the structural value for each type of the constituent components corresponding to moldability (fluidity), density, yield stress, tensile strength, tensile modulus, flexural strength, deflection temperature under load, Rockwell hardness, rigidity, impact resistance, glossiness, and the like of the recycled polymer material by a plurality of first learned models M5-1.

Since the parameter including the descriptor of the recycled polymer material is similar to the parameter including the descriptor of the recycled polymer material predicted by the first prediction device 2 described above or the parameter including the descriptor of the recycled polymer composite material obtained by the second prediction device 4, the details thereof will be omitted.

The first learned model M5-1 is a learned model learned using a learning data set (first learning data set) in which a physical property value of a virgin polymer composite material containing a virgin polymer material and a parameter including a descriptor of the virgin polymer composite material are associated with each other. The first learned model M5-1 may use the learned model M1 of the first learning device 1 in Fig. 1 or the learned model M2 of the first prediction device 2 in Fig. 1. Since the first learned model M5-1 is similar to the learned model M1 or the learned model M2, the details thereof will be omitted.

The second acquisition unit 104 acquires the parameter including the descriptor of the recycled polymer material predicted by the first learned model M5-1 by the prediction unit 103.

The learning data set generation unit 105 regards the parameter including the descriptor of the recycled polymer material acquired by the second acquisition unit 104 as a parameter including a descriptor of the recycled polymer composite material. The learning data set generation unit 105 adds a parameter including a descriptor of the recycled polymer composite material to the learning data set as an explanatory variable. That is, the learning data set generation unit 105 creates a parameter including a descriptor of the recycled polymer composite material according to the composition ratio on the basis of the parameter including the descriptor of the recycled polymer material acquired by the second acquisition unit 104, and adds the parameter including the descriptor of the recycled polymer composite material to the learning data set as an explanatory variable.

The learning data set may already contain a parameter including a descriptor of the recycled polymer composite material in the past as an explanatory variable and a physical property value of the recycled polymer composite material in the past as an objective variable. The learning data set generation unit 105 can use the learning data set generation unit 33 of the second learning device 3. Since the learning data set generation unit 105 is similar to the learning data set generation unit 33, the details thereof will be omitted.

The learning unit 106 generates the second learned model M5-2 by performing learning using a learning data set in which a parameter (explanatory variable) including a descriptor of the recycled polymer composite material and a physical property value (objective variable) of the recycled polymer composite material are associated with each other.

The second learned model M5-2 is a learned model learned using a learning data set (second learning data set) in which a parameter including a descriptor of the recycled polymer composite material and a physical property value of the recycled polymer composite material are associated with each other. The second learned model M5-2 may use the learned model M3 of the second learning device 3 in Fig. 2 or the learned model M4 of the second prediction device 4 in Fig. 2. Since the second learned model M5-2 is similar to the learned model M3 or the learned model M4, the details thereof will be omitted.

Since the learning unit 106 is similar to the learning unit 34 of the second learning device 3 in Fig. 2, the details thereof will be omitted.

The setting unit 107 sets a target value of a physical property of the virtual recycled polymer composite material.

The target value may be appropriately set according to the use of the recycled polymer composite material or the like.

The third acquisition unit 108 regards the parameter including the descriptor of the recycled polymer material acquired by the second acquisition unit 104 as a parameter including a descriptor of the recycled polymer composite material similarly to the learning data set generation unit 105, and acquires the parameter as an explanatory variable. That is, the third acquisition unit 108 creates a parameter including a descriptor of the recycled polymer composite material according to the composition ratio on the basis of the parameter including the descriptor of the recycled polymer material acquired by the second acquisition unit 104, and acquires the parameter including the descriptor of the recycled polymer composite material as an explanatory variable.

The physical property prediction unit 109 sets the parameter including the descriptor of the recycled polymer composite material acquired by the third acquisition unit 108 to the second learned model M5-2 as a parameter including a descriptor of the virtual recycled polymer composite material, and inputs the parameter including the descriptor of the virtual recycled polymer composite material as an explanatory variable. The physical property prediction unit 109 outputs a physical property value of a virtual recycled polymer composite material as an objective variable by the second learned model M5-2. That is, the physical property prediction unit 109 predicts a physical property value of the virtual recycled polymer composite material corresponding to one kind of parameter including the descriptor of the virtual recycled polymer material from one second learned model M5-2.

The comparison unit 110 compares the physical property value of the virtual recycled polymer composite material predicted by the physical property prediction unit 109 with the target value of the physical property of the virtual recycled polymer composite material set by the setting unit 107.

The determination unit 111 determines whether the parameter including the descriptor of the virtual recycled polymer composite material needs to be optimized. For example, the determination unit 111 determines whether repetitive optimization is performed a predetermined number of times.

The predetermined number of times may be appropriately set according to the type, composition, and the like of the recycled polymer composite material to be used.

In addition, the determination unit 111 may also determine whether a difference between the physical property value of the virtual recycled polymer composite material and the target value of the physical property of the virtual recycled polymer composite material predicted by the physical property prediction unit 109 is within a predetermined range.

Note that the predetermined range may be appropriately set according to the type, composition, and the like of the recycled polymer composite material to be used. For example, in the predetermined range, the physical property value of the virtual recycled polymer composite material may be the same as the target value, or an error from the target value of the physical property of the virtual recycled polymer composite material may be in a range of several percent or less. In addition, when there are a plurality of physical property values of the virtual recycled polymer composite material, an error between at least one of the physical properties of the virtual recycled polymer composite material and the target value may be in a range of several percent or less.

The optimization unit 112 optimizes the physical property value of the virtual recycled polymer composite material predicted by the physical property prediction unit 109 by changing a parameter including a descriptor (for example, a mass ratio, a characteristic value, a structural value, and the like) of the virtual recycled polymer composite material so as to approach a target value. Each time a physical property value of the virtual recycled polymer composite material is predicted, the optimization unit 112 optimizes a mass ratio of the recycled polymer material and components other than the recycled polymer material contained in the virtual recycled polymer composite material as constituent components by changing a parameter including a descriptor of the virtual recycled polymer composite material.

That is, the optimization unit 112 optimizes the parameter including the descriptor of the virtual recycled polymer composite material so that the physical property value of the virtual recycled polymer composite material is changed in a desired direction.

As described above, the constituent components include the recycled polymer material and components other than the recycled polymer material as essential components, and may include one or more of each of the recycled polymer material and components other than the recycled polymer material. As a descriptor of the recycled polymer composite material, for example, when the type of the constituent component contained in the recycled polymer composite material and the mass ratio of the constituent component are optimized, it is preferable that the type of the recycled propylene-based polymer, the polyolefin elastomer, and the filler contained in the recycled polymer composite material as the constituent components and the mass ratio of the recycled propylene-based polymer, the polyolefin elastomer, the filler, and other constituent components are optimized.

The mass ratio of the recycled polymer composite material may include at least one or more of a talc amount, a rubber amount, and a glass fiber amount. Note that, when the mass ratio of the recycled polymer composite material is included, the virgin polymer composite material and the virtual recycled polymer composite material may include at least one or more of a talc amount, a rubber amount, and a glass fiber amount, similarly to the recycled polymer composite material.

The descriptor of the recycled polymer composite material may include at least one of a characteristic value and a structural value of the constituent component contained in the recycled polymer composite material. Note that, when the descriptor of the recycled polymer composite material is included, the descriptors of the virgin polymer composite material and the virtual recycled polymer composite material may each include at least one of a characteristic value and a structural value of a constituent component, similarly to the recycled polymer composite material.

The optimization unit 112 inputs the optimized descriptor of the virtual recycled polymer composite material to the third acquisition unit 108 again as a virtual descriptor.

The optimization unit 112 may use, for example, a library included in Anaconda (registered trademark), which is software distributed from Anaconda, Inc., USA. Anaconda (registered trademark) includes libraries used in Python (registered trademark) and machine learning. In order to optimize a parameter including a descriptor of the virtual recycled polymer composite material, the optimization unit 112 can propose a parameter including a descriptor of the virtual recycled polymer composite material having preferred physical properties using Optuna (registered trademark) which is a Python library.

That is, from the parameter including the descriptor of the virtual recycled polymer composite material and the predicted value of the physical property of the virtual recycled polymer composite material, the optimization unit 112 can propose a descriptor including a desired mass ratio, a characteristic value, a structural value, and the like of the virtual recycled polymer composite material when optimized using Optuna (registered trademark) which is a Python library.

The optimization unit 112 is not limited to changing the parameter including the descriptor of the virtual recycled polymer composite material to the parameter including the descriptor of the recycled polymer composite material recorded in the learning data set (second learning data set). The optimization unit 112 may change the parameter to a parameter that is not recorded in the learning data set (second learning data set) and includes a descriptor of the recycled polymer composite material.

That is, based on the physical property value of the virtual recycled polymer composite material predicted by the physical property prediction unit 109, the optimization unit 112 may change the descriptor of the virtual recycled polymer composite material to the mass ratio, the characteristic value, the structural value, and the like of the polymer composite material recorded in the learning data set (second learning data set), or may change the descriptor to the mass ratio, the characteristic value, the structural value, and the like of the polymer composite material not recorded in the learning data set (second learning data set).

In addition, the optimization unit 112 may change the parameter including the descriptor of the virtual recycled polymer composite material so as to avoid a specific parameter including a descriptor of the virtual recycled polymer composite material regardless of whether or not the parameter is recorded in the learning data set (second learning data set).

That is, the optimization unit 112 may change the descriptor of the specific recycled polymer composite material so as to avoid a specific mass ratio, characteristic value, structural value, and the like of the recycled polymer composite material, regardless of whether or not the descriptor is recorded in the learning data set (second learning data set).

It is preferable that the optimization unit 112 optimizes and proposes a mass ratio of the recycled polymer material and components other than the recycled polymer material contained as constituent components for the descriptor of the virtual recycled polymer composite material, using an algorithm to realize a parameter including a descriptor including a desired mass ratio, characteristic value, structural value, and the like of the virtual recycled polymer composite material at the time of optimization.

Examples of the algorithm include random search and mathematical optimization processing, and among them, mathematical optimization processing is preferable from the viewpoint of more efficiently performing optimization.

As the mathematical optimization processing, a genetic algorithm, Bayesian optimization, Tree-structured Parzen Estimator (TPE), and the like can be used. Only one of these algorithms may be used alone, or two or more thereof may be used in combination. Among them, a genetic algorithm is preferable from the viewpoint of a balance between a calculation speed per one processing and optimization efficiency of the predicted value of the physical property of the recycled polymer composite material.

The optimization unit 112 may optimize the parameter including the descriptor of the virtual recycled polymer composite material so that the physical property of the virtual recycled polymer composite material is maximized in a desired direction.

When the parameter including the descriptor of the virtual recycled polymer composite material is optimized from the predicted value of the physical property of the virtual recycled polymer composite material predicted by the physical property prediction unit 109, the optimization unit 112 may propose an optimized group of compositions of the virtual recycled polymer composite material (Pareto optimal solution).

That is, the optimization unit 112 may propose a plurality of combinations of compositions of the virtual recycled polymer composite material from the physical property of the virtual recycled polymer composite material predicted by the physical property prediction unit 109. For example, the optimization unit 112 may propose a plurality of combinations of mass ratios of the recycled polymer material and components other than the recycled polymer material contained in the virtual recycled polymer composite material as constituent components.

The optimization unit 112 may change the parameter including the descriptor of the virtual recycled polymer composite material so that each of the two or more physical property values of the virtual recycled polymer composite material predicted by inputting the parameter including the descriptor of the virtual recycled polymer composite material to the second learned model M5-2 generated for each of the two or more physical property values of the recycled polymer composite material approaches the target value of each of the two or more physical properties.

Even when the recycled polymer composite material has two or more physical properties, the optimization unit 112 may propose a group of compositions of the recycled polymer composite material for the two or more physical properties (Pareto optimum solution).

As a method for optimizing a parameter including a descriptor of a virtual recycled polymer composite material, there is the following method.

As a first optimization method, there is a method for optimizing a parameter including a descriptor of the virtual recycled polymer composite material so as to maximize or minimize a physical property value of the virtual recycled polymer composite material in a desired direction, and proposing a group of descriptors of the recycled polymer composite material.

The optimization method preferably optimizes a parameter including a descriptor of the virtual recycled polymer composite material so as to maximize a physical property of the virtual recycled polymer composite material in a desired direction.

In the optimization method, the optimization unit 112 can optimize the descriptor of the recycled polymer composite material to provide a preferred physical property value of the recycled polymer composite material by specifying the item of the physical property value of the recycled polymer composite material to be optimized and an optimization direction (maximization or minimization).

For example, the optimization unit 112 obtains a group P of predicted values of physical properties of the recycled polymer composite material corresponding to a composition R from the proposed composition R of the recycled polymer composite material via the third acquisition unit 108 and the physical property prediction unit 109. At this time, by repeating the above operation, the optimization unit 112 obtains a pair of a virtual composition $R_j$($j = 1, 2, \cdots n$) of the recycled polymer composite material and a group $P_j$($j = 1, 2, \cdots n$) of predicted values of physical property values of the recycled polymer composite material corresponding to the composition of the recycled polymer composite material. Then, the optimization unit 112 can select a group of compositions of the recycled polymer composite material in which the predicted value of the physical property value of the recycled polymer composite material is preferable from the pair of the obtained virtual composition $R_j$ of the recycled polymer composite material and the group $P_j$ of the predicted values of the physical property values of the recycled polymer composite material corresponding to the composition.

For example, a genetic algorithm is used as the mathematical optimization processing. In this case, a pair of a plurality of virtual compositions $R_j$($j = 1, 2, \cdots n$) initially (randomly) input to the second acquisition unit 104 and a group $P_j$($j = 1, 2, \cdots n$) of predicted values of corresponding physical property values is defined as a first generation. Then, a crossing-over operation of rearranging parameters including descriptors of the recycled polymer composite material between virtual compositions and a mutation operation of randomly replacing some parameters including the descriptors of the recycled polymer composite material are performed from a subset of the virtual compositions $R_j$ in which the value of the group $P_j$ of predicted values is desirable (that is, it is maximized or minimized in a desired direction). Accordingly, the optimization unit 112 obtains a group of compositions of the recycled polymer composite material in which the predicted value of the physical property value of the recycled polymer composite material is preferable. The above operation is repeated with the pair of the group of compositions thus obtained and the group of corresponding predicted values as a second generation. Accordingly, the optimization unit 112 further obtains a group of compositions of the recycled polymer composite material in which the predicted value of the physical property value of the recycled polymer composite material is preferable.

The optimization unit 112 can propose a group of descriptors of the recycled polymer composite material in which the physical property value of the recycled polymer composite material is maximum or minimum in a desired direction as a group of virtual descriptors by the above method.

Thus, the optimization unit 112 can propose a group of descriptors of the recycled polymer composite material in which the physical property value of the recycled polymer composite material is maximized or minimized in a desirable direction among the recycled polymer composite materials that can be considered in many combinations by the above method.

As a second optimization method, there is a method for proposing a group of descriptors of the recycled polymer composite material so that the physical property value of the virtual recycled polymer composite material approaches a desired value.

In the optimization method, the optimization unit 112 can optimize the composition of the recycled polymer composite material to provide a preferred physical property value of the recycled polymer composite material by specifying the item of the physical property value of the recycled polymer composite material to be optimized and the target value thereof as the objective function for optimization.

When a target value of a physical property value k of the recycled polymer composite material is set to OK and a predicted value of a physical property value of the recycled polymer composite material corresponding to the composition Rj of the recycled polymer composite material is set to Pk,j, the optimization unit 112 obtains an objective function fk for optimization by the following Equation (1).

$$fk = |Ok - Pk,j| / |Ok| \quad \cdots \quad (1)$$

Then, the optimization unit 112 optimizes the composition so as to minimize the objective function fk by the same method as the first optimization method described above.

The optimization unit 112 can propose a group of descriptors of the recycled polymer composite material that brings the physical property value of the recycled polymer composite material close to a desired value (target value) as a group of virtual descriptors by the above method.

Even when the physical property value of the recycled polymer composite material is two or more, the optimization unit 112 can propose a group of descriptors of the recycled polymer composite material by using a method for optimizing a parameter including a descriptor of the virtual recycled polymer composite material as described above.

That is, for each of the two or more physical property values of the recycled polymer composite material, the optimization unit 112 may optimize the parameter including the descriptor of the virtual recycled polymer composite material so as to maximize or minimize each of the two or more physical property values of the virtual recycled polymer composite material predicted by inputting the parameter including the descriptor of the virtual recycled polymer composite material to the second learned model M5-2, and may propose a group of descriptors of the recycled polymer composite material.

In addition, for each of the two or more physical properties of the recycled polymer composite material, the optimization unit 112 may optimize the parameter including the descriptor of the virtual recycled polymer composite material so that the two or more physical property values of the virtual recycled polymer composite material predicted by inputting the parameter including the descriptor of the virtual recycled polymer composite material to the second learned model M5-2 approach respective target values of the two or more physical property values, and may propose a group of descriptors of the recycled polymer composite material.

The display unit 113 displays information on a parameter including a descriptor of a virtual recycled polymer composite material. The descriptor of the recycled polymer composite material may be a descriptor of the recycled polymer composite material proposed by the optimization unit 112 once or more and determined again by the determination unit 111, or may be a descriptor of the recycled polymer composite material not optimized by the optimization unit 112. In the determination unit 111, when the physical property of the recycled polymer composite material predicted by the physical property prediction unit 109 is within a predetermined range of the target value, the descriptor of the recycled polymer composite material first acquired by the second acquisition unit 104 may be displayed.

The display unit 113 may display, for example, a group of descriptors of the recycled polymer composite material proposed by the optimization unit 112 as a list of descriptors of the recycled polymer composite material such as a map, a ranking format, and an optimum combination order.

As described above, the composition proposal system 10 includes the prediction unit 103, the setting unit 107, the physical property prediction unit 109, the comparison unit 110, the determination unit 111, and the optimization unit 112. In the composition proposal system 10, the comparison unit 110 compares the physical property value of the virtual recycled polymer composite material predicted by the physical property prediction unit 109 with the target value set by the setting unit 107, and the determination unit 111 determines whether the physical property of the virtual recycled polymer composite material is within a predetermined range of the target value. In the composition proposal system 10, the optimization unit 112 optimizes the parameter including the descriptor of the virtual recycled polymer composite material so that the physical property value of the virtual recycled polymer composite material predicted by inputting the parameter including the descriptor of the virtual recycled polymer composite material to the second learned model M5-2 approaches the target value.

In the composition proposal system 10, until the determination unit 111 determines that the physical property of the virtual recycled polymer composite material obtained by the physical property prediction unit 109 is within the predetermined range of the target value, the optimization unit 112 repeats the operation of optimizing the parameter including the descriptor of the virtual recycled polymer composite material so that the physical property value of the virtual recycled polymer composite material approaches the target value. Accordingly, the composition proposal system 10 can make the parameter including the descriptor of the virtual recycled polymer composite material substantially equivalent to the parameter including the descriptor of the recycled polymer composite material having a substantially equivalent physical property to the set target value. Therefore, the composition proposal system 10 can propose a parameter including a descriptor of the recycled polymer composite material having a physical property substantially equivalent to that of the recycled polymer composite material set as the target value from the physical property value of the virtual recycled polymer composite material.

Thus, the composition proposal system 10 can propose a parameter including a descriptor of the recycled polymer composite that provides the set desired physical property value of the recycled polymer composite material.

In addition, since the composition proposal system 10 includes the optimization unit 112, it is possible to reduce the burden and time required when optimizing the descriptor of the virtual recycled polymer composite material so as to satisfy the desired physical property of the virtual recycled polymer composite material. Thus, since the composition proposal system 10 can efficiently propose a parameter including a descriptor of the recycled polymer composite material satisfying a desired physical property, energy consumption required for producing the recycled polymer composite material and confirming the physical property can be reduced.

The composition proposal system 10 can include at least one or more of a talc amount, a rubber amount, and a glass fiber amount as a mass ratio of each of the recycled polymer composite material acquired by the first acquisition unit 101, the virgin polymer composite material used in the first learned model M5-1, the recycled polymer composite material used in the second learned model M5-2, and the virtual recycled polymer composite material (also referred to as "recycled polymer composite material or the like") acquired by the physical property prediction unit 109. A composition ratio of the recycled polymer material is often not constant, but a talc amount, a rubber amount, and a glass fiber amount are relatively hardly changed, and are easily used for predicting performance of the recycled polymer composite material containing the recycled polymer material. The composition proposal system 10 can improve the prediction performance of the physical property value of the recycled polymer composite material by including at least one or more of the talc amount, the rubber amount, and the glass fiber amount in the mass ratio of the recycled polymer composite material or the like, such that the parameter including the descriptor of the recycled polymer composite material having a desired physical property value can be proposed with higher accuracy.

The composition proposal system 10 can include at least one of a characteristic value and a structural value of a constituent component included in a descriptor of the recycled polymer composite material or the like. Since the characteristic value and the structural value of the component are easily used as a descriptor of the recycled polymer composite material or the like, the composition proposal system 10 can easily propose a parameter including at least one of the characteristic value and the structural value of the constituent component as a descriptor of the recycled polymer composite material having a desired physical property value.

The composition proposal system 10 can be modified in the optimization unit 29 to avoid a specific parameter including a descriptor for the virtual recycled polymer composite material. Accordingly, the composition proposal system 10 can propose a parameter including a descriptor of the recycled polymer composite material while avoiding overlapping of the descriptors of the virtual recycled polymer composite material.

The composition proposal system 10 can propose a mass ratio of the recycled polymer and components other than the recycled polymer contained as constituent components for the descriptor of the virtual recycled polymer composite material by performing mathematical optimization processing by the optimization unit 29. Accordingly, in the composition proposal system 10, the optimization unit 112 can more efficiently optimize the parameter including the descriptor of the recycled polymer composite material, such that the parameter including the descriptor of the recycled polymer composite material can be more efficiently proposed.

In the composition proposal system 10, the optimization unit 112 can use any one of a genetic algorithm, Bayesian optimization, and TPE as the mathematical optimization processing. In the composition proposal system 10, even when any one of a genetic algorithm, Bayesian optimization, and TPE is used for the mathematical optimization processing, the optimization unit 112 can optimize the parameter including the descriptor of the recycled polymer composite material and can propose the parameter including the descriptor of the recycled polymer composite material.

In the composition proposal system 10, the optimization unit 112 can optimize the parameter including descriptor of the virtual recycled polymer composite material so that the physical property of the virtual recycled polymer composite material is maximized in a desired direction. Accordingly, in the composition proposal system 10, the optimization unit 112 can appropriately optimize the parameter including the descriptor of the recycled polymer composite material, such that the parameter including the descriptor of the recycled polymer composite material can be proposed appropriately.

In the composition proposal system 10, the optimization unit 112 can propose a plurality of combinations of mass ratios of the recycled polymer material and components other than the recycled polymer material contained in the virtual recycled polymer composite material as constituent components. The composition proposal system 10 can collectively provide a user with a plurality of parameter candidates including descriptors of the recycled polymer composite materials at a time.

In the composition proposal system 10, the optimization unit 112 can change the parameter including the descriptor of the virtual recycled polymer composite material so that each of two or more physical properties of the virtual recycled polymer composite material predicted by inputting the parameter including the descriptor of the virtual recycled polymer composite material to the second learned model M5-2 approaches the target value of each of the two or more physical properties. Accordingly, the composition proposal system 10 can appropriately propose a parameter including a descriptor of the recycled polymer composite material having a desired physical property value.

In the composition proposal system 10, the optimization unit 112 can propose a Pareto solution for two or more physical property values. The composition proposal system 10 can propose a group of compositions of the recycled polymer composite material for two or more physical property values even when the recycled polymer composite material has two or more physical property values.

The composition proposal system 10 can include an average value for each type of constituent components such as the recycled polymer composite material as the mass ratio of the recycled polymer composite material or the like. The composition proposal system 10 can more appropriately propose the parameter including the descriptor of the recycled polymer composite material having the desired physical property value by using the average value for each type of these constituent components for the mass ratio of the recycled polymer composite material or the like.

The composition proposal system 10 can contain a copolymer containing two or more kinds of monomer units in the virgin polymer material and the recycled polymer material. Accordingly, even when the virgin polymer material and the recycled polymer material contain a copolymer, the composition proposal system 10 can propose a parameter including a descriptor of the recycled polymer composite material having a desired physical property value.

In the composition proposal system 10, a composition containing a propylene-based polymer, a polyolefin elastomer, and a filler can be used for the recycled polymer composite material and the like. Even when the recycled polymer composite material or the like is composed of a composition containing a propylene-based polymer, a polyolefin elastomer, and a filler, the composition proposal system 10 can propose a parameter including a descriptor of the recycled polymer composite material having a desired physical property value.

In the composition proposal system 10, the propylene-based polymer can include a copolymer containing a monomer unit derived from propylene and a monomer unit derived from ethylene. Even when the propylene-based polymer contained in the recycled polymer composite material or the like is composed of a copolymer containing ethylene, the composition proposal system 10 can propose a parameter including a descriptor of the recycled polymer composite material having a desired physical property value.

Note that the first learning device 1, the first prediction device 2, the second learning device 3, and the second prediction device 4 described above may be configured as a first learning system, a first prediction system, a second learning system, and a second prediction system. That is, the first learning device 1 and the second learning device 3 are independent devices such as a personal computer (PC) including each configuration in the device, but one or more of the configurations may be arranged outside the device and connected via a network.

For example, the learning data set may be provided on a cloud. In this case, the first learning device 1 and the second learning device 3 are configured as a learning system by a learning data set connected via a network.

Similarly, one or more of the configurations of the first prediction device 2 and the second prediction device 4 may be arranged outside the devices and connected via a network.

<Hardware configuration>

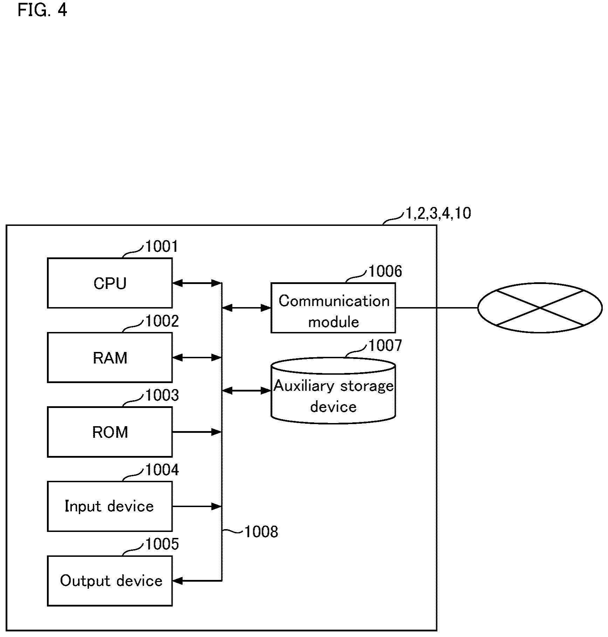

Next, an example of a hardware configuration of the first learning device 1, the first prediction device 2, the second learning device 3, the second prediction device 4, and the composition proposal system 10 will be described. Fig. 4 is a block diagram illustrating the hardware configuration of the first learning device 1, the first prediction device 2, the second learning device 3, the second prediction device 4, and the composition proposal system 10. As illustrated in Fig. 4, the first learning device 1, the first prediction device 2, the second learning device 3, the second prediction device 4, and the composition proposal system 10 are configured by an information processing device (computer), and can be physically configured as a computer system including a central processing unit (CPU) 1001 that is an arithmetic processing unit, a random access memory (RAM) 1002 and a read only memory (ROM) 1003 that are main storage devices, an input device 1004 that is an input device, an output device 1005, a communication module 1006, an auxiliary storage device 1007 such as a hard disk, and the like. These devices are mutually connected by a bus 1008. Note that the output device 1005 and the auxiliary storage device 1007 may be provided outside.

The CPU1001 controls the overall operations of the first learning device 1, the first prediction device 2, the second learning device 3, the second prediction device 4, and the composition proposal system 10, and performs various types of information processing. The CPU1001 can execute, for example, a learning method, a prediction method, and a composition proposal method, or a learning program, a prediction program, and a composition proposal program, which will be described below, stored in the ROM103 or the auxiliary storage device 1007, to perform learning, prediction, and composition proposal.

The RAM1002 is used as a work area of the CPU1001, and may include a non-volatile RAM that stores main control parameters and information.

The ROM103 stores a basic input/output program and the like. The learning program, the prediction program, or the composition proposal program may be stored in the ROM103.

The input device 1004 is an input device such as a keyboard, a mouse, an operation button, a touch panel, or a display screen, receives information input by a user as an instruction signal, and outputs the instruction signal to the CPU1001.

The output device 1005 is a display device such as a monitor display, a speaker, a printing device such as a printer, or the like. In the output device 1005, for example, information such as a learning result, a prediction result, and a composition proposal result is displayed on a display device such as a monitor display, and a screen to be displayed is updated according to an input operation via the input device 1004 or the communication module 1006.

The communication module 1006 is a data transmission/reception device such as a network card, and functions as a communication interface that captures information from an external data recording server or the like and outputs analysis information to another electronic device.

The auxiliary storage device 1007 is a storage device such as a solid state drive (SSD) or a hard disk drive (HDD), and stores, for example, various data, files, and the like required for the operations of the first learning device 1, the first prediction device 2, the second learning device 3, the second prediction device 4, and the composition proposal system 10.

Each function of the first learning device 1, the first prediction device 2, the second learning device 3, the second prediction device 4, and the composition proposal system 10 is realized by reading predetermined computer software (including a learning program, a prediction program, and a composition proposing program) from the main storage device such as the RAM1002 or the auxiliary storage device 1007, executing the computer software by the CPU1001, reading and writing data in the main storage device such as the RAM1002 or the auxiliary storage device 1007, and operating the input device 1004, the output device 1005, and the communication module 1006.

Therefore, in each unit of the first learning device 1, the first prediction device 2, the second learning device 3, the second prediction device 4, and the composition proposal system 10 illustrated in Figs. 1 to 3, in a computer including the first learning device 1, the first prediction device 2, the second learning device 3, the second prediction device 4, and the composition proposal system 10, a processor executes predetermined computer software (including a learning program, a prediction program, and a composition proposing program) stored in advance, such that software and hardware are realized in cooperation.

The learning program, the prediction program, and the composition proposal program can be stored in, for example, the main storage device or the auxiliary storage device 1007 included in the computer. In addition, the learning program, the prediction program, and the composition proposal program may be stored on a computer connected to a communication line such as the Internet, and may be provided by downloading some or all of the learning program, the prediction program, and the composition proposal program via the communication line. Furthermore, the learning program, the prediction program, and the composition proposal program may be provided or distributed via a communication line.

Some or all of the learning program, the prediction program, and the composition proposal program may be recorded (including installation) in the computer from a state of being stored in a portable storage medium such as an optical disk such as a CD-ROM or a DVD-ROM, a semiconductor memory such as a flash memory, or the like.

<First learning method>

Next, a first learning method will be described. The first learning method is a method for generating a learned model for predicting a parameter including a descriptor of a polymer composite material containing a polymer material using a learning data set in which a physical property (explanatory variable) of the polymer composite material and a parameter (objective variable) including a descriptor of the polymer composite material are associated with each other in the first learning device 1 having a configuration as illustrated in Fig. 1.

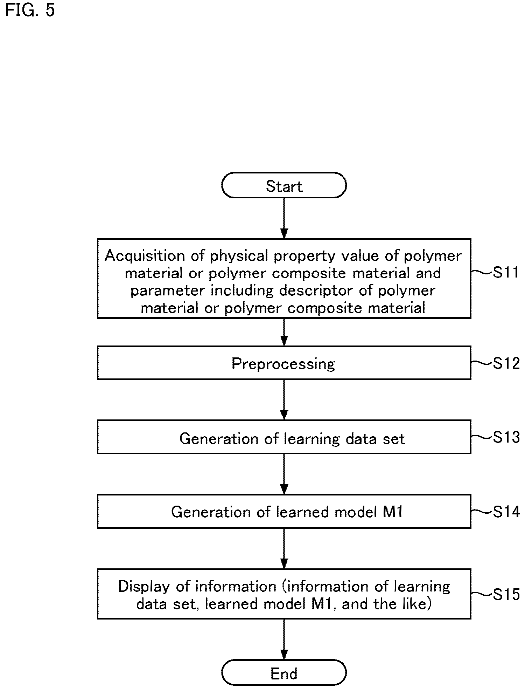

Fig. 5 is a flowchart illustrating the first learning method. As illustrated in Fig. 5, the acquisition unit 11 acquires a physical property value of the polymer material or the polymer composite material as an explanatory variable, and acquires a parameter including a descriptor of the polymer material or the polymer composite material as an objective variable (first learning information acquisition step: step S11).

Next, the preprocessing unit 12 performs preprocessing of the acquired physical property value of the polymer material or the polymer composite material and the acquired parameter including the descriptor of the polymer material or the polymer composite material (first learning information preprocessing step: step S12).

Next, the learning data set generation unit 13 extracts the preprocessed physical property value of the polymer material or the polymer composite material as an explanatory variable and the preprocessed parameter including the descriptor of the polymer material or polymer composite material as an objective variable, and adds the extracted variables to the learning data set (first learning data set generation step: step S13).

The learning data set generation unit 13 creates a learning data set by associating the input physical property value of the polymer material or the polymer composite material with the parameter including the descriptor of the polymer material or the polymer composite material.

Next, the learning unit 14 generates the learned model M1 by using the learning data set in which the physical property value of the polymer material or the polymer composite material and the parameter including the descriptor of the polymer material or the polymer composite material are associated with each other (first learning step: step S14).

In response to the input of the physical property value of the polymer material or the polymer composite material included in the learning data set, the learning unit 14 generates the learned model M1 so that an output matches a parameter including a descriptor of the polymer material or the polymer composite material associated with the physical property value of the polymer material or the polymer composite material.

Next, the display unit 15 displays the information of the learning data used in the learning of the learned model M1 and the information on the learned model M1 (first learning information display step: step S15).

The first learning method includes a first learning step (step S14) to generate a learned model M1 for predicting a parameter including a descriptor of the polymer material or the polymer composite material. Accordingly, the learning method uses the learned model M1 generated in the first learning step (step S14) to perform learning so as to predict a parameter including a descriptor of the polymer material or the polymer composite material from the input physical property value of the polymer material or the polymer composite material.

In addition, the first learning method includes the first learning step (step S14), and generates the learned model M1 in the first learning step (step S14). Therefore, the first learning method is used to predict a parameter including a descriptor of the polymer material or the polymer composite material from a physical property value of the polymer material or the polymer composite material, thereby reducing the burden and time required for prediction of the parameter including the descriptor of the polymer material or the polymer composite material. Therefore, when the first learning method is used, it is possible to reduce energy consumption when predicting a parameter including a descriptor of the polymer material or the polymer composite material.

<First prediction method>

Next, a first prediction method will be described. A first prediction method is a method of predicting a parameter including a descriptor of the recycled polymer material to be predicted from a physical property value of the recycled polymer material by the learned model M2 in the first prediction device 2 having the configuration as illustrated in Fig. 1.

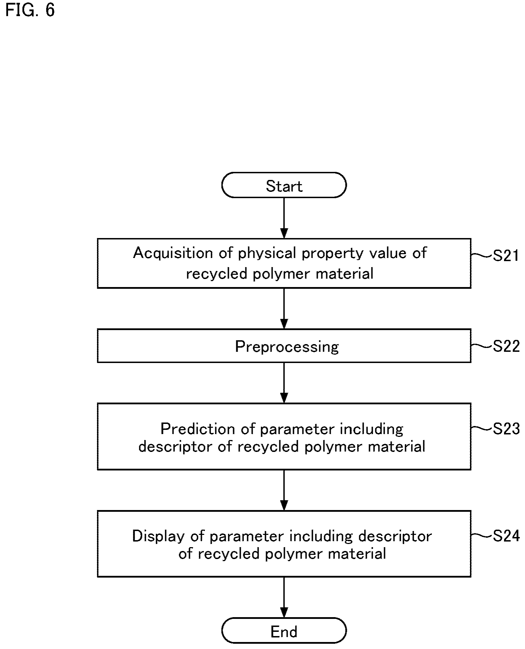

Fig. 6 is a flowchart illustrating the first prediction method. As illustrated in Fig. 6, the acquisition unit 21 acquires a physical property value of the recycled polymer material to be predicted as an explanatory variable (first prediction information acquisition step: step S21).

Next, the preprocessing unit 12 performs preprocessing of the acquired physical property value of the recycled polymer material to be predicted (first prediction information preprocessing step: step S22).

Next, the prediction unit 23 inputs the preprocessed physical property value of the recycled polymer material to be predicted to the learned model M2, thereby outputting the parameter including the descriptor of the recycled polymer material to be predicted, which is predicted by the learned model M2 (first prediction step: step S23).

Next, in the first prediction step S23, the display unit 24 displays information on the parameter including the descriptor of the recycled polymer material predicted by the learned model M2 (first prediction information display step: step S24).

The first prediction method includes the first prediction step (step S24), such that the learned model M2 predicts, as an objective variable, a parameter including a descriptor of the recycled polymer material or the polymer composite material from the physical property of the recycled polymer material or the polymer composite material. Therefore, the first prediction method predicts a parameter including a descriptor of the recycled polymer material or the polymer composite material corresponding to the input physical property value of the recycled polymer material or the polymer composite material.

In addition, the first prediction method includes the first prediction step (step S24), such that a parameter including a descriptor of the recycled polymer material is predicted while reducing the burden and time required for prediction of the parameter including the descriptor of the recycled polymer material from the physical property value of the recycled polymer material. Therefore, since the first prediction method efficiently predicts the parameter including the descriptor of the recycled polymer material, it is possible to reduce energy consumption when predicting the parameter including the descriptor of the recycled polymer material.

<Second learning method>

Next, a second learning method will be described. The learning method is a method for generating a learned model M3 for predicting a physical property value of the recycled polymer composite material from a parameter including a descriptor of the recycled polymer composite material using a learning data set in which a parameter (explanatory variable) including a descriptor of the recycled polymer composite material containing the recycled polymer material and a physical property (objective variable) of the recycled polymer composite material are associated with each other in the second learning device 3 having the configuration as illustrated in Fig. 2.

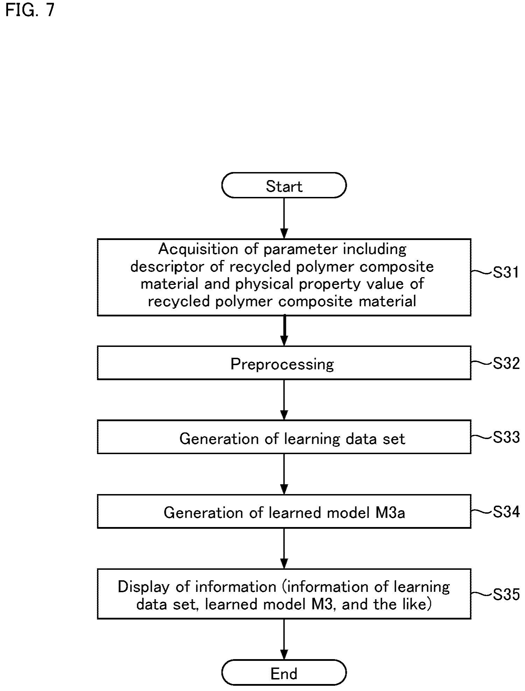

Fig. 7 is a flowchart illustrating the second learning method. As illustrated in Fig. 7, the acquisition unit 31 acquires a parameter including a descriptor of the recycled polymer composite material as an explanatory variable, and acquires a physical property value of the recycled polymer composite material as an objective variable (second learning information acquisition step: step S31).

Next, the preprocessing unit 32 performs preprocessing of the parameter (explanatory variable) including the descriptor of the recycled polymer composite material and the physical property value (objective variable) of the recycled polymer composite material that are acquired (second learning information preprocessing step: step S32).

Next, the learning data set generation unit 33 extracts each of the preprocessed parameter including the descriptor of the recycled polymer composite material as an explanatory variable and the physical property value of the recycled polymer composite material as an objective variable, and adds the extracted variables to the learning data set (second learning data set generation step: step S33).

The learning data set generation unit 33 creates a learning data set by associating the input parameter including the descriptor of the recycled polymer composite material with the physical property value of the recycled polymer composite material.

Next, the learning unit 34 generates a learned model M3 by using a learning data set in which a parameter including a descriptor of the recycled polymer composite material and a physical property of the recycled polymer composite material are associated with each other (second learning step: step S34).

In response to the input of the parameter including the descriptor of the recycled polymer composite material included in the learning data set, the learning unit 34 generates the learned model M3 so that the output matches the physical property value of the recycled polymer composite material associated with the parameter including the descriptor of the recycled polymer composite material.

Next, the display unit 35 displays the information of the learning data used in the learning of the learned model M3 and the information on the learned model M3 (second learning information display step: step S35).

The second learning method includes the second learning step (step S34) to generate the learned model M3 for predicting the physical property value of the recycled polymer composite material. Therefore, the learning method performs learning to predict the physical property value of the recycled polymer composite material from the input parameter including the descriptor of the recycled polymer composite material by using the learned model M3 generated in the second learning step (step S33).

In addition, the second learning method includes the second learning step (step S34), and generates the learned model M3 in the second learning step (step S34). Therefore, when using the second learning method, the second learning method is used to predict the physical property value of the recycled polymer composite material from the parameter including the descriptor of the recycled polymer composite material, thereby reducing the burden and time required for predicting the physical property value of the recycled polymer composite material. Therefore, when the second learning method is used, energy consumption when predicting the physical property value of the recycled polymer composite material can be reduced.

<Second prediction method>

Next, a second prediction method will be described. The second prediction method is a method for predicting the physical property value of the recycled polymer composite material to be predicted from the parameter including the descriptor of the recycled polymer composite material by the learned model M4 in the second prediction device 4 having the configuration as illustrated in Fig. 2.

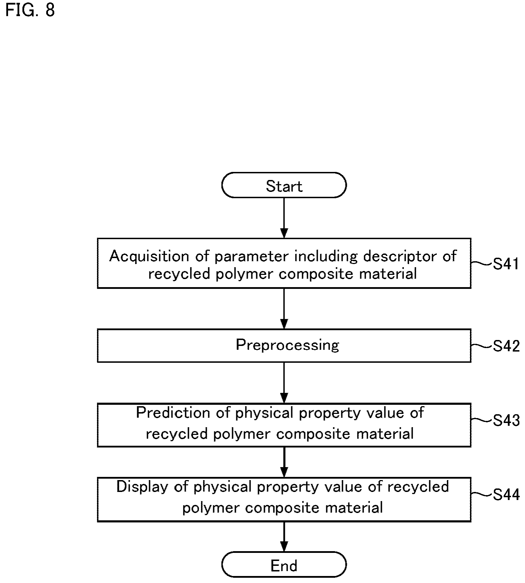

Fig. 8 is a flowchart illustrating the second prediction method. As illustrated in Fig. 8, the acquisition unit 41 acquires a parameter including a descriptor of the recycled polymer composite material to be predicted as an explanatory variable (second prediction information acquisition step: step S41).

Next, the preprocessing unit 42 performs preprocessing of the acquired parameter including the descriptor of the recycled polymer composite material to be predicted (second prediction information preprocessing step: step S42).

Next, the prediction unit 43 inputs the preprocessed parameter including the descriptor of the recycled polymer composite material to be predicted to the learned model M4, thereby outputting the physical property value of the recycled polymer composite material to be predicted by the learned model M4 (second prediction step: step S43).

Next, in the second prediction step S43, the display unit 44 displays information on the physical property value and the like of the recycled polymer composite material predicted by the learned model M4 (second prediction information display step: step S44).

The second prediction method includes the second prediction step (step S43), such that the physical property value of the recycled polymer composite material is predicted from the parameter including the descriptor of the recycled polymer composite material by the learned model M4. Therefore, the second prediction method predicts a physical property value of the recycled polymer composite material from the input parameter including the descriptor of the recycled polymer composite material.

In addition, the second prediction method includes the second prediction step (step S43), thereby predicting the physical property value of the recycled polymer composite material while reducing the burden and time required for predicting the physical property value of the recycled polymer composite material from the parameter including the descriptor of the recycled polymer composite material. Therefore, since the second prediction method efficiently predicts the physical property value of the recycled polymer composite material, it is possible to reduce energy consumption when predicting the physical property value of the recycled polymer composite material.

<Composition proposal method>

Next, a composition proposal method according to the present embodiment will be described. The composition proposal method according to the present embodiment is a method for proposing a composition of the recycled polymer composite material by the learned model M5 in the composition proposal system 10 having the configuration as illustrated in Fig. 3.

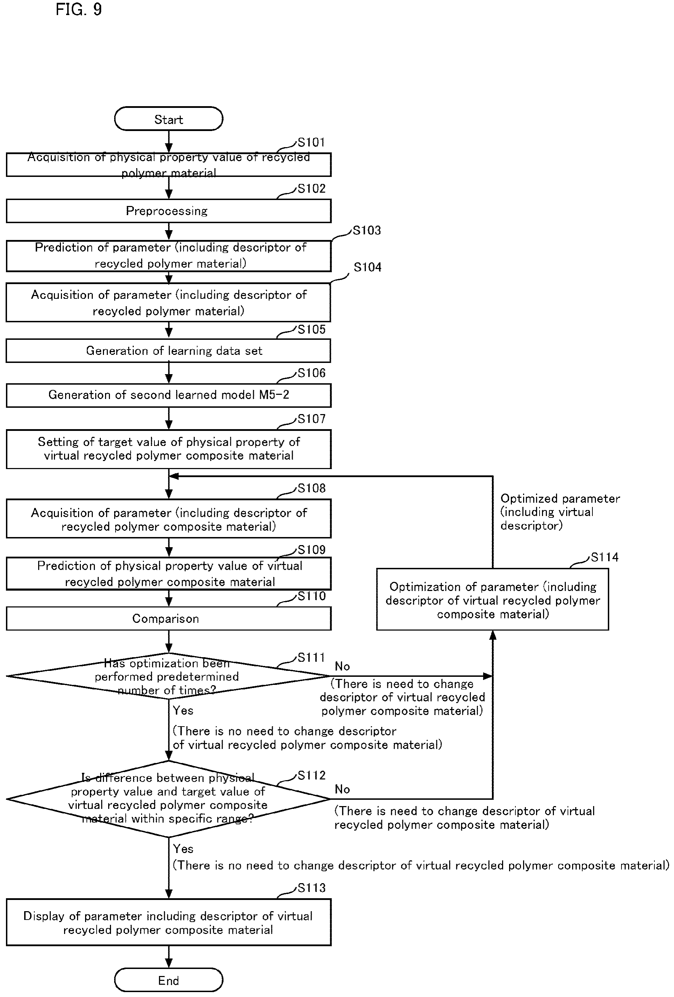

Fig. 9 is a flowchart illustrating the composition proposal method according to the present embodiment. As illustrated in Fig. 9, the first acquisition unit 101 acquires a physical property value of the recycled polymer material as an explanatory variable (acquisition step: step S101).

The physical property value of the recycled polymer material acquired first may be a randomly acquired physical property value, and for example, randomly acquired moldability (fluidity), density, yield stress, tensile strength, tensile modulus, flexural strength, deflection temperature under load, Rockwell hardness, rigidity, impact resistance, glossiness, and the like of the recycled polymer material may be used. In addition, the recycled polymer material initially input may be a plurality of groups corresponding to the plurality of recycled polymer materials.

As the recycled polymer material, a recycled polymer material is randomly selected from a list of recycled polymer materials obtained from the outside, usable recycled polymer materials, and the like. At this time, the recycled polymer material may be one kind or a plurality of kinds.

Next, the preprocessing unit 102 performs preprocessing of the physical property value of the recycled polymer material acquired in the acquisition step (step S101) (preprocessing step: step S102). Note that the preprocessing step (step S102) may be performed as necessary, and when the physical property value of the recycled polymer material preprocessed in advance is acquired in the acquisition step (step S101), the preprocessing step (step S102) may not be performed.

Next, the prediction unit 103 inputs the physical property value of the recycled polymer material preprocessed in the preprocessing step (step S102) to the first learned model M5-1, thereby predicting a parameter including a descriptor of the recycled polymer material as an objective variable (prediction step: step S103).

In the prediction step (step S103), a group of predicted values of parameters including descriptors of the recycled polymer material corresponding to the physical property values of the recycled polymer material may be predicted using a plurality of first learned models M5-1.

In the prediction step (step S103), the mass ratio of the recycled polymer material may include at least one or more of a talc amount and a rubber amount.

In the prediction step (step S103), the descriptor of the recycled polymer material may include at least one of a characteristic value and a structural value of the constituent component included in the recycled polymer material.

Next, the second acquisition unit 104 acquires the parameter including the descriptor of the recycled polymer material predicted by the first learned model M5-1 by the prediction unit 103 (second acquisition step: step S104).

Next, the learning data set generation unit 105 regards the parameter including the descriptor of the recycled polymer material acquired by the second acquisition unit 104 as a parameter including a descriptor of the recycled polymer composite material. The learning data set generation unit 105 adds a parameter including a descriptor of the recycled polymer composite material to the learning data set as an explanatory variable (learning data set step process: step S105).

That is, the learning data set generation unit 105 creates a parameter including a descriptor of the recycled polymer composite material according to the composition ratio on the basis of the parameter including the descriptor of the recycled polymer material acquired by the second acquisition unit 104, and adds the parameter including the descriptor of the recycled polymer composite material to the learning data set as an explanatory variable.

The learning data set may already contain a parameter including a descriptor of the recycled polymer composite material in the past as an explanatory variable and a physical property value of the recycled polymer composite material in the past as an objective variable using a past recycled polymer composite material. The learning data set generation step (step S105) may be performed similarly to the learning data set generation step (step S33) of the second learning method.

Next, the learning unit 106 generates the second learned model M5-2 by performing learning using a learning data set in which a parameter (explanatory variable) including a descriptor of the recycled polymer composite material and a physical property value (objective variable) of the recycled polymer composite material are associated with each other (learning step: step S106).

The second learned model M5-2 is a learned model learned using a second learning data set in which a parameter including a descriptor of the recycled polymer composite material and a physical property value of the recycled polymer composite material are associated with each other. The second learned model M5-2 may use the learned model M3 generated by the second learning method or the learned model M4 used in the second prediction method. Since the second learned model M5-1 is similar to the learned model M3 or the learned model M4, the details thereof will be omitted.

Since the learning step (step S106) is similar to the second learning step (step S34) of the second learning method, the details thereof will be omitted.

Next, the setting unit 107 sets a target value of a physical property of the virtual recycled polymer composite material (physical property target value setting step: step S107).

As described above, the target value may be appropriately set according to the use of the recycled polymer composite material or the like.

Next, similarly to the learning data set generation step (step S105), the third acquisition unit 108 regards a parameter including a descriptor of the recycled polymer material acquired in the second acquisition step (step S104) as a parameter including a descriptor of the recycled polymer composite material, and acquires the parameter as an explanatory variable (third acquisition step: step S108).

That is, the third acquisition unit 108 creates a parameter including a descriptor of the recycled polymer composite material according to the composition ratio on the basis of the parameter including the descriptor of the recycled polymer material acquired in the second acquisition step (step S104), and acquires the parameter including the descriptor of the recycled polymer composite material as an explanatory variable.

Next, the physical property prediction unit 109 sets the parameter including the descriptor of the recycled polymer composite material acquired in the third acquisition step (step S108) to the second learned model M5-2 as a parameter including a descriptor of the virtual recycled polymer composite material, and inputs the parameter including the descriptor of the virtual recycled polymer composite material as an explanatory variable. The physical property prediction unit 109 predicts a physical property value of the virtual recycled polymer composite material as an objective variable using the second learned model M5-2 (physical property prediction step: step S109).

In the physical property prediction step (step S109), a physical property value of the virtual recycled polymer composite material corresponding to one kind of parameter including the descriptor of the virtual recycled polymer composite material is predicted from one second learned model M5-2.

In the physical property prediction step (step S109), a group of predicted values of physical properties of the recycled polymer composite material corresponding to parameters including descriptors of the virtual recycled polymer composite material may be predicted using a plurality of second learned models M5-2.

Next, the physical property of the virtual recycled polymer composite material predicted in the physical property prediction step (step S109) is compared with the target value of the physical property of the virtual recycled polymer composite material set in the physical property target value setting step (step S107) by the comparison unit 110 (comparison step: step S110).

Next, the determination unit 111 determines whether or not the optimization of the parameter including the descriptor of the virtual recycled polymer composite material predicted in the physical property prediction step (step S109) is performed a predetermined number of times (first determination step: step S111).

The predetermined number of times may be appropriately set according to the type, composition, and the like of the recycled polymer composite material to be used.

When the parameter including the descriptor of the virtual recycled polymer composite material is optimized a predetermined number of times (step S111: Yes), the determination unit 111 determines that there is no need to change the parameter including the descriptor of the virtual recycled polymer composite material.

Next, the determination unit 111 determines whether or not a difference between the physical property value of the virtual recycled polymer composite material and the target value is within a predetermined range (second determination step: step S112). Note that, in the composition proposal method according to the present embodiment illustrated in Fig. 9, the first determination step (step S111) and the second determination step (step S112) are performed by the determination unit 111, but the second determination step (step S1112) may be omitted.

When the difference between the physical property value of the virtual recycled polymer composite material and the target value is within the predetermined range (step S112: Yes), the determination unit 111 determines that there is no need to change the parameter including the descriptor of the virtual recycled polymer composite material.

The parameter including the descriptor of the virtual recycled polymer composite material acquired in the third acquisition step (step S108) is not changed and maintained.

Next, the display unit 113 displays information on the parameter including the descriptor of the virtual recycled polymer composite material (display step: step S113).

On the other hand, when the optimization of the parameter including the descriptor of the virtual recycled polymer composite material is not performed the predetermined number of times (step S111: No), or when the difference between the physical properties of the virtual recycled polymer composite material and the target value does not fall within the predetermined range (step S112: No), the determination unit 111 determines that there is a need to optimize by changing the parameter including the descriptor of the virtual recycled polymer composite material.

Next, the optimization unit 112 optimizes the parameter including the descriptor (for example, the mass ratio, the characteristic value, the structural value, and the like) of the virtual recycled polymer composite material by changing the parameter to an optimal parameter so that the physical property value of the virtual recycled polymer composite material predicted in the prediction step (step S109) approaches the target value set in the physical property target value setting step (step S107) (optimization step: step S114).

In the optimization step (step S114), each time a physical property value of the virtual recycled polymer composite material is predicted in the third acquisition step (step S108), the optimization unit 112 optimizes a mass ratio of the recycled polymer material and components other than the recycled polymer material contained in the virtual recycled polymer composite material as constituent components by changing a parameter including a descriptor of the virtual recycled polymer composite material.

As described above, the constituent components include the recycled polymer material and components other than the recycled polymer material as essential components, and may include one or more of each of the recycled polymer material and components other than the recycled polymer material. As a descriptor of the recycled polymer composite material, for example, when the type of the constituent component contained in the recycled polymer composite material and the mass ratio of the constituent component are optimized, it is preferable that the type of the recycled propylene-based polymer and the polyolefin elastomer contained in the recycled polymer composite material as the constituent components and the mass ratio of the recycled propylene-based polymer, the polyolefin elastomer, and other constituent components are optimized.

In the optimization step (step S114), the mass ratio of the recycled polymer composite material may include at least one or more of a talc amount, a rubber amount, and a glass fiber amount. Note that, when the mass ratio of the recycled polymer composite material is included, the virgin polymer composite material and the virtual recycled polymer composite material may include at least one or more of a talc amount, a rubber amount, and a glass fiber amount, similarly to the recycled polymer composite material.

In the optimization step (step S114), the descriptor of the recycled polymer composite material may include at least one of a characteristic value and a structural value of the constituent component contained in the recycled polymer composite material. Note that, when the descriptor of the recycled polymer composite material is included, the descriptors of the virgin polymer composite material and the virtual recycled polymer composite material may each include at least one of a characteristic value and a structural value of a constituent component, similarly to the recycled polymer composite material.

In the optimization step (step S114), the optimization unit 112 may use, for example, a library included in Anaconda (registered trademark), which is software distributed from Anaconda, Inc., USA. Anaconda (registered trademark) includes libraries used in Python (registered trademark) and machine learning. In order to optimize a parameter including a descriptor of the virtual recycled polymer composite material, the optimization unit 112 can propose a parameter including a descriptor of the virtual recycled recycled polymer composite material having preferred physical properties using Optuna (registered trademark) which is a Python library.

In the optimization step (step S114), the optimization unit 112 may not be limited to changing the parameter including the descriptor of the virtual recycled polymer composite material to the parameter including the descriptor of the recycled polymer composite material recorded in the learning data set. The optimization unit 112 may change the parameter to a parameter that is not recorded in the learning data set and includes a descriptor of the recycled polymer composite material.

In the optimization step (step S114), the optimization unit 112 may change the parameter including the descriptor of the virtual recycled polymer composite material so as to avoid a specific parameter including a descriptor of the virtual recycled polymer composite material regardless of whether or not the parameter is recorded in the learning data set.

In the optimization step (step S114), in order to realize a parameter including a desirable descriptor (within a predetermined range from a target value) of the virtual recycled polymer composite material when optimized, it is preferable to optimize a mass ratio of the recycled polymer material and components other than the recycled polymer material as constituent components for the descriptor of the virtual recycled polymer composite material, using an algorithm such as random search and a mathematical optimization method.

In the optimization step (step S114), when the parameter including the descriptor of the virtual recycled polymer composite material is optimized from the predicted value of the physical property of the virtual recycled polymer composite material predicted in the prediction step (step S108), the optimization unit 112 may propose an optimized group of compositions of the virtual recycled polymer composite material (Pareto optimal solution).

In the optimization step (step S114), the optimization unit 112 may change the parameter including the descriptor of the virtual recycled polymer composite material so that each of the two or more physical property values of the virtual recycled polymer composite material predicted by the second learned model M5-2 generated for each of the two or more physical properties of the recycled polymer composite material approaches the target value of each of the two or more physical properties.

Even when the recycled polymer composite material has two or more physical properties, the optimization unit 112 may propose a group of compositions of the recycled polymer composite material for the two or more physical properties (Pareto optimum solution).

In the optimization step (step S114), as a method for optimizing the parameter including the descriptor of the virtual recycled polymer composite material by the optimization unit 112, a method for optimizing the parameter including the descriptor of the virtual recycled polymer composite material to maximize or minimize the physical property of the virtual recycled polymer composite material in a desired direction and proposing a group of descriptors of the recycled polymer composite material may be used.

In addition, as another method for optimizing the parameter including the descriptor of the virtual recycled polymer composite material, a method for proposing a group of descriptors of the recycled polymer composite material so as to bring the physical property of the virtual recycled polymer composite material close to a desired value may be used.

In the optimization step (step S114), the optimization unit 112 optimizes the parameter including the descriptor of the recycled polymer composite material, and then the process again proceeds to the third acquisition step (step S108). Then, the third acquisition unit 108 sets the optimized descriptor of the virtual recycled polymer composite material as a virtual descriptor, inputs a parameter including the virtual descriptor as an explanatory variable to the third acquisition unit 108, and performs the same process as described above.

Then, in the first determination step (step S111), the optimization of the parameter including the descriptor of the virtual recycled polymer composite material is performed a predetermined number of times, and in the second determination step (step S112), the optimization of the descriptor of the virtual recycled polymer composite material is repeatedly performed in the optimization step (step S114) until it is determined that the difference between the parameter including the descriptor of the virtual recycled polymer composite material and the target value falls within a predetermined range.

Finally, in the first determination step (step S111), the parameter including the descriptor of the virtual recycled polymer composite material is optimized a predetermined number of times, and in the second determination step (step S112), it is determined that the difference between the parameter including the descriptor of the virtual recycled polymer composite material and the target value falls within the predetermined range, and then, as described above, in the optimization step (step S114), the display unit 113 displays information on the proposed parameter including the descriptor of the virtual recycled polymer composite material (display step: step S113).

The composition proposal method according to the present embodiment includes a prediction step (step S103), a characteristic target value setting step (step S107), a comparison step (step S110), and an optimization step (step S114). In the composition proposal method, a parameter including a descriptor of the recycled polymer material is predicted by inputting a physical property value of the recycled polymer material to the first learned model M5-1 in the prediction step (step S103). In the composition proposal method, in the third acquisition step (step S108), the parameter including the descriptor of the virtual recycled polymer composite material is acquired based on the parameter including the descriptor of the obtained recycled polymer material.

In the composition proposal method, in the optimization step (step S114), the parameter including the descriptor of the virtual recycled polymer composite material is optimized so that the physical property value of the virtual recycled polymer composite material predicted by inputting the parameter including the descriptor of the virtual recycled polymer composite material to the second learned model M5-2 approaches the target value. Accordingly, the composition proposal method can make the parameter including the descriptor of the virtual recycled polymer composite material substantially equivalent to the parameter including the descriptor of the recycled polymer composite material having a substantially equivalent physical property to the set target value. Therefore, the composition proposal method can propose a parameter including a descriptor of the recycled polymer composite material having a physical property substantially equivalent to that of the recycled polymer composite material set as the target value from the physical property value of the virtual recycled polymer composite material.

Therefore, the composition proposal method according to the present embodiment can propose a parameter including a descriptor of the recycled polymer composite material, by which the recycled polymer composite material having a desired physical property can be obtained.

In addition, the composition proposal method according to the present embodiment includes the optimization step (step S114), such that the composition proposal method according to the present embodiment can reduce the burden and time required when optimizing the parameter including the descriptor of the virtual recycled polymer composite material. Therefore, since the composition proposal method according to the present embodiment can efficiently propose a parameter including a descriptor of the recycled polymer composite material substantially equivalent to a parameter including a descriptor of the recycled polymer composite material satisfying a desired physical property, it is possible to reduce energy consumption required for production of the recycled polymer composite material, confirmation of physical properties, and the like.

The composition proposal method according to the present embodiment includes a first determination step (step S111), and in the first determination step (step S111), it is determined whether or not the optimization of the parameter including the descriptor of the virtual recycled polymer composite material acquired in the third acquisition step (step S108) is performed a predetermined number of times. In the composition proposal method according to the present embodiment, until it is determined in the first determination step (step S111) that the parameter including the descriptor of the virtual recycled polymer composite material is optimized a predetermined number of times, in the optimization step (step S114), an operation of optimizing the parameter including the descriptor of the virtual recycled polymer composite material is repeated so that the physical property value of the virtual recycled polymer composite material approaches the target value. Accordingly, the composition proposal method according to the present embodiment can propose a parameter including a descriptor of the recycled polymer composite material from the physical property value of the virtual recycled polymer composite material. Therefore, the composition proposal method according to the present embodiment can propose a parameter including a descriptor of the recycled polymer composite material, by which a desired physical property of the recycled polymer composite material to be set can be obtained.

The composition proposal method according to the present embodiment includes the second determination step (step S112). Accordingly, in the composition proposal method according to the present embodiment, until it is determined in the second determination step (step S112) that the physical property of the virtual recycled polymer composite material obtained in the physical property prediction step (step S106) is within the predetermined range of the target value, in the optimization step (step S114), the operation of optimizing the parameter including the descriptor of the virtual recycled polymer composite material can be repeated so that the physical property value of the virtual recycled polymer composite material approaches the target value.

The composition proposal method according to the present embodiment includes a comparison step (step S110). In the composition proposal method according to the present embodiment, in the comparison step (step S110), the physical property value of the virtual recycled polymer composite material predicted in the physical property prediction step (step S106) is compared with the target value set in the physical property target value setting step (step S107). In the composition proposal method according to the present embodiment, it can be determined whether or not the physical property value of the virtual recycled polymer composite material is within a predetermined range of the target value in the second determination step (step S112). Accordingly, in the composition proposal method according to the present embodiment, in the optimization step (step S114), the parameter including the descriptor of the virtual recycled polymer composite material can be optimized so that the physical property value of the virtual recycled polymer composite material predicted by inputting the parameter including the descriptor of the virtual recycled polymer composite material to the second learned model M5-2 approaches the target value.

In the composition proposal method, in the comparison step (step S110), the descriptor of the virtual recycled polymer composite material acquired in the third acquisition step (step S108) is compared with the target value set in the physical property target value setting step (step S107), and in the second determination step (step S112), it can be determined whether the descriptor of the virtual recycled polymer composite material is within a predetermined range of the target value. Accordingly, in the composition proposal method according to the present embodiment, in the optimization step (step S114), the parameter including the descriptor of the virtual recycled polymer composite material can be optimized so that the physical property value of the virtual recycled polymer composite material approaches the target value.

Since the composition proposal method according to the present embodiment has the characteristics as described above, the composition proposal method can be used for producing a recycled polymer composite material containing a recycled polymer as a constituent component and having desired physical properties.

<Method for producing recycled polymer composite material>

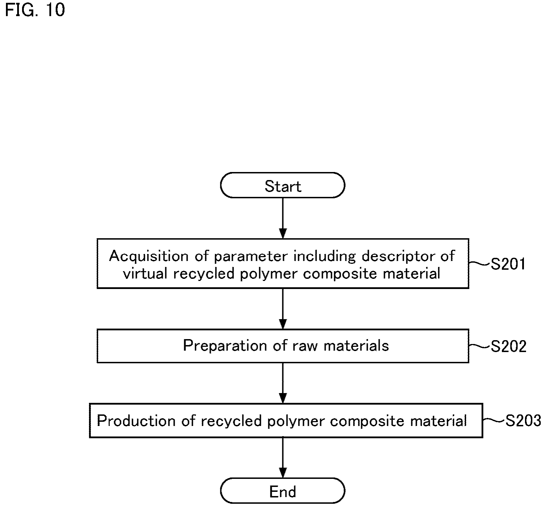

A method for producing a recycled polymer composite material according to the present embodiment will be described. The method for producing a recycled polymer composite material according to the present embodiment is a method for producing a recycled polymer composite material using the composition proposal method according to the present embodiment described above. Fig. 10 is a flowchart for illustrating the method for producing a recycled polymer composite material according to an embodiment. As illustrated in Fig. 10, a parameter including a descriptor of the virtual recycled polymer composite material obtained by the composition proposal system 10 illustrated in Fig. 3 is obtained (acquisition step: step S201).

Next, a recycled polymer material and components other than the recycled polymer material that satisfy the parameters are prepared as raw materials (raw material preparation step: step S202).

Next, the respective raw materials prepared in the raw material preparation step (step S202) are mixed to obtain a recycled polymer composite material satisfying the parameter (recycled polymer composite material production step).

The recycled polymer composite material contains a recycled polymer material, a fibrous filler, and other components contained as necessary other than the recycled polymer material as constituent components contained in the recycled polymer composite material, and has desired physical property values.

<Recycled polymer composite material>

The recycled polymer composite material according to the present embodiment can be produced by the method for producing a recycled polymer composite material according to the present embodiment described above. The recycled polymer composite material according to the present embodiment contains, for example, a recycled polymer material and components other than the recycled polymer material as constituent components, and satisfies any one or more of the following first parameter to third parameter when a talc amount in the recycled polymer composite material is Wt, a glass fiber amount in the recycled polymer composite material is Wg, and a rubber amount in the recycled polymer composite material is Wr.

The first parameter is the following parameters (I) to (IV):

(I): a density of the recycled polymer composite material is 1.08 $g/cm^3$ to 1.16 $g/cm^3$,
(II) an elastic modulus of the recycled polymer composite material is $128 \times Wt + 213 \times Wg - 102 \times Wr + 1,081 > 5,000$ MPa,
(III) a flexural strength of the recycled polymer composite material is $-0.375 \times Wt + 1.52 \times Wg - 3.30 \times Wr + 106 > 80$ MPa, and
(IV) an impact of the recycled polymer composite material is $-0.102 \times Wt + 0.149 \times Wg + 0.180 \times Wr + 4.49 > 6$.

The second parameter is the following parameters (I) to (IV):

(I): a density of the recycled polymer composite material is 1.20 $g/cm^3$ to 1.25 $g/cm^3$,
(II) an elastic modulus of the recycled polymer composite material is $128 \times Wt + 213 \times Wg - 102 \times Wr + 1,081 > 7,500$ MPa,
(III) a flexural strength of the recycled polymer composite material is $-0.375 \times Wt + 1.52 \times Wg - 3.30 \times Wr + 106 > 115$ MPa, and
(IV) an impact of the recycled polymer composite material is $-0.102 \times Wt + 0.149 \times Wg + 0.180 \times Wr + 4.49 > 9.0$.

The third parameter is the following parameters (I) to (IV):

(I): a density of the recycled polymer composite material is 1.30 $g/cm^3$ to 1.35 $g/cm^3$,
(II) an elastic modulus of the recycled polymer composite material is $128 \times Wt + 213 \times Wg - 102 \times Wr + 1,081 > 10,000$ MPa,
(III) a flexural strength of the recycled polymer composite material is $-0.375 \times Wt + 1.52 \times Wg - 3.30 \times Wr + 106 > 140$ MPa, and
(IV) an impact of the recycled polymer composite material is $-0.102 \times Wt + 0.149 \times Wg + 0.180 \times Wr + 4.49 > 11.0$.

As described above, the embodiments have been described, but the above embodiments are presented as an example, and the present invention is not limited by the above embodiments. The embodiments can be implemented in various other forms, and various combinations, omissions, substitutions, changes, and the like can be made without departing from the gist of the invention. These embodiments and modifications thereof are included in the scope and gist of the invention, and are included in the invention described in the claims and the equivalent scope thereof.

EXAMPLES

Hereinafter, embodiments will be described more specifically with reference to Examples, but the embodiments are not limited to these Examples and Comparative Examples.

<Example 1>

(1) Generation of learning data set 1

A learning data set used in a learning device (a first learning device 1 illustrated in Fig. 1 and a second learning device 3 illustrated in Fig. 2) to be described below was generated by the following procedure. Note that, in the present example, a heterophasic propylene polymerization material was used for a propylene-based polymer (A) as a polymer material contained in a glass fiber reinforced composite material as a polymer composite material, and a glass fiber was used as a fibrous filler.

(Acquisition of physical properties of polypropylene-based resin composition)

According to the method described in WO 2020/149284, a polypropylene-based resin composition was biaxially extruded to obtain a polypropylene-based resin composition. As physical property values of the polypropylene-based resin composition, MFR, density of an injection molded product, tensile strength, tensile modulus, flexural strength, flexural modulus, HDT, room-temperature notched Charpy impact strength, and Rockwell hardness of pellets formed by molding the polypropylene-based resin composition were measured.

(Generation of learning data set 1)

A learning data set, in which the physical property values of the prepared polypropylene-based resin composition were associated with a talc amount and a rubber amount as descriptors of the polypropylene-based resin composition, was created.

(Generation of learned model M1)

A learned model M1 was generated using the first learning device 1 illustrated in Fig. 1.

(2) First learning device

(2-1) Learning unit

A learning unit 14 performed learning using the learning data set to generate the learned model M1 (learning step).
As the learned model M1, a learned model M1-1 for predicting the talc amount of the polypropylene-based resin material and a learned model M1-2 for predicting the rubber amount were generated.

(Generation of learned model M1-1 for predicting talc amount)

Standardization processing was performed on 200 learning data sets by the preprocessing unit 12, the data sets were divided into a learning data set and a verification data set by a $5 \times 5$ double cross validation method, and hyperparameter optimization and verification of prediction accuracy of the learned model M1-1 for talc amount prediction were performed.
Then, using the regression equation construction data, a learned model M1 was generated by Support Vector Regression (SVR). A learning program was included in the generated learned model M1-1 for talc amount prediction, and Python was used as a program language for the learning program.

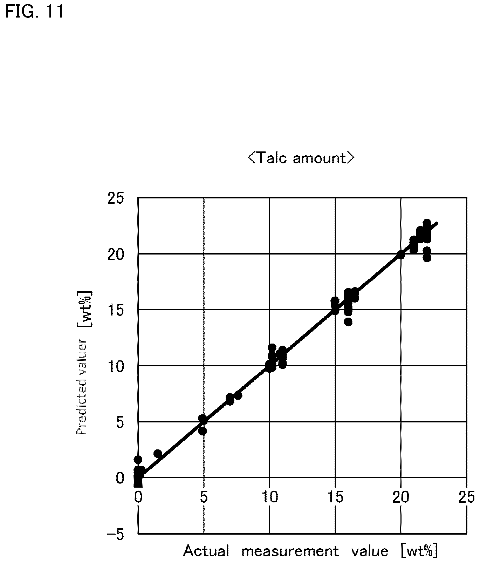

Fig. 11 illustrates a relationship between the actual measurement value of the talc amount and the predicted value of the talc amount predicted by the learned model constructed with the regression equation construction data using the verification data. Note that the actual measurement value is an actually measured talc amount. The coefficient of determination between the actual measurement value and the predicted value of the talc amount was $R^2 = 0.99$. The coefficient of determination $R^2$ was calculated by a $5 \times 5$ double cross validation method. The root mean square error RMSE between the actual talc amount and the predicted talc amount was 0.46, and the optimal hyperparameters were 710.9680713994642 for C, 0.0057724966787030315 for $\varepsilon$, and 0.003367343151112364 for $\gamma$.

(Generation of learned model M1-2 for predicting rubber amount)

Standardization processing was performed on 200 learning data sets by the preprocessing unit 22, the data sets were divided into a learning data set and a verification data set by a $5 \times 5$ double cross validation method, and hyperparameter optimization and verification of prediction accuracy of the learned model were performed.
Then, using the regression equation construction data, a learned model M1-2 for rubber amount prediction was generated by Support Vector Regression (SVR). A learning program was included in the generated learned model M1-2 for rubber amount prediction, and Python was used as a program language for the learning program.

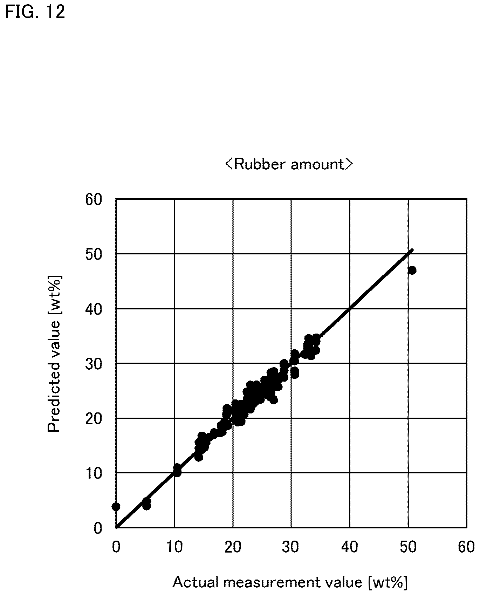

Fig. 12 illustrates a relationship between the actual measurement value of the rubber amount and the predicted value of the rubber amount predicted by the learned model constructed with the regression equation construction data using the verification data. Note that the actual measurement value is an actually measured rubber amount. The coefficient of determination between the actual measurement value and the predicted value of the rubber amount was $R^2 = 0.96$. The

coefficient of determination R2 was calculated by a 5 × 5 double cross validation method. The root mean square error (RMSE) between the actual rubber amount and the predicted rubber amount was 1.13, and the optimal hyperparameters were 338.2984019418194 for C, 0.0004977635704389426 for $\varepsilon$, and 0.008957273495603121 for $\gamma$.

From Figs. 11 and 12, it was confirmed that the learned model M1 with high accuracy can be obtained for each of the talc amount and the rubber amount of the polypropylene-based resin composition.

(3) First prediction device 2

The first prediction device 2 illustrated in Fig. 2 was created using the learned model M1 generated by the first learning device 1 illustrated in Fig. 1. The physical property value of the polymer material that was recycled (recycled polymer material) was predicted by the created first prediction device 2.

(1) Generation of learning data set 2

A learning data set 2 used in a second learning device 3 to be described below was generated by the following procedure.

(Acquisition of characteristics of recycled glass fiber reinforced resin material containing recycled polymer material)

The talc amount, the rubber amount, and the glass fiber amount, which are characteristics of the recycled glass fiber reinforced resin material containing the recycled polymer material used in the learning data set 2, were calculated based on the talc amount, the rubber amount, and the glass fiber amount to be blended predicted using the first prediction device 2 described above.

(Calculation of descriptor of recycled glass fiber reinforced resin material containing recycled polymer material)

As a descriptor of the recycled glass fiber reinforced resin material containing the recycled polymer material, an average structural value and a composition ratio of each kind of raw materials of the recycled glass fiber reinforced resin material containing the recycled polymer material were calculated according to the following.

When the respective characteristic values of the recycled polymer material A-i(i = 1, 2, ···n) were the P part content Pcont,i, the filler content Fcont,i, and the rubber content Rcont,i, and the composition ratio was XAi, the following five average structural values were calculated as descriptors. Note that Pcont represents the P part content.

- 

$$av.Pcont = \Sigma\_i(100-Fcont,i-Rcont,i) \times XAi/100$$

- 

$$av.Fcont = \Sigma\_iFcont,i \times XAi/100$$

.

$$av.Rcont = \Sigma\_iRcont,i \times XAi/100$$

(Descriptor related to other composition ratio)

For the glass fiber, the total value of the composition ratios was calculated.

(Calculation of descriptor of recycled glass fiber reinforced resin material containing recycled polymer material)

The calculation result of the descriptor of the recycled glass fiber reinforced resin material containing the recycled polymer material used is shown below. A descriptor of a composition (mixture), in which a recycled glass fiber reinforced resin material was composed of a recycled polymer material A-1, a recycled polymer material A-2, a recycled polymer material A-3, an acid-modified polymer material A-4, and a glass fiber B, and a mass ratio of each of the raw materials was A-1:A-2:A-3:A-4:B = 30:20:20:1.5:28.5, was calculated. Here, the total amount of the recycled polymer materials A-1, A-2, and A-3 and the glass fiber B was 100 parts by mass. The characteristics of each raw material were as follows.

A-1: Talc amount = 1.8, rubber amount = 18.8
A-2: Talc amount = 5.8, rubber amount = 26.2
A-3: Talc amount = 17.0, rubber amount = 19.6
A-4: Talc amount = 0, rubber amount = 0

First, the following four kinds of descriptors were calculated.

(A). Recycled polymer material

(A-1). P part content in recycled glass fiber

reinforced resin material av.Pcont = (100 - 1.8 - 18.8) $\times$ 30/100 + (100 - 5.8 - 26.2) $\times$ 20/100 + (100 - 17.0 - 19.6) $\times$ 20/100 + (100 - 0 - 0) $\times$ 1.5/100 = 51.6

(A-2). Talc amount in recycled glass fiber

reinforced resin material av.Fcont = 1.8 $\times$ 30/100 + 5.8 $\times$ 20/100 + 17.0 $\times$ 20/100 = 5.1

(A-3). Rubber amount in recycled glass fiber

reinforced resin material av.Rcont =18.8 $\times$ 30/100 + 26.2 $\times$ 20/100 + 19.6 $\times$ 20/100 = 14.8

(B). Glass fiber

Glass fiber content in recycled glass fiber reinforced resin material Gcont = 28.5

(Acquisition of physical properties of recycled glass fiber reinforced resin material containing recycled polymer material)

According to the method described in WO 2022/080363, a recycled glass fiber reinforced resin material containing a recycled polymer material was biaxially extruded to obtain a recycled glass fiber reinforced resin material containing a recycled polymer material. As physical property values of the recycled glass fiber reinforced resin material containing the recycled polymer material, the flexural modulus, flexural strength, and room-temperature Charpy impact strength of the injection molded product were measured.

(Generation of learning data set 2)

A learning data set 2, in which a parameter including a descriptor of the recycled glass fiber reinforced resin material containing the prepared recycled polymer material was associated with flexural modulus, flexural strength, and room-temperature Charpy impact strength, which are physical property values of the recycled glass fiber reinforced resin material containing the recycled polymer material, was created.

(Generation of learned model M2)

Then, a learned model M3 is generated using the second learning device 3 illustrated in Fig. 2.

(2) Second learning device

(2-1) Preprocessing unit

The created learning data set 2 was subjected to a standardization process.

(2-2) Learning unit

A learning unit 34 performed learning using the learning data set 2 to generate the learned model M3 (learning step).

As the learned model M3, a learned model M3-1 for predicting flexural modulus of a recycled glass fiber reinforced resin material containing a recycled polymer material, a learned model M3-2 for predicting flexural strength, and a learned model M3-3 for predicting room-temperature Charpy impact strength were generated.

(Generation of learned model M3-1 for predicting flexural modulus)

27 learning data sets of glass fiber reinforced resin materials containing a recycled polymer material were subjected to standardization processing by the preprocessing unit 32, the data sets were divided into a learning data set and a verification data set by a $5 \times 5$ double cross validation method, and hyperparameter optimization and verification of prediction accuracy of the learned model M3-1 were performed.

Then, using the regression equation construction data, a learned model M3 was generated by Support Vector Regression (SVR). A learning program was included in the generated learned model M3-1 for flexural modulus prediction, and Python was used as a program language for the learning program.

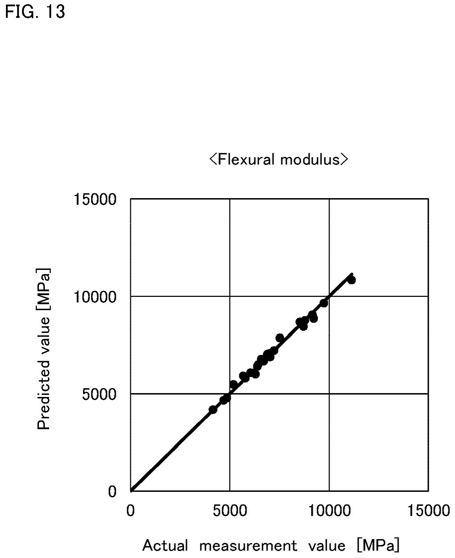

Fig. 13 illustrates a relationship between the actual measurement value of the flexural modulus and the predicted value of the flexural modulus predicted by the learned model M3-1 for predicting the flexural modulus, which is generated by the regression equation generation data using the verification data. Note that the actual measurement value is an actually measured flexural modulus. The coefficient of determination between the actual flexural modulus and the predicted flexural modulus was $R2 = 0.99$. The coefficient of determination $R2$ was calculated by a $5 \times 5$ double cross validation method. The root mean square error (RMSE) between the actual flexural modulus and the predicted flexural modulus was 171 MPa, and the optimal hyperparameters were 860.7773617595948 for C, 0.006556323859521451 for $\varepsilon$, and 0.00019036464103819754 for $\gamma$.

(Generation of learned model M3-2 for predicting flexural strength)

Standardization processing was performed on 27 learning data sets by the preprocessing unit 32, the data sets were divided into a learning data set and a verification data set by a $5 \times 5$ double cross validation method, and hyperparameter optimization and verification of prediction accuracy of the learned model M3-2 for flexural strength prediction were performed.

Then, using the regression equation construction data, a learned model M3-2 for flexural strength prediction was generated by Support Vector Regression (SVR). A learning program was included in the generated learned model M3-2 for flexural strength prediction, and Python was used as a program language for the learning program.

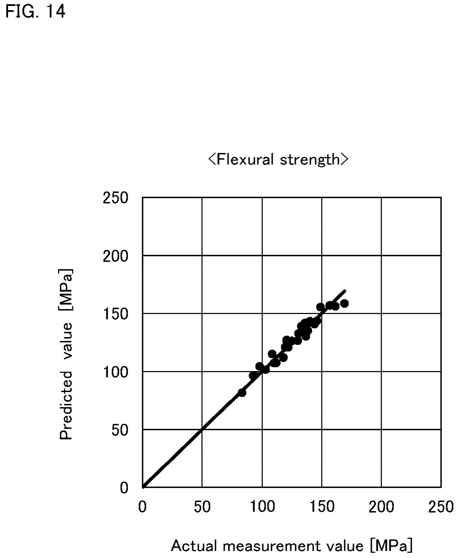

Fig. 14 illustrates a relationship between the actual measurement value of the flexural strength and the predicted value of the flexural strength predicted by the learned model M3-2 for predicting the flexural strength, which is generated using the regression equation generation data using the verification data. Note that the actual measurement value is an actually measured flexural strength. The coefficient of determination between the actual flexural strength and the predicted flexural strength was $R2 = 0.95$. The coefficient of determination $R2$ was calculated by a $5 \times 5$ double cross validation method. The root mean square error (RMSE) between the actual flexural strength and the predicted flexural strength was 4.7 MPa, and the optimal hyperparameters were 494.29663614862807 for C, 0.014722045129405956 for $\varepsilon$, and 0.002062280440242451 for $\gamma$.

(Generation of learned model M3-3 for predicting room-temperature Charpy impact strength)

Standardization processing was performed on 27 learning data sets by the preprocessing unit 32, the data sets were divided into a learning data set and a verification data set by a $5 \times 5$ double cross validation method, and hyperparameter optimization and verification of prediction accuracy of the learned model M3-3 for room-temperature Charpy impact strength prediction were performed.

Then, using the regression equation construction data, a learned model M3-3 for room-temperature Charpy impact strength prediction was generated by Support Vector Regression (SVR). A learning program was included in the generated learned model M3-3 for room-temperature Charpy impact strength prediction, and Python was used as a program language for the learning program.

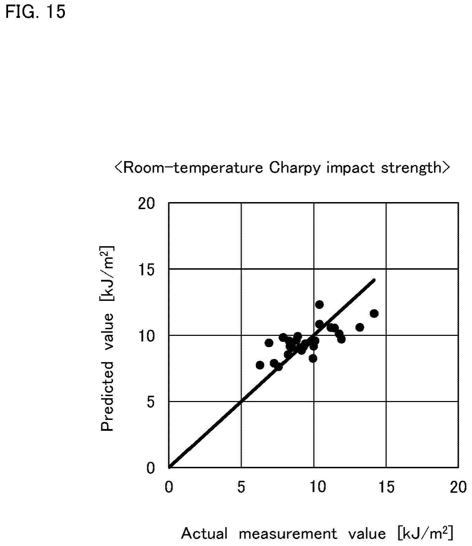

Fig. 15 illustrates a relationship between the actual measurement value of the room-temperature Charpy impact strength and the predicted value of the room-temperature Charpy impact strength predicted by the learned model M3-3 for predicting the room-temperature Charpy impact strength, which is generated by the regression equation construction data using the verification data. Note that the actual measurement value is an actually measured room-temperature Charpy impact strength. The coefficient of determination between the actual room-temperature Charpy impact strength and the predicted room-temperature Charpy impact strength was $R2 = 0.48$. The coefficient of determination $R2$ was calculated by a $5 \times 5$ double cross validation method. The root mean square error (RMSE) between the actual room-temperature Charpy impact strength and the predicted room-temperature Charpy impact strength was 1.32 $kJ/m^2$, and the optimal hyperparameters were 3.1511970815454693 for C, 0.04367160457726784 for $\varepsilon$, and 0.6227761135297052 for $\gamma$.

From Figs. 13 to 15, it was confirmed that a learned model with high accuracy was obtained for the flexural modulus, the flexural strength, and the room-temperature Charpy impact strength.

(3) Second prediction device 4

The second prediction device 4 illustrated in Fig. 2 was prepared using the learned model M4 generated by the second learning device 3 illustrated in Fig. 2. The physical properties of the recycled glass fiber reinforced resin material containing the recycled polymer material were predicted by the prepared second prediction device 4.

In order to compare with the prediction result, a molded body was prepared and evaluated using a recycled glass fiber reinforced resin material containing a corresponding recycled polymer material. An example of experimentally synthesizing and evaluating the recycled glass fiber reinforced resin material containing the recycled polymer material was used as a synthesis example, and an example of predicting the physical property value of the recycled glass fiber reinforced resin material containing the recycled polymer material using the learned model M4 described above was used as a use example.

(3-1) Synthesis Example 1 and Use Example 1

(Synthesis Example 1) Synthesis of recycled glass fiber reinforced resin material containing recycled polymer material)

(Use Example 1) Prediction of physical property value of recycled glass fiber reinforced resin material containing recycled polymer material)

The flexural modulus, the flexural strength, and the room-temperature Charpy impact strength were predicted as the physical property values of the recycled polymer composite material using the prediction device (the first prediction device 2 illustrated in Fig. 1 and the second prediction device 4 illustrated in Fig. 2) described above. The predicted value of the flexural modulus was 9,050 MPa, the predicted value of the flexural strength was 156, and the prediction value of the room-temperature Charpy impact strength was 10.1 $kj/m^2$.

Note that the parameter including the descriptor of the recycled glass fiber reinforced resin material containing the recycled polymer material to be predicted was input to the learned models M2 and M4, and the parameter including the descriptor of the recycled glass fiber reinforced resin material containing the recycled polymer material was calculated in the same manner as the parameter including the descriptor of the recycled glass fiber reinforced resin material containing the recycled polymer material when the learned models M2 and M4 were generated.

<Example 2>

(1) Generation of learning data set

The same procedure as in Example 1 was performed.

(2) Learning device

The same procedure as in Example 1 was performed.

(3) Prediction device

The same procedure as in Example 1 was performed.

(4) Composition proposal system

A composition of a propylene-based resin composition having excellent properties was proposed using Optuna, which is a Python library for optimization. Hereinafter, an example in which a group of compositions of the propylene-based resin composition that maximizes or minimizes physical property values of the recycled glass fiber reinforced resin material, which is a recycled polymer composite material, in a desired direction is proposed (Example 2-1) is shown.

(Example 2-1) Method for proposing group of compositions of propylene-based resin composition that maximizes or minimizes physical property values of recycled glass fiber reinforced resin material to desired value

A pair of the hypothetical composition Rj(j = 1, 2, ···n) and the group of predicted values of the characteristics of the composite material corresponding to the composition Pj(j = 1, 2, ···n) were obtained as follows.

For the recycled glass fiber reinforced resin material containing the recycled polymer material and the glass fiber, the recycled polymer material A and the glass fiber B were randomly selected from the list of usable raw materials, and the composition ratio was randomly set to obtain an initial composition R1. Here, the total amount of the recycled polymer material A and the glass fiber B in the initial composition R1 was set to 100 parts by mass. At this time, one kind or a plurality of kinds of raw materials were used.

The parameter including the descriptor of the initial composition R1 was calculated by the same method as the method described in the first learning device 1 illustrated in Fig. 1 and the second learning device 3 illustrated in Fig. 2. Then, a group P1 of predicted values of physical properties corresponding to the initial composition R1 was obtained using the first prediction device 2 and the second prediction device 4 described above. The group P1 of predicted values of physical properties is a group of predicted values of physical properties corresponding to the set learned models M2 and M4.

From the virtual composition R1 and the group P1 of predicted values of physical properties, a composition R2 having desirable physical properties was proposed using an optimization library Optuna. A genetic algorithm was used as an algorithm for the proposal.

As an objective function for optimization, by specifying a physical property item to be optimized and an optimization direction (maximization or minimization), the composition of the recycled glass fiber reinforced resin material was optimized so that a plurality of physical property values were improved.

From the proposed composition R2, a group P2 of predicted values of physical properties of the corresponding recycled glass fiber reinforced resin material was obtained.

Hereinafter, a pair of a virtual composition Rj (j = 1, 2, ···n) of the recycled glass fiber reinforced resin material and a group Pj (j=1, 2, ···n) of predicted values of physical properties of the recycled glass fiber reinforced resin material corresponding to the composition was obtained by repetition.

From a pair of the obtained virtual composition Rj (j = 1, 2, ···n) of the recycled glass fiber reinforced resin material and a group Pj (j=1, 2, ···n) of predicted values of physical properties of the recycled glass fiber reinforced resin material corresponding to the composition, a group of compositions in which the predicted value of the physical property of the recycled glass fiber reinforced resin material was excellent was selected.

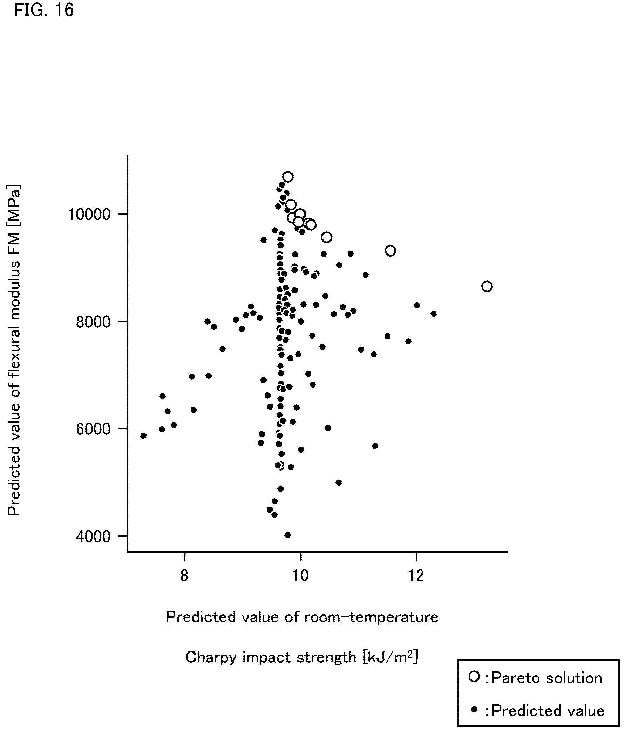

Fig. 16 illustrates a result of proposing a group of compositions in which a plurality of physical properties including the flexural modulus and the room-temperature Charpy impact strength of the recycled glass fiber reinforced resin material are optimized in the direction of maximization using a genetic algorithm. As illustrated in Fig. 16, a group of compositions (Pareto optimal solution, white circle in Fig. 16) optimized so that both the flexural modulus and the room-temperature Charpy impact strength were in the maximization direction was obtained.

Therefore, it was confirmed that the composition proposal system can efficiently propose a group of compositions (Pareto optimum solution) in which physical properties are maximized in a desirable direction among many possible combinations of recycled glass fiber reinforced resin materials.

(Example 2-2) Method for proposing group of compositions that bring characteristics of recycled glass fiber reinforced resin material close to desired values

When a target value of a physical property k of the recycled glass fiber reinforced resin material is set to OK and a predicted value of the physical properties of the recycled glass fiber reinforced resin material corresponding to the composition Rj is set to Pk,j, an objective function fk for optimization is determined as in the following Equation (4).

$$fk = |Ok - Pk,j| / |Ok| \quad \cdots \quad (4)$$

(In the equation, |Ok-Pk,j| and |Ok| represent absolute values of Ok-Pk,j and Ok, respectively.)

It was confirmed that a group of compositions of the propylene-based resin composition contained in the recycled glass fiber reinforced resin material, which bring the physical properties of the recycled glass fiber reinforced resin material close to desired values, can be proposed by optimizing the composition so as to minimize the objective function fk for optimization by the same method as the method described in (Example 2-1).

(Example 3-1) Calculation of composition ratio so that each of plurality of physical properties of glass fiber reinforced resin material is equal to or more than desired value

A learned model was generated by Ridge regression using 27 learning data sets of recycled glass fiber reinforced resin materials containing the recycled polymer materials described above. Furthermore, multiple regression analysis was performed using three variables of the talc amount, the glass fiber amount, and the rubber component amount as explanatory variables and three variables of the flexural modulus, the flexural strength, and the Charpy impact strength as objective variables to obtain the following multiple regression equations (5) to (7).

$$\text{Flexural modulus} = 128\ Wf + 213\ Wg - 102\ Wr + 1{,}081 \cdots (5)$$

$$\text{Flexural strength} = -0.38\ Wf + 1.52\ Wg - 3.30\ Wr + 106.30 \cdots (6)$$

$$\text{Charpy room temperature impact} = -0.10\ Wf + 0.15\ Wg + 0.18\ Wr + 4.49 \cdots (7)$$

Wf: mass percent of filler in recycled glass fiber reinforced resin material
Wg: mass percentage of glass fiber in recycled glass fiber reinforced resin material
Wr: mass percentage of rubber component in recycled glass fiber reinforced resin material

From the obtained multiple regression equations (5) to (7), the composition ratio of the propylene-based resin composition required for simultaneously satisfying a plurality of desired physical properties can be calculated as follows.

(Use Example 3-1)

When the density of the recycled glass fiber reinforced resin material is 1.10 g/cm$^3$ to 1.15 g/cm$^3$, it is predicted that the recycled glass fiber reinforced resin material satisfying all of the following Formulas (8) to (10) satisfies all of the flexural modulus > 5,000 MPa, the flexural strength > 90 MPa, and the room-temperature Charpy impact strength > 7.0 kj/m$^2$.

$$5{,}000 < 128\ Wt + 213\ Wg - 102\ Wr + 1{,}081 \cdots (8)$$

$$90 < -0.38\ Wt + 1.52\ Wg - 3.30\ Wr + 106.30 \cdots (9)$$

$$7.0 < -0.10\ Wt + 0.15\ Wg + 0.18\ Wr + 4.49 \cdots (10)$$

(Use Example 3-2)

When the density of the recycled glass fiber reinforced resin material is 1.20 g/cm$^3$ to 1.25 g/cm$^3$, it is predicted that the recycled glass fiber reinforced resin material satisfying all of the following Formulas (11) to (13) satisfies all of the flexural modulus > 7,500 MPa, the flexural strength > 115 MPa, and the room-temperature Charpy impact strength > 9.0 kj/m$^2$.

$$7{,}500 < 128\ Wt + 213\ Wg - 1\ 02\ Wr + 1{,}081 \cdots (11)$$

$$115 < -0.38 \ \text{Wt} + 1.52 \ \text{Wg} - 3.30 \ \text{Wr} + 106.30 \quad \cdots \quad (12)$$

$$9.0 < -0.10 \ \text{Wt} + 0.15 \ \text{Wg} + 0.18 \ \text{Wr} + 4.49 \quad \cdots \quad (13)$$

(Use Example 3-3)

When the density of the recycled glass fiber reinforced resin material is 1.30 g/cm$^3$ to 1.35 g/cm$^3$, it is predicted that the recycled glass fiber reinforced resin material satisfying all of the following Formulas (14) to (16) satisfies all of the flexural modulus > 10,000 MPa, the flexural strength > 140 MPa, and the room-temperature Charpy impact strength > 11.0 kj/m$^2$.

$$10,000 < 128 \ \text{Wt} + 213 \ \text{Wg} - 102 \ \text{Wr} + 1081 \quad \cdots \quad (14)$$

$$140 < -0.38 \ \text{Wt} + 1.52 \ \text{Wg} - 3.30 \ \text{Wr} + 106.30 \quad \cdots \quad (15)$$

$$11.0 < -0.10 \ \text{Wt} + 0.15 \ \text{Wg} + 0.18 \ \text{Wr} + 4.49 \quad \cdots \quad (16)$$

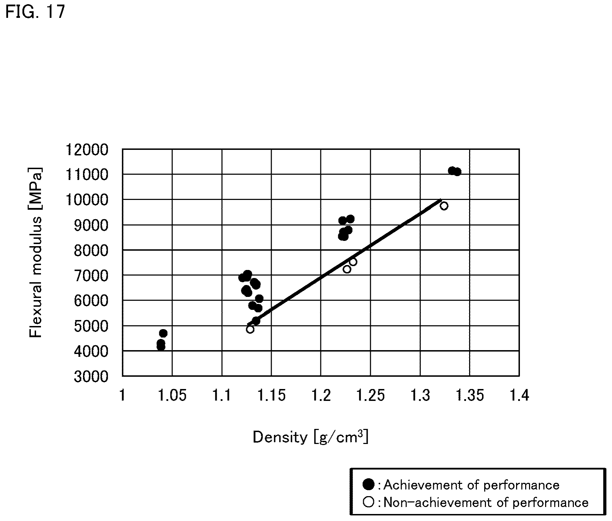

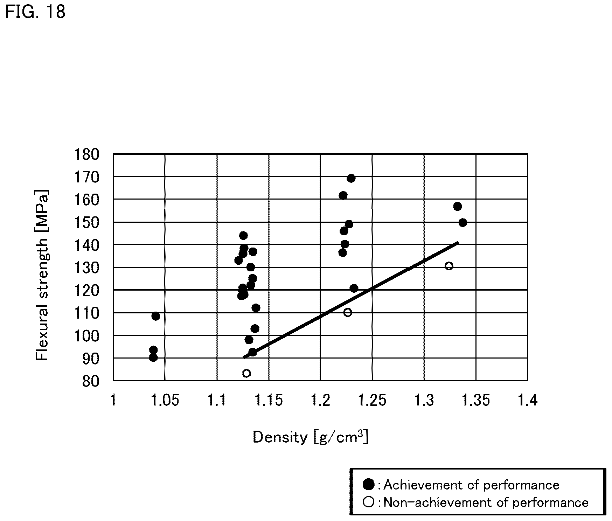

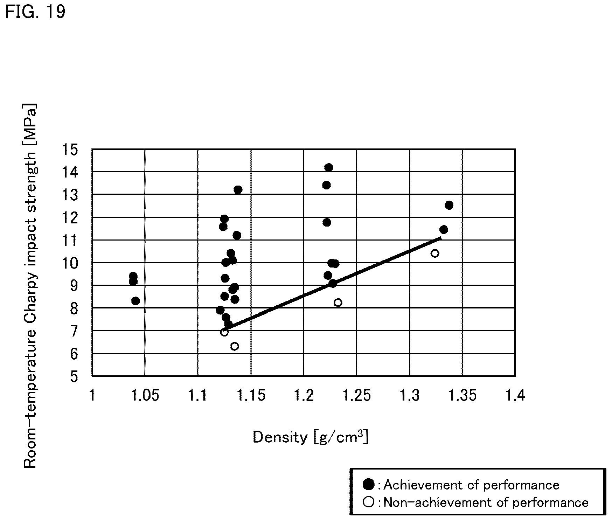

Figs. 17 to 19 illustrate the relationship between the actual flexural modulus, flexural strength, room-temperature Charpy impact strength, and density of 27 learning data sets of recycled glass fiber reinforced resin materials containing the recycled polymer materials described above. Note that, in the drawings, samples plotted with black circles satisfy all of the multiple regression equations (5) to (7), and samples plotted with white circles do not satisfy the multiple regression equations (5) to (7).

As illustrated in Figs. 17 to 19, among the recycled glass fiber reinforced resin materials of Use Examples 3-1 to 3-3, when a propylene-based resin composition having a composition satisfying all of the multiple regression equations (5) to (7) is used, it can be said that the recycled glass fiber reinforced resin material has desired physical properties. Therefore, it can be said that a recycled glass fiber reinforced resin material containing a recycled polymer material excellent in physical property balance is obtained at a composition ratio satisfying all of the multiple regression equations (5) to (7).

Note that aspects of the embodiment of the present invention are, for example, as follows.

<1> A composition proposal system that proposes a composition of a constituent component contained in a recycled polymer composite material containing a recycled polymer material so that the recycled polymer composite material satisfies one or more desired physical property values, the composition proposal system including:

an acquisition unit that acquires a physical property value of the recycled polymer material;
a prediction unit that outputs a parameter including a descriptor of the recycled polymer material predicted by a first learned model by inputting the physical property value of the recycled polymer material acquired by the acquisition unit to the first learned model learned using a first learning data set in which a physical property value of a virgin polymer composite material containing a virgin polymer material and a parameter including a descriptor of the virgin polymer composite material are associated with each other;
a setting unit that sets a target value of a physical property of a virtual recycled polymer composite material; and
an optimization unit that changes a parameter including a descriptor of the virtual recycled polymer composite material so that a physical property value of the virtual recycled polymer composite material predicted by inputting the parameter including the descriptor of the virtual recycled polymer composite material to a second learned model learned using a second learning data set in which the parameter including the descriptor of the recycled polymer composite material and the physical property value of the recycled polymer composite material are associated with each other approaches the target value,
in which the optimization unit optimizes a mass ratio of the recycled polymer material and components other than the recycled polymer material contained in the virtual recycled polymer composite material as the constituent components by changing the parameter including the descriptor of the virtual recycled polymer composite material each time the physical property value of the virtual recycled polymer composite material is predicted.

<2-0> The composition proposal system according to <1>, in which mass ratios of the recycled polymer composite material, the virgin polymer composite material, the polymer composite material, and the virtual recycled polymer composite material each include at least one or more of a talc amount, a rubber amount, and a glass fiber amount.
<2> The composition proposal system according to <1>, in which mass ratios of the recycled polymer composite material, the virgin polymer composite material, and the virtual recycled polymer composite material each include at least one or more of a talc amount, a rubber amount, and a glass fiber amount.

<3-0> The composition proposal system according to <1> or <2>, in which descriptors of the recycled polymer composite material, the virgin polymer composite material, the polymer composite material, and the virtual recycled polymer composite material each include at least one of a characteristic value and a structural value of the constituent components contained in the recycled polymer composite material, the virgin polymer composite material, the polymer composite material, and the virtual recycled polymer composite material.

<3> The composition proposal system according to <1> or <2>, in which descriptors of the recycled polymer composite material, the virgin polymer composite material, and the virtual recycled polymer composite material each include at least one of a characteristic value and a structural value of the constituent components contained in the recycled polymer composite material, the virgin polymer composite material, the polymer composite material, and the virtual recycled polymer composite material.

<4> The composition proposal system according to any one of <1> to <3>, in which the optimization unit changes the parameter including the descriptor of the virtual recycled polymer composite material to avoid a specific parameter.

<5> The composition proposal system according to any one of <1> to <4>, in which the optimization unit proposes a mass ratio of the recycled polymer material and components other than the recycled polymer material contained as the constituent components for the descriptor of the virtual recycled polymer composite material by performing mathematical optimization processing.

<6> The composition proposal system according to <5>, in which the mathematical optimization processing is any of a genetic algorithm, Bayesian optimization, and TPE.

<7> The composition proposal system according to any one of <1> to <6>, in which the optimization unit optimizes a parameter including a descriptor of the virtual recycled polymer composite material so that a physical property of the virtual recycled polymer composite material is maximized in a desired direction.

<8> The composition proposal system according to any one of <1> to <7>, in which the optimization unit proposes a plurality of combinations of mass ratios of the recycled polymer material and components other than the recycled polymer material contained in the virtual recycled polymer composite material as the constituent components.

<9-0> The composition proposal system according to any one of <1> to <8>, in which the optimization unit changes the parameter including the descriptor of the virtual recycled polymer composite material so that each of the two or more physical properties of the virtual recycled polymer composite material predicted by inputting the parameter including the descriptor of the virtual recycled polymer composite material to the second learned model generated for each of the two or more physical properties of the polymer composite material approaches the target value of each of the two or more physical properties.

<9> The composition proposal system according to any one of <1> to <8>, in which the optimization unit changes the parameter including the descriptor of the virtual recycled polymer composite material so that each of the two or more physical properties of the virtual recycled polymer composite material predicted by inputting the parameter including the descriptor of the virtual recycled polymer composite material to the second learned model generated for each of the two or more physical properties of the recycled polymer composite material approaches the target value of each of the two or more physical properties.

<10> The composition proposal system according to <9>, in which the optimization unit proposes a Pareto solution for the two or more physical properties.

<11> The composition proposal system according to any one of <1> to <10>, in which the mass ratio includes an average value for each type of the constituent components.

<12> The composition proposal system according to any one of <1> to <11>, in which the virgin polymer material and the recycled polymer material contain a copolymer containing two or more kinds of monomer units.

<13-0> The composition proposal system according to any one of <1> to <12>, in which each of the recycled polymer composite material, the virgin polymer composite material, the polymer composite material, and the virtual recycled polymer composite material is a fiber reinforced composite material containing a propylene-based polymer and a filler as constituent components.

<13> The composition proposal system according to any one of <1> to <12>, in which each of the recycled polymer composite material, the virgin polymer composite material, and the virtual recycled polymer composite material is a fiber reinforced composite material containing a propylene-based polymer and a filler as constituent components.

<14> The composition proposal system according to <13>, in which the propylene-based polymer includes a copolymer containing a monomer unit derived from propylene and a monomer unit derived from ethylene.

<15> A method for producing a recycled polymer composite material, the method including:

a step of obtaining a parameter including a descriptor of the virtual recycled polymer composite material obtained by the composition proposal system according to any one of <1> to <14>;
a step of preparing the recycled polymer material and the components other than the recycled polymer material that satisfy the parameter as raw materials; and
a step of mixing each of the raw materials to obtain the recycled polymer composite material satisfying the parameter.

<15-1> A method for producing a recycled polymer composite material, the method including:

a step of obtaining a parameter including a descriptor of a virtual recycled polymer composite material obtained by a composition proposal method that proposes a composition of a constituent component contained in a recycled polymer composite material containing a recycled polymer material so that the recycled polymer composite material satisfies one or more desired physical property values;
a step of preparing the recycled polymer material and the components other than the recycled polymer material as raw materials that satisfy the parameter; and
a step of mixing each of the raw materials to obtain the recycled polymer composite material that satisfies the parameter,
in which the composition proposal method includes:

an acquisition step of acquiring a physical property value of the recycled polymer material;
a prediction step of outputting a parameter including a descriptor of the recycled polymer material predicted by a first learned model by inputting the physical property value of the recycled polymer material acquired by the acquisition step to the first learned model learned using a first learning data set in which a physical property value of a virgin polymer composite material containing a virgin polymer material and a parameter including a descriptor of the virgin polymer composite material are associated with each other;
a setting step of setting a target value of a physical property of a virtual recycled polymer composite material; and
an optimization step of changing a parameter including a descriptor of the virtual recycled polymer composite material so that a physical property value of the virtual recycled polymer composite material predicted by inputting the parameter including the descriptor of the virtual recycled polymer composite material to a second learned model learned using a second learning data set in which the parameter including the descriptor of the recycled polymer composite material and the physical property value of the recycled polymer composite material are associated with each other approaches the target value, and
the optimization step optimizes a mass ratio of the recycled polymer material and components other than the recycled polymer material contained in the virtual recycled polymer composite material as the constituent components by changing the parameter including the descriptor of the virtual recycled polymer composite material each time the physical property value of the virtual recycled polymer composite material is predicted.

<15-2> The method for producing a recycled polymer composite material according to <15-1>, in which mass ratios of the recycled polymer composite material, the virgin polymer composite material, and the virtual recycled polymer composite material each include at least one or more of a talc amount, a rubber amount, and a glass fiber amount.
<15-3> The method for producing a recycled polymer composite material according to <15-1> or <15-2>, in which descriptors of the recycled polymer composite material, the virgin polymer composite material, and the virtual recycled polymer composite material each include at least one of a characteristic value and a structural value of the constituent components contained in the recycled polymer composite material, the virgin polymer composite material, and the virtual recycled polymer composite material.
<15-4> The method for producing a recycled polymer composite material according to <15-1> or <15-2>, in which the optimization step changes the parameter including the descriptor of the virtual recycled polymer composite material to avoid a specific parameter.
<15-5> The method for producing a recycled polymer composite material according to <15-1> or <15-2>, in which the optimization step proposes a mass ratio of the recycled polymer material and components other than the recycled polymer material contained as the constituent components for the descriptor of the virtual recycled polymer composite material by performing mathematical optimization processing.
<15-6> The method for producing a recycled polymer composite material according to <15-5>, in which the mathematical optimization processing is any of a genetic algorithm, Bayesian optimization, and TPE.
<15-7> The method for producing a recycled polymer composite material according to <15-1> or <15-2>, in which the optimization step optimizes a parameter including a descriptor of the virtual recycled polymer composite material so that a physical property of the virtual recycled polymer composite material is maximized in a desired direction.
<15-8> The method for producing a recycled polymer composite material according to <15-1> or <15-2>, in which the optimization step proposes a plurality of combinations of mass ratios of the recycled polymer material and the components other than the recycled polymer material contained in the virtual recycled polymer composite material as the constituent components.

<15-9> The method for producing a recycled polymer composite material according to <15-1> or <15-2>, in which the optimization step changes the parameter including the descriptor of the virtual recycled polymer composite material so that each of the two or more physical properties of the virtual recycled polymer composite material predicted by inputting the parameter including the descriptor of the virtual recycled polymer composite material to the second learned model generated for each of the two or more physical properties of the recycled polymer composite material approaches the target value of each of the two or more physical properties.

<15-10> The method for producing a recycled polymer composite material according to <15-9>, in which the optimization step proposes a Pareto solution for the two or more physical properties.

<15-11> The method for producing a recycled polymer composite material according to <15-1> or <15-2>, in which the mass ratio includes an average value for each type of the constituent components.

<15-12> The method for producing a recycled polymer composite material according to <15-1> or <15-2>, in which the virgin polymer material and the recycled polymer material contain a copolymer containing two or more kinds of monomer units.

<15-13> The method for producing a recycled polymer composite material according to <15-1> or <15-2>, in which each of the recycled polymer composite material, the virgin polymer composite material, and the virtual recycled polymer composite material is a fiber reinforced composite material containing a propylene-based polymer and a filler as constituent components.

<15-14> The method for producing a recycled polymer composite material according to <15-13>, in which the propylene-based polymer includes a copolymer containing a monomer unit derived from propylene and a monomer unit derived from ethylene.

<16> A recycled polymer composite material containing a recycled polymer material and components other than the recycled polymer material as constituent components,

in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.08 $g/cm^3$ to 1.16 $g/cm^3$,
(II) an elastic modulus of the recycled polymer composite material is $128 \times Wt + 213 \times Wg - 102 \times Wr + 1,081 > 5,000$ MPa,
(III) a flexural strength of the recycled polymer composite material is $-0.375 \times Wt + 1.52 \times Wg - 3.30 \times Wr + 106 > 80$ MPa, and
(IV) an impact of the recycled polymer composite material is $-0.102 \times Wt + 0.149 \times Wg + 0.180 \times Wr + 4.49 > 6$.

<16-2> A recycled polymer composite material containing a recycled polymer material and components other than the recycled polymer material as constituent components,

in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.10 $g/cm^3$ to 1.15 $g/cm^3$,
(II) an elastic modulus of the recycled polymer composite material is $128 \times Wt + 213 \times Wg - 102 \times Wr + 1,081 > 5,000$ MPa,
(III) a flexural strength of the recycled polymer composite material is $-0.375 \times Wt + 1.52 \times Wg - 3.30 \times Wr + 106 > 90$ MPa, and
(IV) an impact of the recycled polymer composite material is $-0.102 \times Wt + 0.149 \times Wg + 0.180 \times Wr + 4.49 > 7.0$.

<16-3> A recycled polymer composite material containing a recycled polymer material, a fibrous filler, and a filler as constituent components,

in which the recycled polymer composite material is a recycled glass fiber reinforced composite material,
the recycled polymer material contains a heterophasic propylene polymerization material as a propylene-based polymer,
the fibrous filler contains a glass fiber,
the filler contains talc, and
when a talc amount in the recycled polymer composite material is Wt, a glass fiber amount in the recycled polymer composite material is Wg, and a rubber amount in the recycled polymer composite material is Wr, the recycled polymer composite material has a composition that satisfies the following parameters (I) to (IV):

(I): a density of the recycled polymer composite material is 1.10 g/cm$^3$ to 1.15 g/cm$^3$,
(II) a flexural modulus of the recycled polymer composite material is 128 × Wt + 213 × Wg - 102 × Wr + 1,081 > 5,000 MPa,
(III) a flexural strength of the recycled polymer composite material is -0.375 × Wt + 1.52 × Wg - 3.30 × Wr + 106 > 90 MPa, and
(IV) a room-temperature Charpy impact strength of the recycled polymer composite material is -0.102 × Wt + 0.149 × Wg + 0.180 × Wr + 4.49 > 7.0.

<17> A recycled polymer composite material containing a recycled polymer material and components other than the recycled polymer material as constituent components,
in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.20 g/cm$^3$ to 1.25 g/cm$^3$,
(II) an elastic modulus of the recycled polymer composite material is 128 × Wt + 213 × Wg - 102 × Wr + 1,081 > 7,500 MPa,
(III) a flexural strength of the recycled polymer composite material is -0.375 × Wt + 1.52 × Wg - 3.30 × Wr + 106 > 115 MPa, and
(IV) an impact of the recycled polymer composite material is -0.102 × Wt + 0.149 × Wg + 0.180 × Wr + 4.49 > 9.0.

<17-2> A recycled polymer composite material containing a recycled polymer material, a fibrous filler, and a filler as constituent components,

in which the recycled polymer composite material is a recycled glass fiber reinforced composite material,
the recycled polymer material contains a heterophasic propylene polymerization material as a propylene-based polymer,
the fibrous filler contains a glass fiber,
the filler contains talc, and
when a talc amount in the recycled polymer composite material is Wt, a glass fiber amount in the recycled polymer composite material is Wg, and a rubber amount in the recycled polymer composite material is Wr, the recycled polymer composite material has a composition that satisfies the following parameters (I) to (IV):

(I): a density of the recycled polymer composite material is 1.20 g/cm$^3$ to 1.25 g/cm$^3$,
(II) a flexural modulus of the recycled polymer composite material is 128 × Wt + 213 × Wg - 102 × Wr + 1,081 > 7,500 MPa,
(III) a flexural strength of the recycled polymer composite material is -0.375 × Wt + 1.52 × Wg - 3.30 × Wr + 106 > 115 MPa, and
(IV) a room-temperature Charpy impact strength of the recycled polymer composite material is -0.102 × Wt + 0.149 × Wg + 0.180 × Wr + 4.49 > 9.0.

<18> A recycled polymer composite material containing a recycled polymer material and components other than the recycled polymer material as constituent components,
in which when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.30 g/cm$^3$ to 1.35 g/cm$^3$,
(II) an elastic modulus of the recycled polymer composite material is 128 × Wt + 213 × Wg - 102 × Wr + 1,081 > 10,000 MPa,
(III) a flexural strength of the recycled polymer composite material is -0.375 × Wt + 1.52 × Wg - 3.30 × Wr + 106 > 140 MPa, and
(IV) an impact of the recycled polymer composite material is -0.102 × Wt + 0.149 × Wg + 0.180 × Wr + 4.49 > 11.0.

<18-2> A recycled polymer composite material containing a recycled polymer material, a fibrous filler, and a filler as constituent components,

in which the recycled polymer composite material is a recycled glass fiber reinforced composite material,

the recycled polymer material contains a heterophasic propylene polymerization material as a propylene-based polymer,

the fibrous filler contains a glass fiber,

the filler contains talc, and

when a talc amount in the recycled polymer composite material is Wt, a glass fiber amount in the recycled polymer composite material is Wg, and a rubber amount in the recycled polymer composite material is Wr, the recycled polymer composite material has a composition that satisfies the following parameters (I) to (IV):

(I): a density of the recycled polymer composite material is 1.30 g/cm$^3$ to 1.35 g/cm$^3$,

(II) a flexural modulus of the recycled polymer composite material is $128 \times Wt + 213 \times Wg - 102 \times Wr + 1,081 > 10,000$ MPa,

(III) a flexural strength of the recycled polymer composite material is $-0.375 \times Wt + 1.52 \times Wg - 3.30 \times Wr + 106 > 140$ MPa, and

(IV) a room-temperature Charpy impact strength of the recycled polymer composite material is $-0.102 \times Wt + 0.149 \times Wg + 0.180 \times Wr + 4.49 > 11.0$.

<19> The recycled polymer composite material according to any one of <16> to <18>, in which the recycled polymer material contains a copolymer containing two or more kinds of monomer units.

<20> The recycled polymer composite material according to any one of <16> to <18>, in which the recycled polymer composite material is a fiber reinforced composite material containing a propylene-based polymer and a filler as constituent components.

<21> The recycled polymer composite material according to <20>, in which the fiber reinforced material is a recycled glass fiber reinforced resin material.

<22> The recycled polymer composite material according to <20>, in which the propylene-based polymer includes a copolymer containing a monomer unit derived from propylene and a monomer unit derived from ethylene.

<23> The recycled polymer composite material according to any one of <16> to <18>, in which the elastic modulus is a flexural modulus.

<24> The recycled polymer composite material according to any one of <16> to <18>, in which the impact is a Charpy impact strength.

<25> The recycled polymer composite material according to any one of <16> to <18>, in which an equation satisfying the parameters (II) to (IV) is obtained by performing multiple regression analysis on a learned model generated by Ridge regression using 27 learning data sets of the recycled polymer composite materials, using three variables of a talc amount, a glass fiber amount, and a rubber component amount as explanatory variables, and using three variables of an elastic modulus, a flexural strength, and a strength as objective variables.

DESCRIPTION OF REFERENCE SIGNS

1 First learning device
2 First prediction device
3 Second learning device
4 Second prediction device
10 Composition proposal system
11, 21, 31, 41, 51 Acquisition unit
12, 22, 32, 42, 102 Preprocessing unit
13, 33 Learning data set generation unit
14, 34, 106 Learning unit
15, 24, 35, 44, 113 Display unit
23, 43, 103 Prediction unit
101 First acquisition unit
104 Second acquisition unit
105 Learning data set generation unit
107 Setting unit
108 Third acquisition unit
109 Physical property prediction unit
110 Comparison unit
111 Determination unit

112 Optimization unit
M1, M2, M3, M4 Learned model
M5-1 First learned model
M5-2 Second learned model

**Claims**

1. A composition proposal system that proposes a composition of a constituent component contained in a recycled polymer composite material containing a recycled polymer material so that the recycled polymer composite material satisfies one or more desired physical property values, the composition proposal system comprising:

   an acquisition unit that acquires a physical property value of the recycled polymer material;
   a prediction unit that outputs a parameter including a descriptor of the recycled polymer material predicted by a first learned model by inputting the physical property value of the recycled polymer material acquired by the acquisition unit to the first learned model learned using a first learning data set in which a physical property value of a virgin polymer composite material containing a virgin polymer material and a parameter including a descriptor of the virgin polymer composite material are associated with each other;
   a setting unit that sets a target value of a physical property of a virtual recycled polymer composite material; and
   an optimization unit that changes a parameter including a descriptor of the virtual recycled polymer composite material so that a physical property value of the virtual recycled polymer composite material predicted by inputting the parameter including the descriptor of the virtual recycled polymer composite material to a second learned model learned using a second learning data set in which the parameter including the descriptor of the recycled polymer composite material and the physical property value of the recycled polymer composite material are associated with each other approaches the target value,
   wherein the optimization unit optimizes a mass ratio of the recycled polymer material and components other than the recycled polymer material contained in the virtual recycled polymer composite material as the constituent components by changing the parameter including the descriptor of the virtual recycled polymer composite material each time the physical property value of the virtual recycled polymer composite material is predicted.

2. The composition proposal system according to claim 1, wherein mass ratios of the recycled polymer composite material, the virgin polymer composite material, and the virtual recycled polymer composite material each include at least one or more of a talc amount, a rubber amount, and a glass fiber amount.

3. The composition proposal system according to claim 1 or 2, wherein descriptors of the recycled polymer composite material, the virgin polymer composite material, and the virtual recycled polymer composite material each include at least one of a characteristic value and a structural value of the constituent components contained in the recycled polymer composite material, the virgin polymer composite material, the polymer composite material, and the virtual recycled polymer composite material.

4. The composition proposal system according to claim 1 or 2, wherein the optimization unit changes the parameter including the descriptor of the virtual recycled polymer composite material to avoid a specific parameter.

5. The composition proposal system according to claim 1 or 2, wherein the optimization unit proposes a mass ratio of the recycled polymer material and components other than the recycled polymer material contained as the constituent components for the descriptor of the virtual recycled polymer composite material by performing mathematical optimization processing.

6. The composition proposal system according to claim 5, wherein the mathematical optimization processing is any of a genetic algorithm, Bayesian optimization, and TPE.

7. The composition proposal system according to claim 1 or 2, wherein the optimization unit optimizes a parameter including a descriptor of the virtual recycled polymer composite material so that a physical property of the virtual recycled polymer composite material is maximized in a desired direction.

8. The composition proposal system according to claim 1 or 2, wherein the optimization unit proposes a plurality of combinations of mass ratios of the recycled polymer material and the components other than the recycled polymer material contained in the virtual recycled polymer composite material as the constituent components.

9. The composition proposal system according to claim 1 or 2, wherein the optimization unit changes the parameter including the descriptor of the virtual recycled polymer composite material so that each of the two or more physical properties of the virtual recycled polymer composite material predicted by inputting the parameter including the descriptor of the virtual recycled polymer composite material to the second learned model generated for each of the two or more physical properties of the recycled polymer composite material approaches the target value of each of the two or more physical properties.

10. The composition proposal system according to claim 9, wherein the optimization unit proposes a Pareto solution for the two or more physical properties.

11. The composition proposal system according to claim 1 or 2, wherein the mass ratio includes an average value for each type of the constituent components.

12. The composition proposal system according to claim 1 or 2, wherein the virgin polymer material and the recycled polymer material contain a copolymer containing two or more kinds of monomer units.

13. The composition proposal system according to claim 1 or 2, wherein each of the recycled polymer composite material, the virgin polymer composite material, and the virtual recycled polymer composite material is a fiber reinforced composite material containing a propylene-based polymer and a filler as constituent components.

14. The composition proposal system according to claim 13, wherein the propylene-based polymer includes a copolymer containing a monomer unit derived from propylene and a monomer unit derived from ethylene.

15. A method for producing a recycled polymer composite material, the method comprising:

a step of obtaining a parameter including a descriptor of the virtual recycled polymer composite material obtained by the composition proposal system according to claim 1 or 2;
a step of preparing the recycled polymer material and the components other than the recycled polymer material that satisfy the parameter as raw materials; and
a step of mixing each of the raw materials to obtain the recycled polymer composite material satisfying the parameter.

16. A recycled polymer composite material comprising a recycled polymer material and components other than the recycled polymer material as constituent components,
wherein when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.08 g/cm$^3$ to 1.16 g/cm$^3$,
(II) an elastic modulus of the recycled polymer composite material is $128 \times Wt + 213 \times Wg - 102 \times Wr + 1,081 > 5,000$ MPa,
(III) a flexural strength of the recycled polymer composite material is $-0.375 \times Wt + 1.52 \times Wg - 3.30 \times Wr + 106 > 80$ MPa, and
(IV) an impact of the recycled polymer composite material is $-0.102 \times Wt + 0.149 \times Wg + 0.180 \times Wr + 4.49 > 6$.

17. A recycled polymer composite material comprising a recycled polymer material and components other than the recycled polymer material as constituent components,
wherein when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.20 g/cm$^3$ to 1.25 g/cm$^3$,
(II) an elastic modulus of the recycled polymer composite material is $128 \times Wt + 213 \times Wg - 102 \times Wr + 1,081 > 7,500$ MPa,
(III) a flexural strength of the recycled polymer composite material is $-0.375 \times Wt + 1.52 \times Wg - 3.30 \times Wr + 106 > 115$ MPa, and
(IV) an impact of the recycled polymer composite material is $-0.102 \times Wt + 0.149 \times Wg + 0.180 \times Wr + 4.49 > 9.0$.

18. A recycled polymer composite material comprising a recycled polymer material and components other than the recycled polymer material as constituent components,
wherein when a talc amount in the recycled polymer composite material is Wt, a glass fiber amount in the recycled polymer composite material is Wg, and a rubber amount in the recycled polymer composite material is Wr, the recycled polymer composite material satisfies the following parameters (I) to (IV):

(I): a density of the recycled polymer composite material is 1.30 $g/cm^3$ to 1.35 $g/cm^3$,
(II) an elastic modulus of the recycled polymer composite material is $128 \times Wt + 213 \times Wg - 102 \times Wr + 1,081 > 10,000$ MPa,
(III) a flexural strength of the recycled polymer composite material is $-0.375 \times Wt + 1.52 \times Wg - 3.30 \times Wr + 106 > 140$ MPa, and
(IV) an impact of the recycled polymer composite material is $-0.102 \times Wt + 0.149 \times Wg + 0.180 \times Wr + 4.49 > 11.0$.

FIG. 1

**<First learning device> 1**

Physical property value and parameter of polymer material or polymer composite material (including descriptor of polymer material or polymer composite material) → Acquisition unit 11 → Preprocessing unit 12 → First learning data set generation unit 13 → Learning data set → <Learning unit> 14 / Learned model M1 → Display unit 15

**<First prediction device> 2**

Physical property value of recycled polymer material → Acquisition unit 21 → Preprocessing unit 22 → Prediction unit 23 → Display unit 24

Learned model M2 → Prediction unit 23

Parameter (including descriptor of recycled polymer material)

FIG. 2

FIG. 3

FIG. 4

FIG. 5

```
                    ┌──────────────┐
                    │    Start     │
                    └──────┬───────┘
                           │
                           ▼
  ┌────────────────────────────────────────────────┐
  │  Acquisition of physical property value of polymer  │
  │  material or polymer composite material and        │──S11
  │  parameter including descriptor of polymer          │
  │  material or polymer composite material             │
  └────────────────────────┬───────────────────────┘
                           │
                           ▼
  ┌────────────────────────────────────────────────┐
  │               Preprocessing                     │──S12
  └────────────────────────┬───────────────────────┘
                           │
                           ▼
  ┌────────────────────────────────────────────────┐
  │           Generation of learning data set        │──S13
  └────────────────────────┬───────────────────────┘
                           │
                           ▼
  ┌────────────────────────────────────────────────┐
  │            Generation of learned model M1         │──S14
  └────────────────────────┬───────────────────────┘
                           │
                           ▼
  ┌────────────────────────────────────────────────┐
  │  Display of information (information of learning   │──S15
  │   data set, learned model M1, and the like)        │
  └────────────────────────┬───────────────────────┘
                           │
                           ▼
                    ┌──────────────┐
                    │     End      │
                    └──────────────┘
```

FIG. 6

```
                    ┌──────────────┐
                    │    Start     │
                    └──────┬───────┘
                           │
                           ▼
        ┌──────────────────────────────────────┐
        │ Acquisition of physical property value of │ ～S21
        │      recycled polymer material         │
        └──────────────────┬───────────────────┘
                           │
                           ▼
        ┌──────────────────────────────────────┐
        │            Preprocessing              │ ～S22
        └──────────────────┬───────────────────┘
                           │
                           ▼
        ┌──────────────────────────────────────┐
        │     Prediction of parameter including  │ ～S23
        │    descriptor of recycled polymer material │
        └──────────────────┬───────────────────┘
                           │
                           ▼
        ┌──────────────────────────────────────┐
        │ Display of parameter including descriptor │ ～S24
        │      of recycled polymer material      │
        └──────────────────┬───────────────────┘
                           │
                           ▼
                    ┌──────────────┐
                    │     End      │
                    └──────────────┘
```

FIG. 7

```
                      ┌─────────────────┐
                      │      Start      │
                      └─────────────────┘
                               │
                               ▼
   ┌──────────────────────────────────────────────┐
   │     Acquisition of parameter including        │
   │   descriptor of recycled polymer composite    │──S31
   │    material and physical property value of    │
   │      recycled polymer composite material      │
   └──────────────────────────────────────────────┘
                               │
                               ▼
   ┌──────────────────────────────────────────────┐
   │                Preprocessing                  │──S32
   └──────────────────────────────────────────────┘
                               │
                               ▼
   ┌──────────────────────────────────────────────┐
   │          Generation of learning data set      │──S33
   └──────────────────────────────────────────────┘
                               │
                               ▼
   ┌──────────────────────────────────────────────┐
   │         Generation of learned model M3a       │──S34
   └──────────────────────────────────────────────┘
                               │
                               ▼
   ┌──────────────────────────────────────────────┐
   │  Display of information (information of learning│──S35
   │   data set, learned model M3, and the like)    │
   └──────────────────────────────────────────────┘
                               │
                               ▼
                      ┌─────────────────┐
                      │       End       │
                      └─────────────────┘
```

FIG. 8

```
                        ┌─────────────┐
                        │    Start    │
                        └──────┬──────┘
                               │
                               ▼
        ┌──────────────────────────────────────────────┐
        │ Acquisition of parameter including descriptor of │───S41
        │       recycled polymer composite material       │
        └──────────────────────┬───────────────────────┘
                               │
                               ▼
        ┌──────────────────────────────────────────────┐
        │                 Preprocessing                 │───S42
        └──────────────────────┬───────────────────────┘
                               │
                               ▼
        ┌──────────────────────────────────────────────┐
        │      Prediction of physical property value of    │───S43
        │         recycled polymer composite material      │
        └──────────────────────┬───────────────────────┘
                               │
                               ▼
        ┌──────────────────────────────────────────────┐
        │   Display of physical property value of recycled  │───S44
        │            polymer composite material            │
        └──────────────────────┬───────────────────────┘
                               │
                               ▼
                        ┌─────────────┐
                        │     End     │
                        └─────────────┘
```

FIG. 9

Start

S101
Acquisition of physical property value of recycled polymer material

S102
Preprocessing

S103
Prediction of parameter (including descriptor of recycled polymer material)

S104
Acquisition of parameter (including descriptor of recycled polymer material)

S105
Generation of learning data set

S106
Generation of second learned model M5-2

S107
Setting of target value of physical property of virtual recycled polymer composite material

S108
Acquisition of parameter (including descriptor of recycled polymer composite material)

S109
Prediction of physical property value of virtual recycled polymer composite material

S110
Comparison

S111
Has optimization been performed predetermined number of times?

No (There is need to change descriptor of virtual recycled polymer composite material)

Yes (There is no need to change descriptor of virtual recycled polymer composite material)

Optimized parameter (including virtual descriptor)

S114
Optimization of parameter (including descriptor of virtual recycled polymer composite material)

S112
Is difference between physical property value and target value of virtual recycled polymer composite material within specific range?

No (There is need to change descriptor of virtual recycled polymer composite material)

Yes (There is no need to change descriptor of virtual recycled polymer composite material)

S113
Display of parameter including descriptor of virtual recycled polymer composite material

End

FIG. 10

```
         ╭─────────────╮
         │    Start     │
         ╰──────┬──────╯
                │
                ▼
 ┌──────────────────────────────────────────┐
 │ Acquisition of parameter including        │ ~S201
 │ descriptor of virtual recycled polymer    │
 │ composite material                        │
 └──────────────────────────────────────────┘
                │
                ▼
 ┌──────────────────────────────────────────┐
 │        Preparation of raw materials       │ ~S202
 └──────────────────────────────────────────┘
                │
                ▼
 ┌──────────────────────────────────────────┐
 │ Production of recycled polymer composite  │ ~S203
 │ material                                  │
 └──────────────────────────────────────────┘
                │
                ▼
         ╭─────────────╮
         │     End      │
         ╰─────────────╯
```

FIG. 11

<Talc amount>

FIG. 12

<Rubber amount>

FIG. 13

<Flexural modulus>

FIG. 14

<Flexural strength>

FIG. 15

<Room-temperature Charpy impact strength>

FIG. 16

Predicted value of room-temperature

Charpy impact strength [kJ/m²]

O : Pareto solution

● : Predicted value

FIG. 17

FIG. 18

FIG. 19

# EP 4 679 433 A1

## INTERNATIONAL SEARCH REPORT

| International application No. |
|---|
| **PCT/JP2024/007237** |

### A. CLASSIFICATION OF SUBJECT MATTER

*G16C 20/30*(2019.01)i; *C08J 5/04*(2006.01)i; *G16C 20/70*(2019.01)i
FI:   G16C20/30; C08J5/04 CES; G16C20/70

According to International Patent Classification (IPC) or to both national classification and IPC

### B. FIELDS SEARCHED

Minimum documentation searched (classification system followed by classification symbols)

G06Q10/00-99/00; G16C10/00-99/00; G16Z99/00; C08J5/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2020-87446 A (TORAY ENGINEERING CO., LTD.) 04 June 2020 (2020-06-04) paragraphs [0006], [0027]-[0028], [0031], [0033]-[0044] | 1-14 |
| A | JP 2021-39534 A (HITACHI, LTD.) 11 March 2021 (2021-03-11) paragraphs [0011], [0023]-[0026], [0050]-[0062] | 1-14 |
| A | US 2022/0101276 A1 (X DEVELOPMENT LLC) 31 March 2022 (2022-03-31) paragraphs [0022]-[0025], [0037]-[0053] | 1-14 |
| A | CN 115616204 A (KINGFA SCIENCE & TECHNOLOGY CO.) 17 January 2023 (2023-01-17) paragraphs [0021]-[0023], [0035]-[0048] | 1-14 |
| A | KR 10-2308311 B1 (DAEJIN ADVANCED MATERIALS INC.) 06 October 2021 (2021-10-06) paragraphs [0040]-[0043], [0049]-[0083] | 1-14 |

✓ Further documents are listed in the continuation of Box C.       ✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 May 2024** | **21 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2024/007237**

## C. DOCUMENTS CONSIDERED TO BE RELEVANT

| Category* | Citation of document, with indication, where appropriate, of the relevant passages | Relevant to claim No. |
|---|---|---|
| P, A | JP 2023-44459 A (KABUSHIKI KAISHA KOBE SEIKO SHO) 30 March 2023 (2023-03-30) paragraphs [0005], [0016]-[0018], [0026]-[0038], [0054]-[0058] | 1-14 |

Form PCT/ISA/210 (second sheet) (July 2022)

## INTERNATIONAL SEARCH REPORT

| International application No. |
| --- |
| **PCT/JP2024/007237** |

**Box No. III   Observations where unity of invention is lacking (Continuation of item 3 of first sheet)**

This International Searching Authority found multiple inventions in this international application, as follows:

(Invention 1) Claims 1-14

The invention in claim 1 has the special technical feature of information processing comprising "an optimization part that changes a parameter including a descriptor of the hypothetical recycled polymer composite material so that the physical property value of the hypothetical recycled polymer composite material approach the target value, the physical property value being predicted by inputting a parameter including a descriptor of the hypothetical recycled polymer composite material into a second trained model, which has been trained by using a second training dataset in which parameters including a descriptor of recycled polymer composite materials and the physical property value of the recycled polymer composite material are associated with each other, wherein the optimization part optimizes a mass ratio of said recycled polymer composite material and a component other than the recycled polymer material included as a component in the hypothetical recycled polymer composite material by changing a parameter including a descriptor of the hypothetical recycled polymer composite material, whenever the property value of said hypothetical recycled polymer composite material is predicted." Thus, the invention in claim 1, and the invention in claims 2-14 which has a special technical feature identical or corresponding to said special technical feature, are classified as invention 1.

(Invention 2) Claim 15

The invention of claim 15 is an invention for a "method of manufacturing recycled polymeric composite material" and cannot be said to have special technical features of information processing as described above, nor can the invention be said to be an invention in the same category as information processing. Also, claim 15 is not dependent on claim 1, is not substantially identical to or similarly closely related to any of the claims classified as invention 1, and thus cannot be classified as invention 1, and are thus classified as invention 2.

(Invention 3) Claims 16-18

The invention of claims 16-18 is an invention for a "recycled polymeric composite material" and cannot be said to have a special technical characteristic in information processing, nor can the invention be said to be an invention in the same category as information processing. Claims 16-18 are not dependent on claim 1 and not substantially identical to or similarly closely related to any of the claims classified as invention 1, and cannot be classified as invention 1 (and invention 2), and are thus classified as invention 3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **(Invention 1) Claims 1-14**

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

| International application No. |
| --- |
| **PCT/JP2024/007237** |

| Patent document cited in search report | | | Publication date (day/month/year) | Patent family member(s) | | | Publication date (day/month/year) |
| --- | --- | --- | --- | --- | --- | --- | --- |
| JP | 2020-87446 | A | 04 June 2020 | WO | 2020/100726 | A1 | |
| JP | 2021-39534 | A | 11 March 2021 | US | 2022/0359047 | A1 | |
| | | | | paragraphs [0012], [0035]-[0038], [0062]-[0074] | | | |
| | | | | WO | 2021/044846 | A1 | |
| | | | | EP | 4027295 | A1 | |
| | | | | CN | 114175171 | A | |
| US | 2022/0101276 | A1 | 31 March 2022 | WO | 2022/072065 | A1 | |
| | | | | EP | 4205128 | A1 | |
| CN | 115616204 | A | 17 January 2023 | (Family: none) | | | |
| KR | 10-2308311 | B1 | 06 October 2021 | (Family: none) | | | |
| JP | 2023-44459 | A | 30 March 2023 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018178107 A
- WO 2020149284 A
- WO 2022080363 A

**Non-patent literature cited in the description**

- Polymer solution, Polymer Experiment 11. KYOR-ITSU SHUPPAN CO., LTD., 1982, 491